# EUROPEAN PATENT APPLICATION

(11) **EP 4 129 339 A1**
(43) Date of publication of application: **08.02.2023**
(21) Application number: 21778969.2
(22) Date of filing: 31.03.2021
(51) Int. Cl.: A61K 45/00, A61P 25/00, A61P 27/02, A61P 27/06, A61P 43/00, A61K 9/06, A61K 9/08, A61K 9/19, C12N 15/12, C12N 15/62, C12Q 1/06, C12Q 1/6841, C12Q 1/6897, G01N 33/15, G01N 33/50, G01N 33/53, A61K 31/167, A61K 31/198, A61K 31/44, A61K 31/4709, A61K 31/506

(54) **METHOD FOR SCREENING FOR, METHOD FOR PRODUCING, AND METHOD FOR DESIGNING DRUG ACTIVE INGREDIENTS**

(30) Priority: 31.03.2020 JP 2020063994; 31.03.2020 JP 2020063995; 31.03.2020 JP 2020063996; 31.03.2020 JP 2020063997
(71) Applicant: Sky Pharma Co., Ltd., Sendai-shi, Miyagi 984-0075 (JP)
(72) Inventor: ANDO, Hideki, Sendai-shi, Miyagi 984-0075 (JP)
(74) Representative: dompatent von Kreisler Selting Werner - Partnerschaft von Patent- und Rechtsanwälten mbB
(86) International application number: PCT/JP2021/014027
(87) International publication number: WO 2021/201170

(57) **Abstract**

An object of the present invention is to provide a novel screening technique for selecting a useful pharmaceutical compound.

The present invention to solve the above problem is a method for screening active ingredients of a medicine, including the following
step A,
step B and/or step C, and
step D.
[step A] a step of administering a candidate substance to an animal (excluding human);
[step B] a step of observing undifferentiated cells in the animal that has undergone step A;
[step C] a step of observing differentiated cells in the animal that has undergone step A; and
[step D] a step of selecting, as the active ingredient,
a candidate substance that improves the amount of undifferentiated cells, or
a candidate substance that improves the amount of differentiated cells or
a candidate substance that improves a function of a tissue composed of differentiated cells,
as compared with a case where the candidate substance is not administered.

## Description

### Technical Field

The present invention relates to a method for screening pharmaceutically active ingredients, a method for producing a medicine, and a method for designing a medicine.

### Background Art

Adult stem cells are rare undifferentiated cells found in all tissues of the body. Although usually kept in a quiescent, non-dividing state, these cells can proliferate and differentiate to replace naturally dying cells in a tissue and repair a wound in response to injury. Adult stem cells have potential to treat not only aging but also various degenerative diseases due to their proliferation and tissue regeneration ability (Non Patent Literature 1).

For example, it is known that a cardiac stem cell/cardiac precursor cell (Cardiac stem cell, CSC) having an ability to differentiate into all cells of the cardiac lineage exist in the heart (Non Patent Literature 2).

In addition, as a result of investigating localization of a transcription factor SOX9 having a function of keeping cells in an undifferentiated state, it has been reported that SOX9 is expressed in a crypt containing Lgr5-positive cells which are stem cells in the intestines, is expressed in a pancreatic duct and centroacinar cells which are terminal duct cells thereof in the pancreas, and is expressed in a bile duct cell in the liver in a localized manner (Non Patent Literature 3).

In mature mammals, neurons generally do not have division capacity. Therefore, once a disorder occurs, the disorder continues for a long period of time. In particular, it has been an established theory in the past that there is no regeneration ability at all in the central nervous system such as the brain and the spinal cord. It has been said that the lack of regenerative ability in the central nerve is one of causes that there is no method for treating apathy due to an exogenous injury such as spinal cord injury.

On the other hand, a peripheral nerve has regeneration ability, and an axon regenerates and recovers its function even after being cut once. However, also in this case, a period required for reproduction is a long period of several months to one year or more. Therefore, a great burden is imposed on improvement of QOL of patients. In addition, since it takes a long period of time to regenerate the nerve, there are many cases where the neurons die during that time and the function is not restored.

Recent studies have revealed that adult neural stem cells are present in an adult brain and are responsible for neurogenesis. Adult neural stem cells are a unique subpopulation of cells with structural plasticity characteristics. In mammals, neurogenesis is present in two germination regions. Neurogenesis occurs in two germination regions, SVZ and SGZ, throughout life. To date, various markers have been discovered that are expressed in a multistep strategy during progression of adult hippocampal neurogenesis.

Radial glia-like neural stem/progenitor cells present in SGZ usually assume a relatively resting state, but can be activated by internal and external stimuli. These cells constitute a pool of neuroblasts by dividing symmetrically and asymmetrically. Some of the cells in this pool differentiate into immature neurons (Non Patent Literature 4).

Regeneration induction of neurons has been studied very widely and deeply due to its medical importance. In vitro, with the development of a technique for unwinding differentiation of somatic cells into pluripotent cells (inducible pluripotent stem cells, iPS cells) as a trigger (Patent Literature 1), search and identification of genes and compounds that proliferate neural stem cells have been performed worldwide (for example, Patent Literature 2 and Patent Literature 3). In parallel with this, search and identification of genes and compounds that induce differentiation of neural stem cells and undifferentiated neural precursor cells into mature neural cells have also been performed (Patent Literature 4).

Currently, there is a method for treating a disease by initializing somatic cells collected from a patient to obtain stem cells, amplifying the stem cells by self-renewal, then inducing differentiation into desired cells, and transplanting the stem cells into the patient again in the clinical stage (for example, Non Patent Literature 5, Non Patent Literature 6, and Non Patent Literature 7).

In addition, it is presumed that a substance that inhibits nerve elongation exists in the central nervous system as a cause of difficulty in regeneration of the central nervous system. When the nerve regeneration inhibitor present in the central nervous system is suppressed by an antibody or the like, it is expected that a part of nerve regeneration occurs in the center and recovery of function is also observed.

Recently, Nogo has been discovered as the central nerve regeneration inhibitory factor (Non Patent Literature 8 and Non Patent Literature 9). However, it is considered that some nerve fibers regenerate by inhibiting Nogo, and other regeneration inhibitors exist.

Semaphorin has also been estimated as one of factors acting on inhibition of nerve regeneration in vivo (Non Patent Literature 10 and Non Patent Literature11).

In addition, the present inventors have reported that BRD7 and Ikaros are downstream factors of CRBN, that these are inhibitors of development of the central nervous system, and that antagonists of BRD7 and Ikaros and activators of CRBN can promote regeneration of the central nervous system (Patent Literature 5).

Besides that, it is known that a wide variety of adult stem cells such as hematopoietic stem cells derived from bone marrow and having an ability to differentiate into all blood cells; satellite cells found in mature muscle, present between a basement membrane and muscle sheath, providing new cells to muscle fibers; hair follicle stem cells generating new hair follicles and maintaining all cell lines of hair follicles for long term; mammary stem cells serving as a cell source for growing mammary gland during adolescence and pregnancy; mesenchymal stem cells derived from bone marrow stroma and capable of differentiating into a variety of tissues; endothelial stem cells that differentiate into vascular endothelium and are responsible for reconstructing blood vessels; olfactory mucosal stem cells that can be taken from olfactory mucosa and have an ability to differentiate into many different cells; and neural cells, Schwann cells, myofibroblasts, chondrocytes, and neural crest stem cells capable of differentiating into melanocytes exist in the adult body.

### Citation List

### Patent Literature

Patent Literature 1: WO 2007/069666 A
Patent Literature 2: WO 2016/002854 A
Patent Literature 3: JP 2015-071565 A
Patent Literature 4: JP 2017-145215 A
Patent Literature 5: WO 2015/127351 A

### Non Patent Literature

Non Patent Literature 1: Ann N Y Acad Sci. 2020 Feb; 1462 (1) : 27-36.
Non Patent Literature 2: Oxid Med Cell Longev. 2019; 2019: 5813147.
Non Patent Literature 3: Nature Genetics, 43, 34-41 (2011) Non Patent Literature 4: Biomed Res Int. 2015; 2015: 727542.
Non Patent Literature 5: New England Journal of Medicine 2017; 376: 1038-1046
Non Patent Literature 6: The Journal of The Japanese Society for Cataract Research, 2015, Vol. 27, No. 1, p. 32-34
Non Patent Literature 7: Regenerative therapy: Journal of the Japanese Society for Regenerative Medicine, 14(4) = 62: 2015.11 p. 319-329
Non Patent Literature 8: Nature 403, 434, 2000.
Non Patent Literature 9: Nature 403, 439, 2000.
Non Patent Literature 10: Cell 75, 217, 1993.
Non Patent Literature 11: Cell 75, 1389, 1993.
Non Patent Literature 12: Marine Biotechnology 8(3): 295-303 2006
Non Patent Literature 13: J Neurochem. 2001 Jan; 76(1): 173-81.
Non Patent Literature 14: J Cancer Res 2011 Jun; 23(2): 140-146
Non Patent Literature 15: PLOS ONE, October 2014 Volume 9 Issue 10 e109588
Non Patent Literature 16: Endocrine Reviews. 28(3): 339-63. Non Patent Literature 17: Development. 124(10): 1887-97. Non Patent Literature 18: Neuron. 40(6): 1105-18.
Non Patent Literature 19: Semin Cell Dev Biol. 2012 Jun; 23(4): 473-480.
Non Patent Literature 20: Inflammation and regeneration VOL. 22 NO. 3 MAY 2002 195-200
Non Patent Literature 21: Sci Signal. 2009 Jan 27; 2(55): ra1.
Non Patent Literature 22: Development 2016 143: 3050-3060 Non Patent Literature 23: Development 2019 146: dev167643 Non Patent Literature 24: Stem Cells Int. 2017; 2017: 1610691.
Non Patent Literature 25: Cell Research volume 18, pages 523-527 (2008)
Non Patent Literature 26: J Clin Invest. 2018; 128(9): 3872-3886.
Non Patent Literature 27: Proc Natl Acad Sci USA. 2005 Apr 26; 102(17): 6068-73
Non Patent Literature 28: Cancer Cell, 30, 792-805 (2016)

### Summary of Invention

### Technical Problem

So far, it has been considered that self-renewal and differentiation induction of stem cells are induced by different factors. Due to the presence of such technical common knowledge, factors that induce self-renewal of stem cells and factors that induce differentiation induction have been separately screened in an in vitro test system. Specifically, cultured stem cells are exposed to a candidate substance, and factors that induce self-renewal are screened using its growth promoting effect as an index, while cultured stem cells are exposed to a candidate substance, and factors that induce differentiation induction are screened using a differentiation-inducing effect on desired mature cells as an index.

However, the factors selected in such a two-stage in vitro screening test system are only those whose effects have been confirmed in vitro. Therefore, as the application, regenerative medicine using ES cells, iPS cells, or the like, that is, application to an in vitro stem cell amplification process and a differentiation induction process is mainly used.

In the prior art, there is a substance that induces self-renewal and differentiation induction of undifferentiated cells in vivo by a single agent, and there is no idea to screen this substance.

In view of such circumstances, an object of the present invention is to provide a novel screening technique for selecting a useful pharmaceutical compound.

### Solution to Problem

As a result of intensive research efforts of the present inventor, it has been found that it is possible to screen compounds capable of realizing "regenerative medicine" in vivo and with a single agent by observing undifferentiated cells or differentiated cells in vivo to which a candidate substance has been administered. Then, it was demonstrated that an active ingredient screened by this method using a living body exhibits a tissue regeneration effect in an adult. Furthermore, it was also demonstrated that the active ingredient has an ability to forcibly induce differentiation of cancer cells into normal cells. The present invention has been completed by such findings.

That is, the present invention to solve the above problem is a method for screening active ingredients for treatment or prevention of a disease, disorder or illness, or symptoms thereof, including the following step A, step B and/or step C, and step D.
[step A] a step of administering a candidate substance to an animal (excluding human);
[step B] a step of observing undifferentiated cells in the animal that has undergone step A;
[step C] a step of observing differentiated cells in the animal that has undergone step A; and
[step D] a step of selecting, as the active ingredient, a candidate substance that improves the amount of undifferentiated cells, or a candidate substance that improves the amount of differentiated cells or a function of a tissue composed of differentiated cells, as compared with a case where the candidate substance is not administered.

The present invention uses an animal living body as a screening tool. Then, undifferentiated cells and/or differentiated cells in an animal to which the candidate substance has been administered are observed. According to the present invention having this technical feature, it is possible to efficiently screen active ingredients that realize regenerative medicine in vivo and with a single agent. In addition, according to the present invention, it is possible to screen active ingredients having an effect of forcibly differentiating cancer cells into normal cells.

In a preferred mode of the present invention, it is a method for screening active ingredients of a regenerative medicine and/or an anticancer agent. According to the present invention, it is possible to efficiently screen active ingredients of a regenerative medicine or an anticancer agent.

In a preferred mode of the present invention, it is a method for screening active ingredients for treatment or prevention of a disease, disorder, or illness of nervous system, heart, or pancreas, or symptoms thereof. According to the present invention, it is possible to efficiently screen active ingredients for treatment or prevention of a disease, disorder, or illness of nervous system, heart, or pancreas, or symptoms thereof.

In a preferred mode of the present invention, it is a method for screening active ingredients for treatment of hematological cancer. According to the present invention, it is possible to efficiently screen active ingredients of therapeutic agents for hematological cancer.

In a preferred mode of the present invention, the candidate substance is one or more selected from a low molecular compound, a protein, a peptide, and a nucleic acid. The type of candidate substance that can be subjected to the screening method of the present invention is not limited.

In a preferred mode of the present invention, the method includes step B and step C. By observing both undifferentiated cells and differentiated cells after administration of the candidate substance, it is possible to more accurately screen active ingredients.

In a preferred mode of the present invention, the animal is a vertebrate.

In a more preferred mode of the present invention, the animal is a mammal, a fish, a bird, a reptile, or an amphibian.

In a preferred mode of the present invention, the animal is a zebrafish or a mouse.

By using these animals as a screening tool, it is possible to more efficiently screen active ingredients exhibiting pharmacological action in humans.

In a preferred mode of the present invention, the candidate substance is administered to an animal embryo in step A. By observing the amount or function of undifferentiated cells and/or differentiated cells in the animal embryo to which the candidate substance has been administered, it is possible to screen active ingredients effective in an adult.

In a preferred mode of the present invention, the animal is a zebrafish embryo. Zebrafish embryos are useful as screening tools for reasons such as transparency and ease of handling. Active ingredients screened in the screening system using zebrafish embryos also exhibit medicinal effects in mammals.

In a preferred mode of the present invention, in step A, the candidate substance is locally administered to the animal. The local administration is preferable because the observation site of the cells in the subsequent step B and/or step C is determined.

In a preferred mode of the present invention, in step A, the candidate substance is locally administered to an embryo of the animal. By observing the state of undifferentiated cells and/or differentiated cells in the embryonic development process of the locally administered site, screening with high accuracy can be realized.

In a preferred mode of the present invention, in step A, the candidate substance is locally administered to a region occurring in a specific tissue in the embryo of the animal or the vicinity thereof. By observing the state of undifferentiated cells and/or differentiated cells in the embryonic development process of a specific tissue, screening with high accuracy can be realized.

In a preferred mode of the present invention, in step B, undifferentiated cells of the specific tissue are observed. By observing undifferentiated cells of the specific tissue, an influence of the candidate substance on self-renewal ability of the undifferentiated cells can be evaluated.

In a preferred mode of the present invention, in step C, differentiated cells of the specific tissue are observed. By observing differentiated cells of the specific tissue, a differentiation-inducing effect of the candidate substance can be evaluated.

In a preferred mode of the present invention, step A includes locally administering the candidate substance to a region occurring in a specific tissue in the zebrafish embryo or the vicinity thereof, step B includes observing undifferentiated cells of the specific tissue, and step C includes observing differentiated cells of the specific tissue.

As described above, since the zebrafish embryos are transparent and easy to handle, efficient screening can be realized.

In a preferred mode of the present invention, the specific tissue is nervous system, heart, or pancreas. In this way, it may be administered to a tissue of any germ layer system of ectoderm, endoderm, and mesoderm.

In a preferred mode of the present invention, the nervous system is central nervous system.

By adopting a mode in which the candidate substance is administered in a mode that directly acts on the central nervous system, it is possible to effectively screen active ingredients exhibiting a regeneration effect of the central nervous system in which regeneration is impossible or extremely difficult.

In a preferred mode of the present invention, the central nervous system is brain, spinal cord, or optic nerve.

By adopting a mode in which the candidate substance is administered in a mode directly acting on the brain, spinal cord, or optic nerve, it is possible to effectively screen active ingredients exhibiting a regeneration effect of the brain, spinal cord, or optic nerve belonging to the central nervous system in which regeneration is impossible or extremely difficult.

In a preferred mode of the present invention, it is a method for screening active ingredients for treatment or prevention of a disease, disorder, or illness of the same tissue as the specific tissue, or symptoms thereof. By adopting such a mode, it is possible to efficiently screen active ingredients of therapeutic agents of diseases and the like of specific tissues.

In a preferred mode of the present invention, it is a method for screening active ingredients for treatment or prevention of a disease, disorder, or illness of a tissue different from the specific tissue, or symptoms thereof. The active ingredient selected in the screening method of the present invention exhibits a medicinal effect also in a tissue different from the tissue to which the candidate substance is administered in the screening system.

In a preferred mode of the present invention, the animal is a transgenic animal into which a reporter gene specifically expressed in the undifferentiated cell and/or the differentiated cell has been introduced. It is preferable to observe the reporter because undifferentiated cells and/or differentiated cells can be easily observed.

In a preferred mode of the present invention, the animal is a transgenic animal into which a reporter gene specifically expressed in the undifferentiated cell has been introduced, and expression of a reporter gene specifically expressed in the undifferentiated cell in step B is observed.

By adopting such a mode, undifferentiated cells can be easily observed.

In a preferred mode of the present invention, the animal is a transgenic animal into which a reporter gene specifically expressed in the differentiated cell has been introduced, and
expression of a reporter gene specifically expressed in the differentiated cell in step C is observed.

By adopting such a mode, differentiated cells can be easily observed.

In a preferred mode of the present invention, the reporter gene encodes a fluorescent protein or a fusion protein of a fluorescent protein and another protein.

By observing fluorescence of the fluorescent protein, expression of the reporter gene can be easily observed.

In a preferred mode of the present invention, expression of the reporter gene is observed by observing fluorescence of the fluorescent protein.

In a preferred mode of the present invention, an undifferentiated cell marker is observed in step B.

By adopting such a mode, undifferentiated cells can be easily observed.

In a preferred mode of the present invention, a differentiated cell marker is observed in step C.

By adopting such a mode, differentiated cells can be easily observed.

In a preferred mode of the present invention, a means for observing the undifferentiated cell marker and/or the differentiated cell marker is immunostaining or in situ hybridization.

By adopting these observation means, the marker can be easily observed.

In a preferred mode of the present invention, the animal is a transgenic animal into which a reporter gene specifically expressed in the undifferentiated cell has been introduced, expression of a reporter gene specifically expressed in the undifferentiated cell in step B is observed, and a differentiated cell marker is observed in step C.

Undifferentiated cells and differentiated cells can be observed by different means.

In a preferred mode of the present invention, the animal is a transgenic animal into which a reporter gene specifically expressed in the differentiated cell has been introduced, an undifferentiated cell marker is observed in step B, and expression of a reporter gene specifically expressed in the differentiated cell in step C is observed.

Undifferentiated cells and differentiated cells can be observed by different means.

In a preferred mode of the present invention, the animal is a transgenic animal into which a reporter gene specifically expressed in the undifferentiated cell and a reporter gene specifically expressed in the differentiated cell are introduced, expression of a reporter gene specifically expressed in the undifferentiated cell in step B is observed; and expression of a reporter gene specifically expressed in the differentiated cell in step C is observed.

In a preferred mode of the present invention, the candidate substance contains one or more selected from a 5-HT receptor inhibitor, an AChR inhibitor, an adenylate cyclase activator, an AhR activator, an Akt inhibitor, an ALK inhibitor, an ATPase inhibitor, an autophagy inhibitor, a calcium channel inhibitor, a carbonic anhydrase inhibitor, a Cdc42 inhibitor, a CDK inhibitor, a COX inhibitor, a dehydrogenase inhibitor, a DHFR inhibitor, an EGFR inhibitor, an ERK inhibitor, an estrogen/progestogen receptor inhibitor, a γ-secretase inhibitor, a glutamate receptor ligand (potentiator), a GSK-3 inhibitor, an HDAC activator, an HDAC inhibitor, a Hedgehog/Smoothened agonist, an IGF-IR inhibitor, an interleukin receptor inhibitor, an IRAK inhibitor, a JAK/STAT inhibitor, a MAO inhibitor, a MEK inhibitor, a mitophagy inhibitor, an NF-kB inhibitor, an NF-kB-AMPK-tyrosine kinase pathway inhibitor, a Notch-1 inhibitor, a P450 inhibitor, a PAEF inhibitor, a PDE inhibitor, a PPAR inhibitor, a TGF-β receptor inhibitor, a TGF-beta/Smad inhibitor, a TNF receptor inhibitor, and a Wnt/β-catenin pathway activator.

By using a substance having such a primary action as the candidate substance, it is possible to efficiently screen active ingredients.

In a preferred mode of the present invention, the candidate substance contains a substance presumed by in silico method to be one or more selected from a 5-HT receptor inhibitor, an AChR inhibitor, an adenylate cyclase activator, an AhR activator, an Akt inhibitor, an ALK inhibitor, an ATPase inhibitor, an autophagy inhibitor, a calcium channel inhibitor, a carbonic anhydrase inhibitor, a Cdc42 inhibitor, a CDK inhibitor, a COX inhibitor, a dehydrogenase inhibitor, a DHFR inhibitor, an EGFR inhibitor, an ERK inhibitor, an estrogen/progestogen receptor inhibitor, a γ-secretase inhibitor, a glutamate receptor ligand (potentiator), a GSK-3 inhibitor, an HDAC activator, an HDAC inhibitor, a Hedgehog/Smoothened agonist, an IGF-IR inhibitor, an interleukin receptor inhibitor, an IRAK inhibitor, a JAK/STAT inhibitor, a MAO inhibitor, a MEK inhibitor, a mitophagy inhibitor, an NF-kB inhibitor, an NF-kB-AMPK-tyrosine kinase pathway inhibitor, a Notch-1 inhibitor, a P450 inhibitor, a PAEF inhibitor, a PDE inhibitor, a PPAR inhibitor, a TGF-β receptor inhibitor, a TGF-beta/Smad inhibitor, a TNF receptor inhibitor, and a Wnt/β-catenin pathway activator.

Many compounds exhibiting the above-described primary action are known, and knowledge such as the three-dimensional structure of a protein, which is a primary action point of a compound, and a pharmacophore has also been accumulated. Therefore, it is possible to easily search for a compound predicted to exhibit the above-described primary action by computer arithmetic processing. By subjecting such a predicted compound as the candidate substance to the screening method of the present invention, more efficient screening can be realized.

In a preferred mode of the present invention, the candidate substance is a substance that positively controls expression of one or more selected from c-Myc, Sox2, Klf4, and Oct4.

These proteins are known to initiate maintenance of self-renewal ability and pluripotency. By using a substance that positively controls expression of these substances as the candidate substance, it is possible to efficiently screen active ingredients.

In a preferred mode of the present invention, the candidate substance is a substance presumed by in silico method to positively control the expression of one or more selected from c-Myc, Sox2, Klf4, and Oct4.

Compounds that positively control the expression of the above-described four proteins have been mainly searched in vitro and many compounds have been discovered, and knowledge including their primary action points has been accumulated. Therefore, it is possible to easily search for a compound predicted to exhibit the above-described expression control action or primary action by computer arithmetic processing. By subjecting such a predicted compound as the candidate substance to the screening method of the present invention, more efficient screening can be realized.

In a preferred mode of the present invention, the candidate substance is a substance that acts on one or more signaling pathways selected from a Notch signaling pathway, a PI3K/AKT/mTOR signaling pathway, a JAK/STAT signaling pathway, a MAPK signaling pathway, a TGFβ/SMAD signaling pathway, and a Wnt signaling pathway.

These substances acting on the signaling pathways are highly likely to exhibit increasing actions of undifferentiated cells and differentiated cells in vivo. Therefore, by using these substances as the candidate substances, screening efficiency can be improved.

In a preferred mode of the present invention, the candidate substance is a substance presumed by in silico method to act on one or more signaling pathways selected from a Notch signaling pathway, a PI3K/AKT/mTOR signaling pathway, a JAK/STAT signaling pathway, a MAPK signaling pathway, a TGFβ/SMAD signaling pathway, and a Wnt signaling pathway.

Many antagonists and agonists of these signaling pathways are known, and a large amount of knowledge including their primary action points is accumulated. Therefore, it is possible to easily search for a compound predicted to exhibit the above-described action by computer arithmetic processing. By subjecting such a predicted compound as the candidate substance to the screening method of the present invention, more efficient screening can be realized.

In a preferred mode of the present invention, the in silico method is SDBB method and/or LBDD method. Software for realizing the SDBB method and the LBDD method is provided for a fee or for free, and is easy to implement.

In a preferred mode of the present invention, step A includes locally administering the candidate substance to a region occurring in a specific tissue in the embryo of the animal or the vicinity thereof, and step C includes observing function of the specific tissue.

By observing the effect of improving the function, screening with high accuracy can be performed.

Step A includes locally administering the candidate substance to a region occurring in a specific tissue in the embryo of the animal or the vicinity thereof, and
step C includes observing motor function of the specific tissue or a secretion amount of a substance secreted by the specific tissue.

In a preferred mode of the present invention, step A includes locally administering the candidate substance to a region occurring in a heart in the embryo of the animal or the vicinity thereof, and step C includes observing motor function of the heart.

According to the present invention in such a mode, it is possible to efficiently screen active ingredients effective for treatment of heart disease.

In a preferred mode of the present invention, step A includes locally administering the candidate substance to a region occurring in a pancreas in the embryo of the animal or the vicinity thereof, and step C includes observing a production amount or a secretion amount of insulin by β cells.

According to the present invention in such a mode, it is possible to efficiently screen active ingredients effective for treatment of pancreatic disease such as diabetes.

In a preferred mode of the present invention, the animal is a model animal of a disease, disorder, or illness of the nervous system.

By using a model animal of a disease or the like, it is possible to efficiently screen active ingredients exhibiting an effect on the disease.

In a preferred mode of the present invention, it is a method for screening active ingredients for treatment or prevention of glaucoma.

In a preferred mode of the present invention, step A is a step of administering a candidate substance to the retina of the animal, step B is a step of observing precursor cells of retinal ganglion cells or neural stem cells that differentiate into these cells, and step C is a step of observing retinal ganglion cells.

By adopting such a mode, it is possible to accurately screen active ingredients having a therapeutic or preventive effect on glaucoma.

In a preferred mode of the present invention, the animal is a glaucoma model.

By using the glaucoma model as a screening tool, it is possible to efficiently screen active ingredients having a therapeutic or preventive effect on glaucoma.

In a preferred mode of the present invention, it is a method for screening active ingredients for treatment of spinal cord injury.

In a preferred mode of the present invention, step A is a step of administering a candidate substance to the spinal cord of the animal, step B is a step of observing undifferentiated cells in the spinal cord, and step C is a step of observing differentiated cells in the spinal cord.

By adopting such a mode, it is possible to accurately screen active ingredients having a therapeutic effect on spinal cord injury.

In a preferred mode of the present invention, the animal is a spinal cord injury model.

By using the spinal cord injury model as a screening tool, it is possible to efficiently screen active ingredients having a therapeutic effect on spinal cord injury.

The present invention also relates to a method for screening active ingredients for treatment or prevention of a disease, disorder, or illness, or symptoms thereof, including the following step E, step F, and step G.
[step E] a step of administering a candidate substance to a model animal (excluding human) of a disease, disorder, or illness;
[step F] a step of determining a therapeutic effect of a disease, disorder, or illness of the nervous system in the animal that has undergone step E; and
[step G] a step of selecting a candidate substance having a therapeutic effect as the active ingredient.

By directly observing the therapeutic effect in a model animal of a disease or the like, it is possible to accurately screen active ingredients having a therapeutic effect on the disease or the like.

In a preferred mode of the present invention, it is a method for screening active ingredients of a regenerative medicine and/or an anticancer agent.

Provided is a method for screening active ingredients for treatment or prevention of a disease, disorder, or illness of nervous system, heart, or pancreas, or symptoms thereof.

In a preferred mode of the present invention, in step E, a candidate substance is administered to a retina of a glaucoma model animal, and in step F, a therapeutic effect on glaucoma is determined.

By adopting such a mode, it is possible to efficiently screen active ingredients having a therapeutic effect on glaucoma.

In a preferred mode of the present invention, in step F, the presence or absence of an increase in retinal ganglion cells is observed. By adopting such a mode, it is possible to efficiently screen active ingredients that bring about a therapeutic effect for glaucoma by regenerating retinal ganglion cells having damage or functional decline in glaucoma.

In a preferred mode of the present invention, in step F, a therapeutic effect on glaucoma is determined by determining whether or not visual acuity is recovered by behavior observation of the animal.

In a preferred mode of the present invention, it is possible to efficiently screen active ingredients having a therapeutic effect on glaucoma.

In a preferred mode of the present invention, in step E, a candidate substance is administered to a spinal cord injury site in a spinal cord injury model animal, and
in step F, a therapeutic effect on spinal cord injury is determined.

By adopting such a mode, it is possible to efficiently screen active ingredients exhibiting a therapeutic effect on spinal cord injury.

In a preferred mode of the present invention, in step F, a regeneration effect of the spinal cord at the spinal cord injury site is determined.

By adopting such a mode, it is possible to more efficiently screen active ingredients having a therapeutic effect on spinal cord injury.

In a preferred mode of the present invention, in step F, the therapeutic effect on spinal cord injury is determined by determining whether or not exercise capacity is recovered by behavior observation of the animal.

By adopting such a mode, it is possible to efficiently screen active ingredients exhibiting a therapeutic effect on movement disorders accompanying spinal cord injury.

In a preferred mode of the present invention, in step E, a candidate substance is injected into a blood vessel of a cancer model animal, and in step F, a therapeutic effect on cancer is determined.

By adopting such a mode, it is possible to efficiently screen anticancer agents.

In a preferred mode of the present invention, in step E, a candidate substance is administered to a leukemia model animal, and in step F, the presence or absence of thickening or tumorigenesis of vascular endothelium is observed.

By adopting such a mode, it is possible to screen active ingredients exhibiting an anticancer effect by forcibly differentiating cancer cells into normal vascular endothelial cells.

In a preferred mode of the present invention, in step E, a candidate substance is administered to a leukemia model animal, and in step F, survival rate of the animal is observed.

By adopting such a mode, it is possible to efficiently screen active ingredients of an anticancer agent.

The present invention also relates to a method for screening active ingredients for treatment or prevention of a disease, disorder, or illness, or symptoms thereof, in which primary screening is performed by the screening method including above-described step A, step B, and/or step C, and step D, and a candidate substance selected as an active ingredient in the primary screening is subjected to secondary screening by the screening method including the above-described step E, step F, and step G.

By performing the primary screening and the secondary screening in this manner, it is possible to screen active ingredients having a therapeutic effect such as diseases with higher accuracy.

In a preferred mode of the present invention, a candidate substance is administered to a normal animal in step A of the primary screening.

As described above, by adopting a mode in which a normal animal is used in the primary screening and a disease model animal is used in the secondary screening, efficient and highly accurate screening can be realized.

In a preferred mode of the present invention, the candidate substance is a compound included in the following general formula (I), general formula (II), or general formula (III) or a salt thereof or a hydrate of the compound or salt.

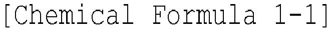

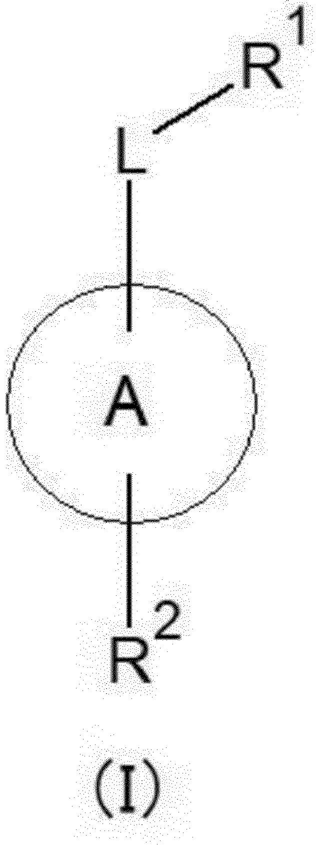

In the general formula (I),
ring A is
a 3- to 8-membered
monocyclic or fused bicyclic group,
which may contain 1 to 4 heteroatoms independently selected from an oxygen atom, a nitrogen atom, and a sulfur atom,
is saturated or unsaturated, and
may be further substituted with a C1-3 alkyl group or a halogen atom;
L is a C1-10 saturated or unsaturated hydrocarbon chain, which may be substituted with substituent R³, or L is absent,
the substituent R³ is a C1-10 saturated or unsaturated hydrocarbon group which may be substituted with a halogen atom, and may have a cyclic structure;
R¹ is
-C=N, -COOH, -CHO, -COOCH₃, -NO₂, or a halogen atom, or
a 3- to 8-membered
monocyclic or fused bicyclic group,
which is
may contain 1 to 4 heteroatoms independently selected from an oxygen atom, a nitrogen atom, and a sulfur atom,
is saturated or unsaturated, and
may be further substituted with a C1-3 alkyl group or a halogen atom;
R² is a 3- to 20-membered monocyclic or fused bicyclic group,
which may contain 1 to 6 heteroatoms independently selected from an oxygen atom, a nitrogen atom, and a sulfur atom,
is saturated or unsaturated, and
may be further substituted with a C1-3 alkyl group or a halogen atom.

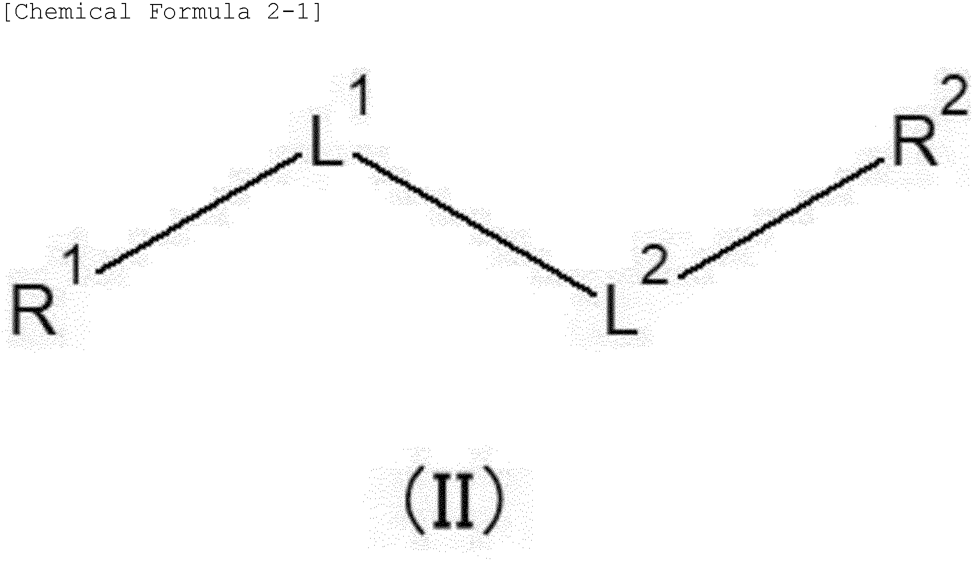

In the general formula (II),
R¹ is
a hydrogen atom,
a halogen atom, or
a C1-20 hydrocarbon group which is saturated or unsaturated, linear or branched, may have a cyclic structure, may be substituted with a halogen atom, and may contain 1 to 4 heteroatoms independently selected from an oxygen atom, a nitrogen atom, and a sulfur atom;
L¹ is any of the following structures: 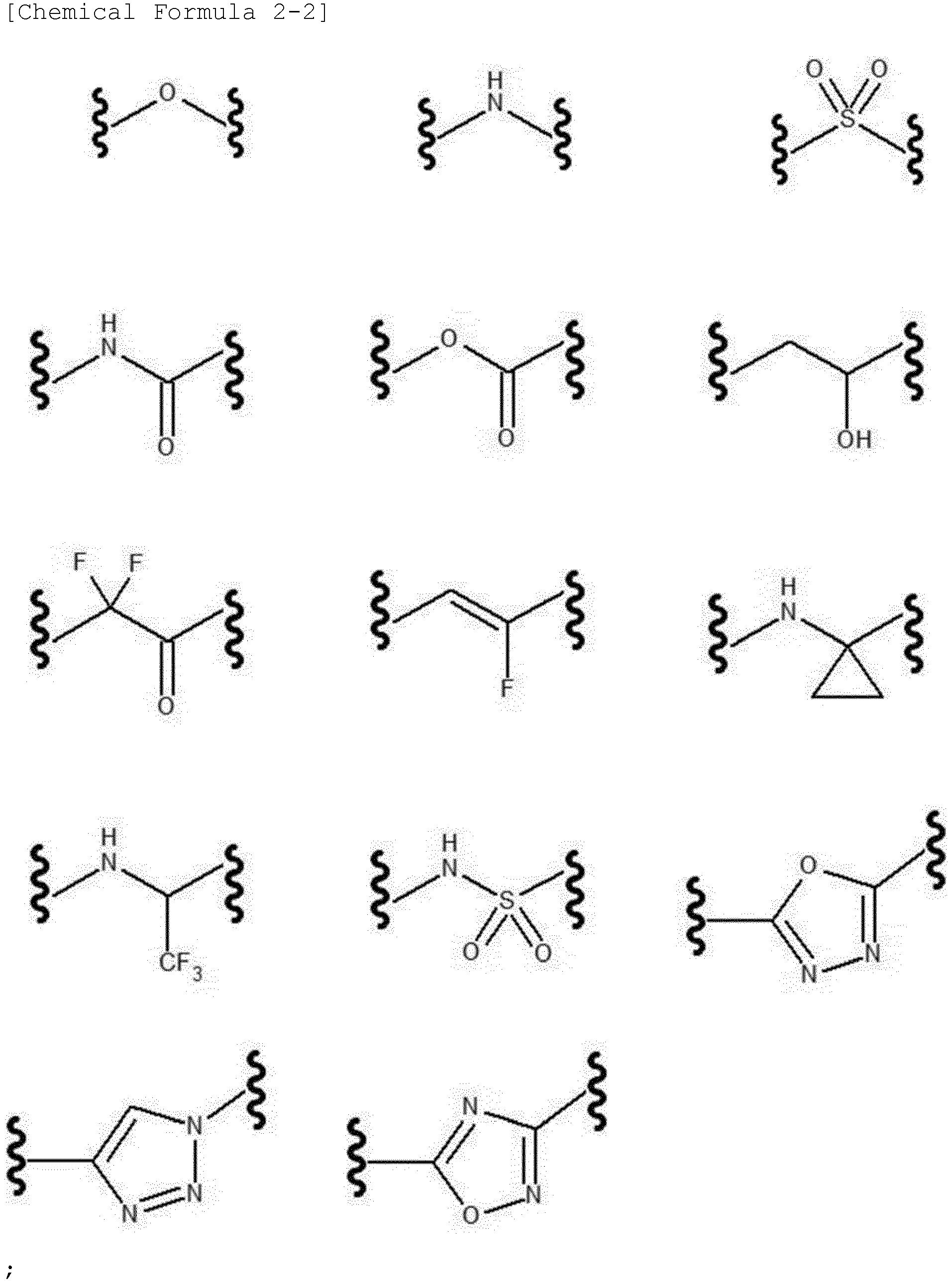
L² is
a C1-30 hydrocarbon chain,
which is saturated or unsaturated,
may be substituted with a group selected from a C1-3 hydrocarbon group, a hydroxyl group which may be substituted with a C1-3 hydrocarbon group, an amino group which may be substituted with a C1-3 hydrocarbon group, a sulfuric acid group which may be substituted with a C1-3 hydrocarbon group, a phosphoric acid group which may be substituted with a C1-3 hydrocarbon group, and a halogen atom, and
may contain 1 to 4 heteroatoms independently selected from an oxygen atom, a nitrogen atom, and a sulfur atom;
R² is a carboxyl group which may be substituted with a C1-3 hydrocarbon group, a hydroxyl group which may be substituted with a C1-3 hydrocarbon group, a hydroxamic acid group which may be substituted with a C1-3 hydrocarbon group, a sulfo group which may be substituted with a C1-3 hydrocarbon group, a boronic acid group which may be substituted with a C1-3 hydrocarbon group, a carbamoyl group which may be substituted with a C1-3 hydrocarbon group, a sulfamoyl group which may be substituted with a C1-3 hydrocarbon group, a sulfoximine group which may be substituted with a C1-3 hydrocarbon group, a cyano group, or a tetrazolyl group.

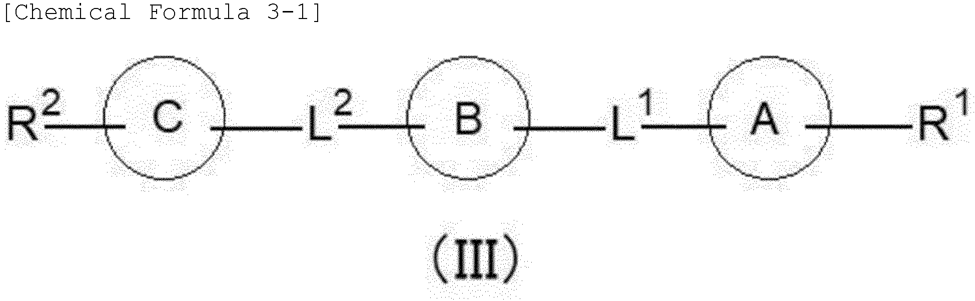

In the general formula (III),
ring A, ring B, and ring C are each independently
a 3- to 8-membered
monocyclic ring
which may contain 1 to 4 heteroatoms independently selected from an oxygen atom, a nitrogen atom, and a sulfur atom,
is saturated or unsaturated, and
further which may be substituted with a C1-3 hydrocarbon group, a halogen atom, a hydroxyl group which may be substituted with a C1-3 hydrocarbon group, an amino group which may be substituted with a C1-3 hydrocarbon group, a sulfuric acid group which may be substituted with a C1-3 hydrocarbon group, or a phosphoric acid group which may be substituted with a C1-3 hydrocarbon group;
R¹ is a group selected from the following: 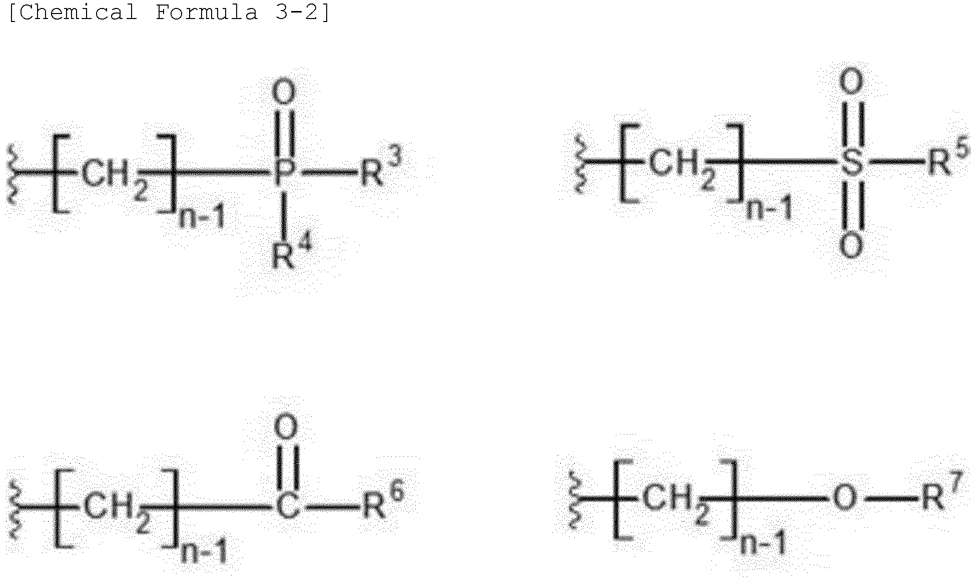
R³ to R⁶ are each independently
a hydrogen atom, a C1-3 hydrocarbon group, a hydroxyl group which may be substituted with a C1-3 hydrocarbon group, or an amino group which may be substituted with a C1-3 hydrocarbon group,
R⁷ is a hydrogen atom, a C1-3 hydrocarbon group, or an amino group which may be substituted with a C1-3 hydrocarbon group, and
n represents an integer of 1 to 4;
L¹ and L² are each independently
a C1-3 alkylene group or alkenylene group, -N(H)-, or -O-,
among these divalent groups, a group other than -O-may be further substituted with a C1-30 hydrocarbon group which is saturated or unsaturated, linear or branched, may have a cyclic structure, may be substituted with a halogen atom, and may contain 1 to 6 heteroatoms independently selected from an oxygen atom, a nitrogen atom, and a sulfur atom; and
R² is a C1-30 hydrocarbon group which is saturated or unsaturated, linear or branched, may have a cyclic structure, may be substituted with a halogen atom, may contain 1 to 6 heteroatoms independently selected from an oxygen atom, a nitrogen atom, and a sulfur atom.

As described above, by using the compounds included in general formulas (I) to (III) as candidate substances for screening, it is possible to screen active ingredients effective for diseases and the like of the nervous system with high probability.

In a preferred mode of the present invention, the candidate substance is a derivative of any one of Compounds 1 to 7 below or a salt thereof or a hydrate of the compound or salt.

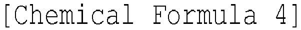

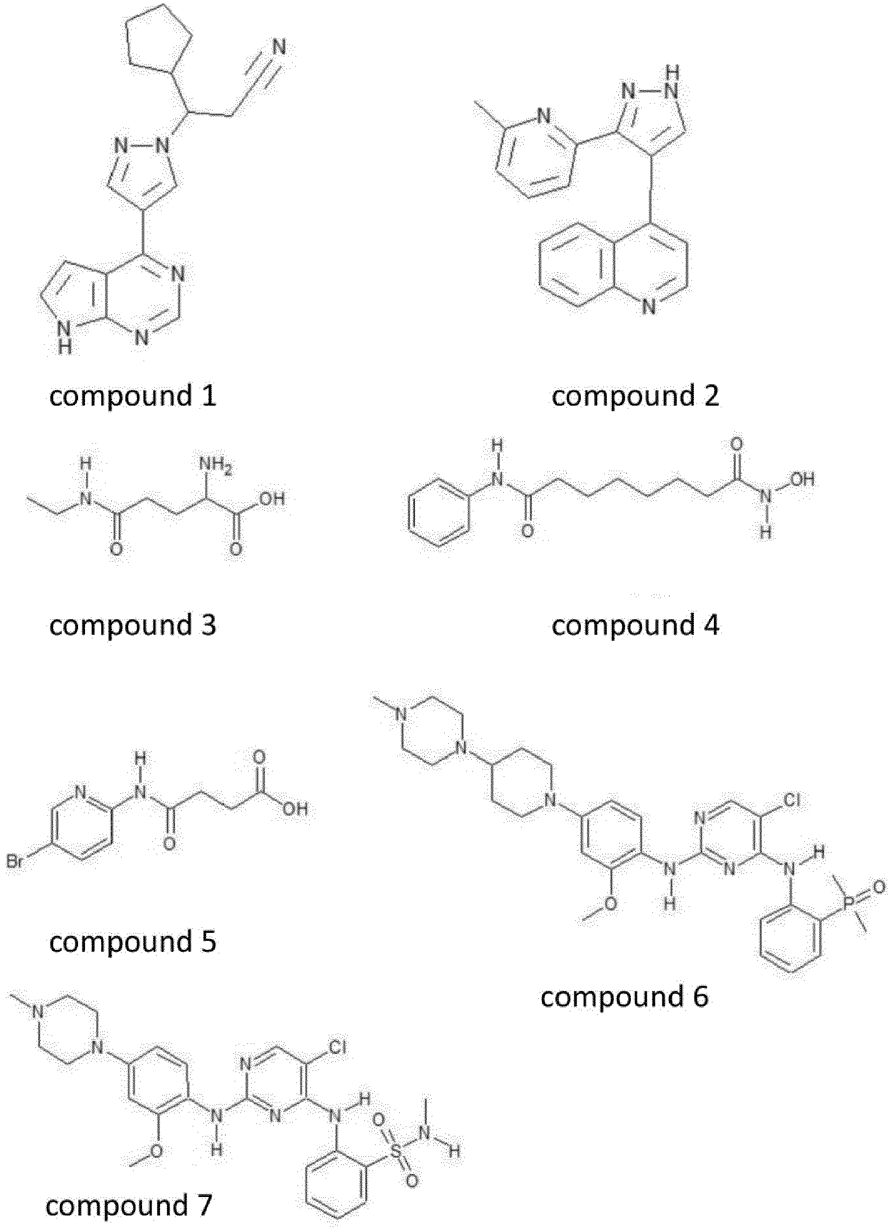

The above seven compounds are compounds selected as active ingredients by the above-described screening method. By using a derivative obtained by substituting, adding, deleting, or the like a structure based on these compounds as a candidate substance for screening, it is possible to screen active ingredients with high probability.

Further, the present invention also relates to a method for producing a pharmaceutical composition, including a formulation step of mixing and formulating a substance selected as an active ingredient and a pharmaceutical additive by the above-described screening method.

According to the production method of the present invention, an effective pharmaceutical composition can be produced.

In a preferred mode of the present invention, the screening method is a screening method using the compounds included in the general formulas (I) to (III) as candidate substances or a screening method using derivatives of the seven compounds as candidate substances.

In a preferred mode of the present invention, the pharmaceutical composition is a regenerative medicine and/or an anticancer agent.

According to the present invention, a regenerative medicine and/or an anticancer agent can be produced.

In a preferred mode of the present invention, the pharmaceutical composition is used for treatment or prevention of a disease, disorder, or illness of nervous system, heart, or pancreas, or symptoms thereof.

In a preferred mode of the present invention, the pharmaceutical composition is used for treatment or prevention of glaucoma.

In a preferred mode of the present invention, the pharmaceutical composition is used for treatment of spinal cord injury.

In a preferred mode of the present invention, the pharmaceutical composition is used for treatment of hematological cancer.

In a preferred mode of the present invention, the pharmaceutical composition is used for treatment of leukemia.

In a preferred mode of the present invention, the pharmaceutical composition is a liquid agent, and
the formulation step includes mixing the active ingredient and an aqueous medium.

In a preferred mode of the present invention, the pharmaceutical composition is an injection.

Also, in a preferred mode of the present invention, the pharmaceutical composition is an eye drop.

The pharmaceutical composition is a lyophilized preparation, and the formulation step includes dissolving the active ingredient in a solvent and freeze-drying the solution.

In a preferred mode of the present invention, the pharmaceutical composition is an ointment, and the formulation step includes mixing the active ingredient and a substrate.

In a preferred mode of the present invention, the pharmaceutical composition is an ophthalmic ointment.

In a preferred mode of the present invention, the pharmaceutical composition is a preparation for nasal administration.

In a preferred mode of the present invention, the pharmaceutical composition is a preparation for oral administration.

The present invention also relates to a method for designing a pharmaceutical composition, including the step of selecting a pharmaceutical additive to be combined with a substance selected as an active ingredient by the above-described screening method.

According to the present invention, an effective pharmaceutical composition can be designed.

In a preferred mode of the present invention, the pharmaceutical composition is a regenerative medicine and/or an anticancer agent.

According to such a mode of the present invention, a regenerative medicine and/or an anticancer agent can be designed.

In a preferred mode of the present invention, the pharmaceutical composition is used for treatment or prevention of a disease, disorder, or illness of nervous system, heart, or pancreas, or symptoms thereof.

According to the present invention, it is possible to design a pharmaceutical composition for treatment or prevention of a disease, disorder, or illness of nervous system, heart, or pancreas, or symptoms thereof.

In a preferred mode of the present invention, the method includes a step of selecting an indication of the pharmaceutical composition. Pharmaceutical additives can be selected according to the selected indication.

In a preferred mode of the present invention, the indication is a disease, disorder, or illness of nervous system, heart, or pancreas, or symptoms thereof, or cancer.

In a preferred mode of the present invention, the method includes a step of selecting a dosage form of the pharmaceutical composition. Pharmaceutical additives can be selected according to the selected dosage form.

In a preferred mode of the present invention, the method includes selecting a liquid agent as the dosage form and selecting an aqueous medium to be mixed with the active ingredient.

In a preferred mode of the present invention, an injection is selected as the dosage form.

In a preferred mode of the present invention, an eye drop is selected as the dosage form.

In a preferred mode of the present invention, a lyophilized preparation is selected as the dosage form.

In a preferred mode of the present invention, the method includes selecting an ointment as the dosage form and selecting a substrate to be mixed with the active ingredient.

In a preferred mode of the present invention, an ophthalmic ointment is selected as the dosage form.

In a preferred mode of the present invention, a preparation for nasal administration is selected as the dosage form.

In a preferred mode of the present invention, a preparation for oral administration is selected as the dosage form.

The present invention also relates to a method including designing a pharmaceutical composition by the method described above, preparing a substance selected as an active ingredient by the above-described screening method, producing a pharmaceutical composition by the production method described above, and statistically processing a data set obtained from persons to whom the pharmaceutical composition was administered in a clinical trial.

### Advantageous Effects of Invention

According to the screening method of the present invention, it is possible to screen active ingredients of medicines, more specifically, active ingredients effective for treatment or prevention of a disease, disorder, or illness of nervous system, heart, or pancreas, or symptoms thereof, or anticancer agents.

According to the production method of the present invention, it is possible to produce a pharmaceutical composition or an anticancer agent effective for treatment or prevention of a disease, disorder, or illness of nervous system, heart, or pancreas, or symptoms thereof.

### Brief Description of Drawings

Fig. 1 is a diagram schematically showing steps of a screening method of the present invention.
Fig. 2 is a diagram schematically showing a mode including step A, step B, and step D.
Fig. 3 is a diagram schematically showing a mode including step A, step C, and step D.
Fig. 4 is a diagram schematically showing a mode including step A, step B, step C, and step D.
Fig. 5 is a diagram which organizes mode including step A, step B, step C, and step D. (a) a mode in which step A, step B, step C, and step D are performed in this order is shown. (b) a mode in which step A, step C, step B, and step D are performed in this order is shown. (c) a mode in which step A is followed by step B and step C simultaneously, and then step D is performed is shown.
Fig. 6 is photographs showing results of Compounds 1 and 2 in Test Example 1. These are photographs in which a fluorescence image obtained by observing fluorescence of GFP indicating mesencephalic neural stem cells and a bright field image are superimposed. The upper stage shows a photograph of a zebrafish embryo in a plan view of the head, and the lower stage shows a photograph in a side view of the head.
Fig. 7 is photographs showing results of Compounds 3 to 5 in Test Example 1. These are photographs in which a fluorescence image obtained by observing fluorescence of GFP indicating mesencephalic neural stem cells and a bright field image are superimposed. The upper stage shows a photograph of a zebrafish embryo in a plan view of the head, and the lower stage shows a photograph in a side view of the head.
Fig. 8 is photographs showing results of Compounds 6 and 7 in Test Example 1. These are photographs in which a fluorescence image obtained by observing fluorescence of GFP indicating mesencephalic neural stem cells and a bright field image are superimposed. The upper stage shows a photograph of a zebrafish embryo in a plan view of the head, and the lower stage shows a photograph in a side view of the head.
Fig. 9 is a diagram showing results of quantitative PCR showing expression levels of sox2 in control and Compounds 2 and 7 in Test Example 1.
Fig. 10 is a diagram showing results of quantitative PCR showing expression levels of sox1α, sox2, sox3, and her6 when control and Compound 2, and a comparative compound in which an increase in neural undifferentiated cells and an increase in nervous system differentiated cells were not observed in Test Example 1 were administered.
Fig. 11 is photographs showing results of Compounds 1 and 2 in Test Example 2. The upper stage shows a bright field image. The lower stage shows a fluorescence image obtained by observing fluorescence indicating cell body clusters of nerves (telencephalon and diencephalon) and axon bundles connecting them.
Fig. 12 is photographs showing results of Compounds 3 to 5 in Test Example 2. These are photographs in which a fluorescence image obtained by observing fluorescence indicating cell body clusters of nerves (telencephalon and diencephalon) and axon bundles connecting them and a bright field image are superimposed.
Fig. 13 is photographs showing results of Compounds 6 and 7 in Test Example 2. The upper stage shows a bright field image. The lower stage shows a fluorescence image obtained by observing fluorescence indicating cell body clusters of nerves (telencephalon and diencephalon) and axon bundles connecting them.
Fig. 14 is photographs showing results of Compound 7 in Test Example 5. The upper stage shows stained images before treatment, and the lower stage shows stained images after treatment. A portion indicated by a black arrow (a portion where green fluorescence is observed) is a retinal ganglion cell.
Fig. 15 is photographs showing results of Compound 2 in Test Example 6. A stained image is shown on the right, and a bar graph showing a result of counting the number of Purkinje cells is shown on the left.
Fig. 16 is photographs showing results of Compound 5 in Test Example 6. A stained image is shown on the right, and a bar graph showing a result of counting the number of Purkinje cells is shown on the left.
Fig. 17 is photographs showing results of Compound 7 in Test Example 6. A stained image is shown on the right, and a bar graph showing a result of counting the number of Purkinje cells is shown on the left.
Fig. 18 is photographs showing fluorescence of mesencephalic neurons in zebrafish injected with Compound 5 in Test Example 7.
Fig. 19 is stained photographs of cardiomyocytes in zebrafish injected with Compounds 2, 5, and 7 in Test Example 8.
Fig. 20 shows results of counting stroke volume of blood cells in zebrafish injected with Compound 7 in Test Example 8.
Fig. 21 shows a captured image of a moving image obtained by photographing heartbeat of the heart of zebrafish injected with Compound 7 in Test Example 8.
Fig. 22 is fluorescent photographs of transgenic zebrafish embryos expressing pancreatic β-cell GFP in Test Example 9.
Fig. 23 is stained photographs of insulin by in situ hybridization of zebrafish injected with a test compound in Test Example 9. The results of injecting Compounds 2, 5, 6, and 7 are shown.
Fig. 24 is immunostaining photographs by an anti-insulin antibody in Test Example 9. The results of injecting Compounds 2 and 6 at a concentration of 100 nM or 5 nM, respectively, are shown. The results of three tests are shown for each condition.
Fig. 25 is a bar graph quantifying the results of Fig. 24. The vertical axis represents average fluorescence intensity.

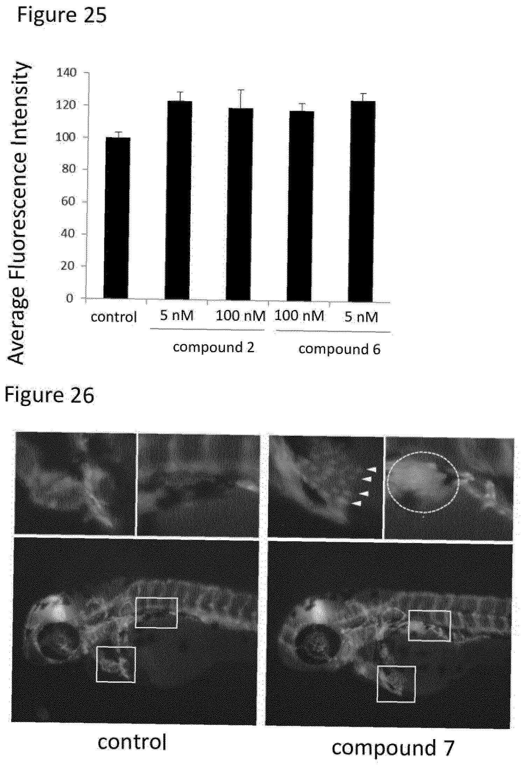

Fig. 26 is a diagram showing results of fluorescence observation of vascular endothelial cells of a T-cell type acute lymphocytic leukemia model zebrafish injected with Compound 7 or E3 Ringer (control) in Test Example 10. The lower diagram shows a low magnification photograph, and the upper diagram shows a photograph in which a portion surrounded by a square in the lower diagram is enlarged. Arrowheads indicate cells that are morphologically blood cell-like but are fusing with the vascular endothelium. A dotted circle indicates blood cells that have thickened or similarly fused with the vascular endothelium.
Fig. 27 is a graph showing survival rates of control and drug-injected individuals in Test Example 10.

### Description of Embodiments

Hereinafter, embodiments of the present invention will be described in detail with reference to the drawings as appropriate. The scope of the present invention is not limited to the embodiments described below.

### [1] First screening method

The screening method of the present invention has been completed based on the fact found as a result of intensive research efforts of the present inventor, that is, the fact that there are a plurality of compounds that realize self-renewal and differentiation induction of undifferentiated cells in vivo with a single agent.

Based on the findings of the present inventor, the screening method of the present invention screens active ingredients of a medicine using an animal as a screening tool and using an effect of promoting proliferation or function of undifferentiated cells and/or differentiated cells as an index.

The "active ingredients of a medicine" as used herein are not limited to an active ingredient of a "medicine" for which a manufacturing and sales license should be obtained from a regulatory agency (the Ministry of Health, Labour and Welfare in Japan, U.S. Food and Drug Administration, and the like), and is a concept broadly encompassing components having useful physiological activity. For example, the "active ingredients of a medicine" in the present specification widely include not only active ingredients of a medicine by Western medicine approach but also active ingredients of a medicine such as Chinese medicine by Oriental medicine approach, a health food, a supplement, and the like.

In one embodiment of the present invention, the present invention is a method for screening active ingredients of a regenerative medicine and/or an anticancer agent. The regenerative medicine as used herein refers to a medicine that acts on stem cells, progenitor cells and the like to promote their proliferation and differentiation, thereby regenerating function of a tissue or organ in which a disorder has occurred. In addition, the anticancer agent is not limited to a medicine that suppresses proliferation of cancer cells or induces cell death. The anticancer agent also includes a medicine having an action of acting on cancer cells to differentiate them into harmless normal cells.

One embodiment of the present invention is a method for screening active ingredients for treatment or prevention of a disease, disorder, or illness of nervous system, heart, or pancreas, or symptoms thereof.

Hereinafter, the "disease, disorder, or illness" is also referred to as "disease or the like".

Examples of the disease or the like of the nervous system include Parkinson's disease; Alzheimer's disease; Creutzfeldt-Jakob disease; Prion disease; corticobasal degeneration; amyotrophic lateral sclerosis; multiple sclerosis; progressive motor weakness; immune neuropathies; central nervous system damage such as brain damage, spinal cord injury, optic nerve damage, and olfactory nerve damage; Alzheimer's disease with parkinsonism; bradykinesia; akinesia; movement disorders that impair detailed motion control and finger dexterity; hypophonia; monotonous utterance; stiffness; dystonia; inflammation associated with Parkinson's disease; tremor of face, chin, tongue, posture; parkinsonian walking; shuffling; brachybasia; festination; mood, cognitive, sensory, sleep disorders; dementia; depression; drug-induced parkinsonism; vascular parkinsonism; multiple system atrophy; progressive supranuclear palsy; disorders with primary tau lesion; corticobasal ganglionic degeneration; parkinsonism with dementia; hyperactivity disorders; chorea; Huntington's disease; dystonia; Wilson's disease; Tourette's syndrome; essential tremor; myoclonus; delayed movement disorder; schizophrenia; bipolar disorder and autism spectrum disorder, and the like.

Examples of the disease or the like of the heart include angina pectoris, myocardial infarction, valvular disease, cardiomyopathy, atrial septal defect, cardiac tumor, heart failure, arrhythmia, and the like.

Examples of the disease or the like of the pancreas include type 1 diabetes, type 2 diabetes, acute pancreatitis, chronic pancreatitis, pancreatic cancer, a mucus-producing tumor, and the like.

In one embodiment of the present invention, there is provided a method for screening active ingredients for treatment of hematological cancer. Examples of the hematological cancer include leukemias such as acute myelogenous leukemia, acute lymphocytic leukemia, chronic myelogenous leukemia and chronic lymphocytic leukemia; Hodgkin lymphoma such as classical Hodgkin lymphoma and nodular lymphocyte predominant Hodgkin lymphoma, malignant lymphomas such as B-cell lymphoblastic leukemia/lymphoma, T-cell lymphoblastic leukemia/lymphoma, chronic lymphocytic leukemia/small lymphocytic lymphoma, follicular lymphoma, MALT lymphoma, lymphoplasmacytic lymphoma, mantle cell lymphoma, diffuse large B-cell lymphoma, Burkitt lymphoma, peripheral T-cell lymphoma, adult T-cell leukemia/lymphoma, extranodal NK/T-cell lymphoma, lymphoma of the skin (mycosis fungoides, Sezary syndrome); and multiple myeloma such as symptomatic multiple myeloma, benign monoclonal hypergammaglobulinemia, asymptomatic myeloma, non-secretory myeloma, and isolated plasmacytoma of bone.

The active ingredient selected by the screening method of the present invention exhibits an action of detoxifying cancer cells by forcibly differentiating them into normal cells (vascular endothelial cells). The present invention is effective in that an active ingredient exhibiting an anticancer effect by such a new mechanism of action can be screened.

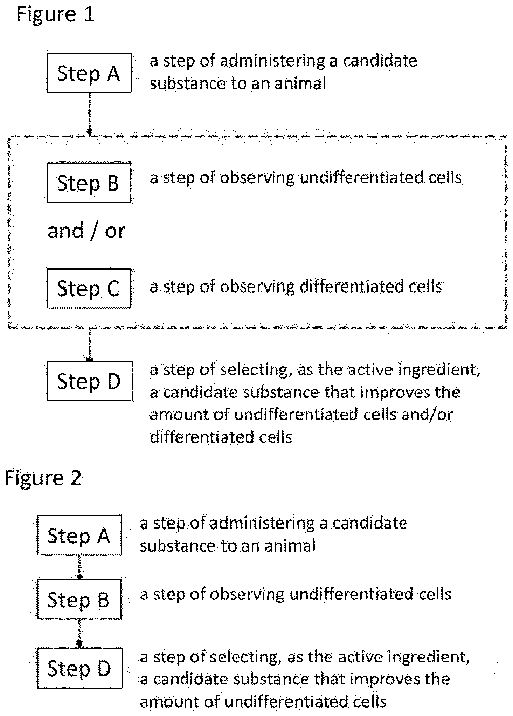

The present invention includes the following step A, step B and/or step C, and step D (Fig. 1).
[step A] a step of administering a candidate substance to an animal (excluding human);
[step B] a step of observing undifferentiated cells in the animal that has undergone step A;
[step C] a step of observing differentiated cells in the animal that has undergone step A; and
[step D] a step of selecting, as the active ingredient, a candidate substance that improves the amount of undifferentiated cells, or a candidate substance that improves the amount of differentiated cells or a function of a tissue composed of differentiated cells, as compared with a case where the candidate substance is not administered.

In the present invention, it is sufficient to perform either step B or step C.

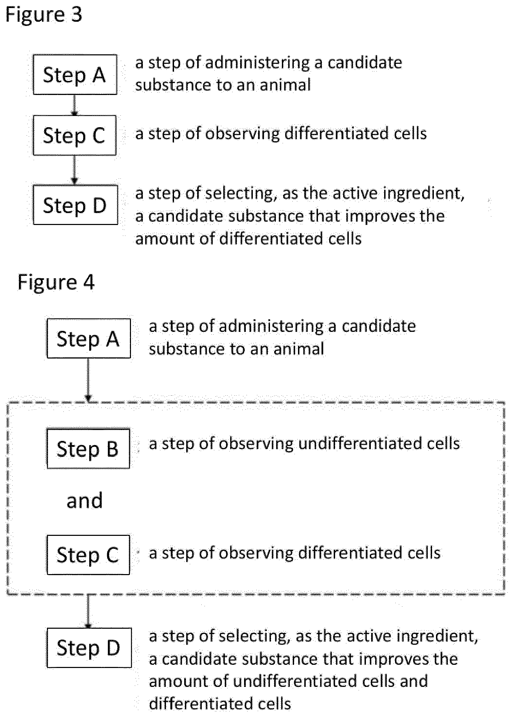

Therefore, an embodiment including step A, step B, and step D may be adopted (Fig. 2), or an embodiment including step A, step C, and step D may be adopted (Fig. 3) .

In the embodiment shown in Fig. 2, in step D, a candidate substance that improves the amount of undifferentiated cells as compared with a case where the candidate substance is not administered is selected as the active ingredient (Fig. 2).

On the other hand, in the embodiment shown in Fig. 3, in step D, a candidate substance that improves the amount of differentiated cells, or a candidate substance that improves the function of the tissue composed of differentiated cells, as compared with a case where the candidate substance is not administered, is selected as the active ingredient (Fig. 3).

In addition, as a matter of course, an embodiment in which both step B and step C are performed may be adopted (Fig. 4).

In this case, in step D, a candidate substance that improves the amounts of neural undifferentiated cells and nervous system differentiated cells as compared with a case where the candidate substance is not administered is selected as the active ingredient (Fig. 4).

In the case of an embodiment in which both step B and step C are performed, the two steps are in no particular order.

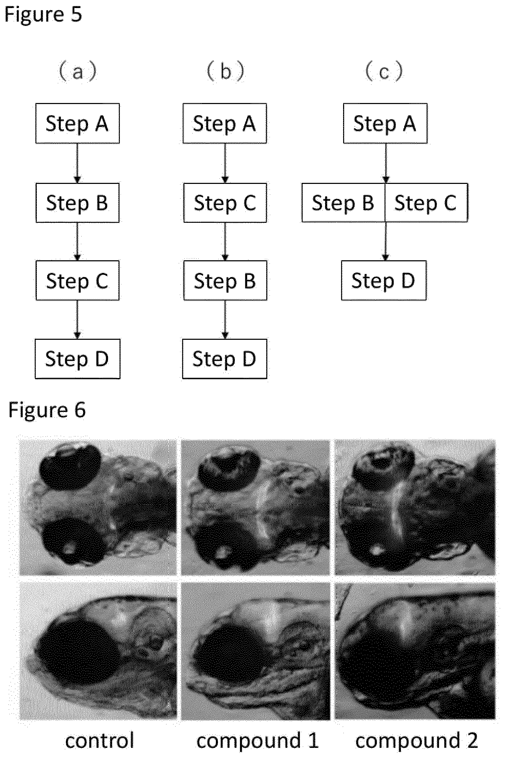

That is, step A, step B, step C, and step D may be performed in this order (Fig. 5(a)), or step A, step C, step B, and step D may be performed in this order (Fig. 5(b)) .

In addition, an embodiment in which step B and step C are simultaneously performed may be employed (Fig. 5(c)).

Hereinafter, each step will be described in detail.

### [1-1] Step A

### [1-1-1] Animals

In step A, the candidate substance is administered to an animal. Since using humans as a screening tool is ethically problematic, the candidate substance is administered to an animal other than humans.

The animal to which the candidate substance is administered in step A is not particularly limited as long as the animal is other than a human, and may be an animal classified into any one of Platyzoa, Lophophorata, Trochozoa, Nematoda, Priapulida, Panarthropoda, Echinodermata, Hemichordata, Cephalochordata, Urochordata, Vertebrata, Xenacoelomorpha, and the like.

Among them, an embodiment in which a vertebrate having a central nervous system including a brain and a spinal cord is used as a screening tool is preferable.

The vertebrate may be any of mammals, fish, birds, reptiles, and amphibians.

Among them, it is preferable to use an animal that is widely used as a model organism. Specifically, examples of the mammal include mouse, guinea pig, rat, rabbit, monkey, dog, and cat. Examples of the fish include zebrafish, killifish, and Japanese puffer. Examples of the birds include chickens and quail. Examples of the reptiles include gecko, and examples of the amphibians include frogs and newts.

From the viewpoint of screening active ingredients that can be easily and quickly applied to humans, it is preferable to use zebrafish that are prolific and have a short life cycle, have a transparent to translucent body, and can observe fluorescence of a fluorescent protein expressed in the body.

In addition, from the viewpoint of easily screening active ingredients having higher applicability to humans, it is preferable to use a mouse which is a mammal animal that is prolific and has a short life cycle.

When zebrafish (including adults and embryos) are used as screening tools, multi-well plates are preferably used. Zebrafish to which different candidate substances are administered are placed in each well. By growing zebrafish to which a plurality of candidate substances are administered on a multi-well plate in this manner, the subsequent step B and/or step C can be easily performed. That is, high-throughput screening becomes possible.

It is particularly preferable to use a transgenic line of zebrafish embryos in which a reporter gene for a fluorescent protein is genetically incorporated as an embodiment in which the zebrafish embryos are grown on a multi-well plate. By adopting such an embodiment, it becomes possible to collectively observe undifferentiated cells and/or differentiated cells by a fluorescence plate reader in step B and/or step C.

The number of wells of the multi-well plate is not particularly limited, but is preferably 6 wells or more, more preferably 12 wells or more, still more preferably 24 wells or more, still more preferably 48 wells or more, and still more preferably 96 wells or more.

The degree of growth of the animal used in step A is not particularly limited. It is preferable to use an embryo in which cell division and differentiation development are active. Hereinafter, an embodiment using an embryo of an animal as a screening tool will be described.

### [1-1-1-1] Screening with embryos

The animal species of the embryo to be used is not particularly limited, but it is preferable to use an embryo of an oviparous animal because an embryo of an embryonic animal exists in a fetus of a mother and is difficult to handle. Specifically, it is preferable to use embryos of fish, reptiles, amphibians, and birds. It is preferable to use an embryo contained in a soft egg with transparent eggshell as a screening tool. It is preferable to use fish embryos and it is particularly preferable to use zebrafish embryos because they are transparent and easy to handle and can be prepared in large quantities.

The method for administering the candidate substance to the embryo is not particularly limited. In one embodiment, the embryo is immersed in a liquid in which the candidate substance is dissolved or dispersed, and the whole embryo is exposed to the candidate substance.

In a more preferred embodiment, the candidate substance is administered locally to a portion of the embryo. In this case, the candidate substance is locally administered to a region occurring in a specific tissue in the animal embryo or the vicinity thereof.

The term "vicinity" refers to a range in which a candidate substance can reach a target region by diffusion or dispersion by a body fluid.

Local administration to the animal embryo can be carried out by a conventional method using a micromanipulator. In addition, it may be effective to adopt the in vivo lipofection method developed by the present inventor for local administration of a molecule having a negative charge due to ionization or the like (Non Patent Literature 12).

The above-described "specific tissue" is not particularly limited. The tissue may be derived from any of ectoderm, endoderm, and mesoderm.

The candidate substance may be administered to a region occurring in any tissue of the ectoderm system such as skin epidermis, hair, nail, skin gland (including mammary gland, sweat gland), sensory organ (including the epithelium at the end of the oral cavity, pharynx, nose, rectum), salivary gland, lens, brain, or spinal cord, or the vicinity thereof.

The candidate substance may be administered to a region occurring in any tissue of the endoderm system such as the digestive tract from the esophagus to the large intestine (excluding the end of the oral cavity/pharynx and rectum), lung, thyroid gland, pancreas, liver, cells of secretory glands opening into the digestive tract, peritoneum, pleura, larynx, auditory canal, trachea, bronchus, or urinary tract (bladder, most of urethra, part of ureter), or the vicinity thereof.

The candidate substance may be administered to a region occurring in any tissue of the endoderm system such as body cavity and mesothelium lining the body cavity, muscle, skeleton, skin dermis, connective tissue, heart, blood vessel (also including vascular endothelium), blood (also including blood cells), lymphatic vessel or spleen, kidney and ureter, or gonadal gland (testis, uterus, gonadal epithelium), or the vicinity thereof.

The active ingredient selected in the embodiment in which the candidate substance is administered to a region occurring in a specific tissue or the vicinity thereof is not an active ingredient effective only for the specific tissue. The active ingredient selected in the screening method of the present embodiment is also an active ingredient for a tissue different from the specific tissue. That is, the active ingredient selected in the screening method of the present invention can be used as a regenerative medicine and/or an anticancer agent with no limitation of application range. Therefore, the present invention can take the following embodiments.

One embodiment of the present invention is a method for screening active ingredients for treatment or prevention of a disease, disorder, or illness of the same tissue as a specific tissue into which a candidate substance is locally injected, or symptoms thereof.

One embodiment of the present invention is a method for screening active ingredients for treatment or prevention of a disease, disorder, or illness of a tissue different from the specific tissue into which a candidate substance is locally injected, or symptoms thereof.

For example, in a case where a candidate substance is locally administered to a region occurring in the nervous system in the embryo or the vicinity thereof, and an increase in neural undifferentiated cells/nervous system differentiated cells is observed, the candidate substance should not be regarded as an ingredient effective only for the disease or the like of the nervous system. In this case, the candidate substance is understood to be an ingredient effective as a regenerative medicine for tissues and organs other than the nervous system. In addition, this candidate substance is understood to be an ingredient which is also effective as an anticancer agent. This is supported by the results of the test examples described later.

Hereinafter, embodiments in which a candidate substance is administered to a region occurring in the nervous system, heart, or pancreas or the vicinity thereof will be described in detail.

### [1-1-1-1-1] Local administration to region occurring in nervous system or vicinity thereof

In one embodiment of the present invention, the candidate substance is locally administered to a region occurring in the nervous system in the embryo of the animal or the vicinity thereof. Hereinafter, the developmental process of the nervous system in the embryonic development stage will be briefly described, and then the embodiment will be described.

Almost all animals such as sea urchins, fruit flies, fish, frogs, mice, and humans undergo a process called gastrulation in an early embryonic stage. At gastrulation stage, pluripotent cells invaginate toward the center of the embryo (gastrulation). The cells thus migrated later form layers of mesoderm and endoderm, which are highly differentiated embryonic cells. Mesoderm forms blood vessels and the musculoskeletal system, and endoderm forms digestive tract and its associated internal organs. On the other hand, the ectodermal region is destined to be differentiated as epidermis by the activity of bone morphogenetic protein 4 (BMP4). However, the ectodermal region where a former-derived BMP inhibitory molecule acts during the gastrulation stage is destined as a future neural tissue from which a neural plate is formed.

The neural plate has a map of future brain. Behavior of the cells is determined according to the map where each region of the brain such as the cerebrum, diencephalon, midbrain, and cerebellum is formed, and the brain is formed. This region-dependent fate mechanism is called "regionalization" or "patterning". This phenomenon involves expression of specific transcription factors induced by molecules derived from local organizers, an expression suppression mechanism between the transcription factors, and a cell sorting mechanism depending on the function of cell surface molecules induced by the transcription factors.

The midportion of the neural plate is recessed into a neural groove, and both sides of the neural plate protrude to the amniotic cavity side and eventually fuse at the end to form a neural tube. Neural tube formation begins in the neck region of the embryo and proceeds both anterior-posterior. Closure of the front part also starts from the lateral head end. A portion that is not yet closed in this process and leads to the amniotic cavity is called cranial neuropore, which gradually becomes smaller. At this time, the anterior portion of the neural tube is observed to be bulging more than the posterior portion. This bulge is called a brain vesicle (primary brain vesicle), and since it has three swelling parts, this developmental stage is also called a three brain vesicle stage (primary brain vesicle stage). The swelling includes prosencephalon, mesencephalon, and rhomboencephalon (or metencephalon) from the front. The wall of the swelling differentiates the neural tissue and the lumen becomes ventricle.

Thereafter, the prosencephalon is distinguished into telencephalon and diencephalon, and the rhomboencephalon is distinguished into metencephalon and myelencephalon to form a secondary brain vesicle. The telencephalon expands left and right, and the lumen becomes lateral ventricle. The diencephalon lumen and the metencephalon lumen becomes the third ventricle and the fourth ventricle, respectively. The mesencephalon lumen becomes the cerebral aqueduct without significant morphological change. The dorsal side of the telencephalon becomes the cerebral cortex, and the basal ganglia and the like are differentiated from the ventral side. From the diencephalon region, epithalamus, thalamus, and hypothalamus are differentiated. The dorsal side of the metencephalon region gives rise to the cerebellum, and the ventral side forms the pons. The myelencephalon region becomes the medulla oblongata and leads to the spinal cord formed from the posterior part of the neural tube.

In the initial stage of morphogenesis, a pair of cavities starts to develop from the lateral side of the forebrain. Before the anterior end of the neural tube closes, this cavity begins to form the optic vesicle, the anlage of the eye, and after the closure of the neural tube ends, the optic vesicle is formed.

Based on the above, one mode of the "region occurring in the nervous system in the embryo of the animal" includes a neural plate forming predetermined region at the gastrulation stage. That is, in step A, an embodiment may be employed in which the candidate substance is locally administered to the neural plate forming predetermined region or the vicinity thereof at the gastrulation stage.

In addition, one mode of the "region occurring in the nervous system in the embryo of the animal" includes a neural plate. That is, in step A, an embodiment may be employed in which the candidate substance is locally administered to the neural plate or the vicinity thereof.

Moreover, one mode of the "region occurring in the nervous system in the embryo of the animal" includes a neural tube. That is, in step A, an embodiment may be employed in which the candidate substance is locally administered to the neural tube or the vicinity thereof.

In this case, it may be locally administered to a condition having a neuropore, that is, a neural tube that is not completely closed, or it may be locally administered to a neural tube that is completely closed.

In addition, in a mode of local administration to the neural tube, it may be locally administered to a predetermined region of the brain in front of the neural tube, or may be locally administered to a predetermined region of the spinal cord behind the neural tube.

Further, one mode of the "region occurring in the nervous system in the embryo of the animal" includes a primary brain vesicle. That is, in step A, an embodiment may be employed in which the candidate substance is locally administered to the primary brain vesicle or the vicinity thereof.

The primary brain vesicle to be locally administered may be any of prosencephalon, mesencephalon, and rhomboencephalon. Alternatively, it may be locally administered to the lumen (ventricle) of the primary brain vesicle.

Furthermore, one mode of the "region occurring in the nervous system in the embryo of the animal" includes a secondary brain vesicle. That is, in step A, an embodiment may be employed in which the candidate substance is locally administered to the secondary brain vesicle or the vicinity thereof.

The secondary brain vesicle to be locally administered may be any of telencephalon, diencephalon, metencephalon, and myelencephalon. In addition, it may be locally administered to the lateral ventricle, the third ventricle, the fourth ventricle, or the cerebral aqueduct which is the lumen of the secondary brain vesicle.

In addition, one mode of the "region occurring in the nervous system in the embryo of the animal" includes an optic vesicle. That is, in step A, an embodiment may be employed in which the candidate substance is locally administered to the optic vesicle or the vicinity thereof.

### [1-1-1-1-2] Local administration to region occurring in heart or vicinity thereof

In one embodiment of the present invention, the candidate substance is locally administered to a region occurring in the heart in the embryo of the animal or the vicinity thereof. Hereinafter, the developmental process of the heart in the embryonic development stage will be briefly described, and then the embodiment will be described.

Cardiogenesis begins with determination of left-right axis in a primitive nodule, and its information is transmitted to the left and right lateral plate mesoderm. In cardiac primordia (cardiogenic region) of the head-side part, immature mesoderm cells differentiate into cardiac progenitor cells that express myocardial-specific transcription factors. The cardiac progenitor cells gradually move to the center of the embryo while being further differentiated to become cardiac myocytes, and form one primitive heart tube in the midline. The primitive heart tube contracts in a peristaltic manner, gradually begins rhythmic contractions, and flexible looping (cardiac looping) to the right of the embryo to form the outer shape of the heart. In the primitive heart tube, from the caudal side to the cranial side, each component is determined in the order of a sinus venosus, a primitive atrium, a primitive ventricle, a bulbus cordis, and a truncus arteriosus. These anterior-posterior segments transform into a left-right positional relationship, particularly in the ventricular portion, as a heart loop is formed. At the same time, the ventricle hangs downward as the heart tube extends, and the vertical relationship of the atrium and the ventricle is reversed. On the other hand, inside the primitive heart tube, four endocardial cushions on the upper, lower, left, and right are developed in the atrioventricular tube portion, and two atrioventricular valves and a part of the ventricular atrial septum are formed. In an outflow channel, two (initially four) conotruncal swellings develop, spirally dividing the pulmonary artery and the aorta. Further, the atrial septum and the ventricular septum are developed and completed.

Previous studies have revealed that a part of the mesoderm cells expressing transcription factor MESP1 differentiates into cardiac muscle. However, it is known that cells expressing MESP1 are differentiated not only into cardiomyocytes but also into cells other than the heart including vascular endothelial cells, vascular smooth muscle cells, and extraembryonic mesoderm constituting placenta. It has been found that, among the mesoderm cells expressing MESP1, cardiomyocytes subsequently develop from a cell population expressing transcription factor NKX2.5. Since this cell population will mostly contribute to the heart in the future, these NKX2.5 positive mesodermal components are considered to be cardiac progenitor cells.

Furthermore, it has been revealed that many transcription factors such as TBX5, GAT A family, ISLET-1, HAND1/2, and MEF2C are involved in cardiomyocyte differentiation while interacting with one another as transcription factors that play an important role in cardiogenesis.

Based on the above, one mode of the "region occurring in the embryo of the animal" includes cardiac primordia formed at an early stage of development. That is, in step A, an embodiment may be employed in which the candidate substance is locally administered to the cardiac primordia or the vicinity thereof.

In addition, one mode of the "region occurring in the embryo of the animal" includes a region where immature mesoderm cells that differentiate into cardiac muscle progenitor cells are localized or a region where cardiac muscle progenitor cells are localized. That is, in step A, an embodiment may be employed in which the candidate substance is locally administered to a region where immature mesoderm cells differentiated into cardiac progenitor cells are localized or a region where cardiac progenitor cells are localized or the vicinity thereof.

Further, one mode of the "region occurring in the embryo of the animal" includes a primitive heart tube. That is, in step A, an embodiment may be employed in which the candidate substance is locally administered to the primitive heart tube or the vicinity thereof.

Furthermore, one mode of the "region occurring in the embryo of the animal" includes a primitive heart tube. That is, in step A, an embodiment may be employed in which the candidate substance is locally administered to the primitive heart tube or the vicinity thereof.

### [1-1-1-1-3] Local administration to region occurring in pancreas or vicinity thereof

In one embodiment of the present invention, the candidate substance is locally administered to a region occurring in the pancreas in the embryo of the animal or the vicinity thereof. Hereinafter, the developmental process of the pancreas in the embryonic development stage will be briefly described, and then the embodiment will be described.

The pancreas is composed of two types of tissues with completely different roles. One is an exocrine tissue that produces and secretes digestive enzymes and releases them into digestive tract through a conduit, and the other is an endocrine tissue that produces and secretes hormones and releases them into the circulation. There are multiple types of cells in each tissue, but it is known that all differentiate from common progenitor cells in development.

Tubular archenteron are formed from endoderm, which is a single layer of sheet-like cells that appear at an early stage of development. All cells constituting the pancreas are derived from Pdx1-positive cells. More particularly, all cells constituting the pancreas are derived from pancreatic buds (pancreatic primordia), which are formed from endoderm epithelial cells that reside in the posterior foregut of the archenteron (archenteron foregut). The pancreatic buds (pancreatic primordia) protrude from the dorsal side of the posterior foregut and slightly later from the ventral side. Pancreatic bud cells proliferate and form highly branched pancreatic epithelium. The pancreatic epithelium then differentiates into endocrine cells (α cells, β cells, δ cells, PP cells, and the like), pancreatic ductal cells, and exocrine cells (acinar cells). The pancreatic buds protruding from the dorsal and ventral sides of the posterior foregut swirl and fuse, and eventually become one pancreas.

Based on the above, one mode of the "region occurring in the pancreas in the embryo of the animal" includes a pancreatic bud (pancreatic primordium). That is, in step A, an embodiment may be employed in which the candidate substance is locally administered to the pancreatic bud or the vicinity thereof.

In addition, one mode of the "region occurring in the pancreas in the embryo of the animal" includes a pancreatic epithelium. That is, in step A, an embodiment may be employed in which the candidate substance is locally administered to the pancreatic epithelium or the vicinity thereof.

### [1-1-1-2] Screening using adult and the like

In one embodiment of the present invention, an animal embryo from a late stage of development to new phase stage that has completed a whole developmental process, and the animal from a young individual to an adult are used as screening tools.

In this case, in step A, the candidate substance is administered to the animal. Examples of the mode of administration include local administration by injection or the like, oral administration, transdermal administration, enteral administration, and the like. In a preferred embodiment, the candidate substance is locally administered to the animal.

When the candidate substance is locally administered, the administration site is not particularly limited. Preferred examples thereof include an embodiment in which the candidate substance is locally administered to the nervous system heart and the pancreas. Hereinafter, local administration to the nervous system will be particularly described.

The nervous system may be either the central nervous system or the peripheral nervous system. Once the central nervous system undergoes a disorder, the central nervous system is hardly regenerated, and the disorder is maintained. For the purpose of screening regenerative medicines for the central nervous system which have been considered not to be regenerated by such conventional common knowledge, it is preferred that the candidate substance is locally administer to the central nervous system in step A.

The central nervous system to which the candidate substance is locally administered in step A may be any of the brain, spinal cord, optic nerve, and olfactory nerve. Preferably, the candidate substance is locally administered to the brain, spinal cord, optic nerve, or the vicinity thereof.

In the case of local administration to the brain, more specifically, a mode of local administration into the ventricle is preferable. The local administration to the brain is preferably carried out in the mode of administration by injection. The injection may be performed manually, but it is preferable to perform the injection after controlling the dose using an injection pump. Also, when the animal to be injected is small, a micromanipulator may be used.

The local administration to the spinal cord is preferably carried out in the mode of administration by injection. The injection may be performed manually, but it is preferable to perform the injection after controlling the dose using an injection pump. Also, when the animal to be injected is small, a micromanipulator may be used.

In the case of local administration to the optic nerve, a mode of local administration to retinal ganglion cells or the vicinity thereof is more preferable. Although the candidate substance may be administered to retinal ganglion cells or the vicinity thereof by injection into the eyeball, a mode in which the candidate substance is administered by dropping or applying to the eyeball is preferable. When the animal to which the candidate substance is to be administered is a terrestrial animal (more specifically mammals, more specifically a mouse), the candidate substance is preferably administered by eye drops.

### [1-1-1-3] Disease model

In one embodiment of the present invention, a candidate substance is administered to a normal animal not suffering from a disease or the like. Since normal animals are easily and inexpensively available in large quantities as compared with the disease model described later, it is advantageous when screening a large number of candidate substance groups.

Also, in one embodiment of the present invention, the animal may be a model animal of a disease or the like. The disease model animal may be a genetic model, a drug induced model, or an injury model that physically damages tissues, organs, or cells.

By using a disease model animal, it is possible to efficiently screen active ingredients exhibiting an effect in application to the disease.

Examples of the model animal of the central nervous system diseases include depression model animals such as learned helplessness model (mouse) and olfactory bulbectomy depression model (rat); dementia model animals such as Tg2576 mouse, APP & PS1 double transgenic (PSAPP) mouse, methamphetamine/chlordiazepoxide-induced hyperlocomotion model (mouse), cholinergic nucleus destruction model (mouse), drug-induced memory disorder model (mouse), and brain local destruction model (rat); schizophrenia model animals such as neonatal phencyclidine (PCP)-administered mouse, NR1 knockdown mouse, G protein-coupled receptor (SREB2) knockout mouse, SREB2 overexpression mouse, and drug-induced catalepsy (rat); Parkinson's disease model animals such as 1-methyl-4 phenyl-1,2,3,6-tetrahydropyridine (MPTP)-induced Parkinson's disease model (mouse); epilepsy models such as spasm model (mouse) and amygdalar nucleus or hippocampus kindling model (rat); nerve injury models (rat, mouse, zebrafish) such as spinal cord injury model due to spinal cord crush, nerve injury model (rat, mouse) due to sciatic nerve crush or cutting; cerebral infarction models (mouse) due to middle cerebral artery occlusion, and the like.

In addition, examples of the disease model animal of the optic nerve include glaucoma model animals such as a Vav gene-deficient model (mouse), a GLAST-deficient normal intraocular pressure glaucoma model (mouse), and a high intraocular pressure model (rat) by cauterization of episcleral vein, and the like.

A model animal of spinal cord injury can be easily created by applying physical pressure to the spinal cord of a normal animal or by piercing the spinal cord.

### [1-1-2] Candidate substance

There is no limitation on the candidate substance to be administered to the animal in step A. The candidate substance may be any of a low molecular compound, a protein, a peptide, and a nucleic acid.

In one embodiment of the present invention, the candidate substance is a low molecular compound. In the present specification, the "low molecular compound" refers to a compound having a molecular weight of 2000 or less, more preferably a compound having a molecular weight of 1500 or less, and more preferably a compound having a molecular weight of 1000 or less.

In one embodiment of the present invention, the candidate substance is a protein. The type of the protein is not particularly limited. The protein may be a protein extracted from a cell or a protein prepared by genetic engineering technique. In addition, the protein may be a fusion protein in which a plurality of proteins are fused, or a protein containing a non-natural amino acid as a constituent element.

In one embodiment, the candidate substance is an antibody. The antibody may be a polyclonal antibody or a monoclonal antibody. According to this embodiment, antibody drugs can be screened.

In one embodiment of the present invention, the candidate substance is a peptide. The number of amino acids constituting the peptide is not particularly limited, and examples thereof include peptides composed of 2 to 100 amino acids.

The peptide may be extracted from cells or prepared by genetic engineering techniques or chemical synthesis. In addition, a non-natural amino acid may be contained as an amino acid constituting the peptide.

In one embodiment of the present invention, the candidate substance is a nucleic acid. The nucleic acid may be any of deoxyribonucleic acid (DNA), ribonucleic acid (RNA), and a chimeric nucleic acid thereof.

In one embodiment of the present invention, the candidate substance is an expression vector expressing a protein or RNA.

In one embodiment of the present invention, the candidate substance is a knockdown vector that suppresses expression of a specific protein in a cell by RNA interference.

In one embodiment of the present invention, the candidate substance is a peptide nucleic acid (PNA).

Moreover, in one embodiment, the candidate substance does not contain a nucleic acid. Further, in one embodiment, the candidate substance does not contain a protein. Furthermore, in one embodiment, the candidate substance does not contain a peptide nucleic acid.

In one embodiment of the present invention, the candidate substance contains one or more selected from the following group A.
[Group A]
a 5-HT receptor inhibitor, an AChR inhibitor, an adenylate cyclase activator, an AhR activator, an Akt inhibitor, an ALK inhibitor, an ATPase inhibitor, an autophagy inhibitor, a calcium channel inhibitor, a carbonic anhydrase inhibitor, a Cdc42 inhibitor, a CDK inhibitor, a COX inhibitor, a dehydrogenase inhibitor, a DHFR inhibitor, an EGFR inhibitor, an ERK inhibitor, an estrogen/progestogen receptor inhibitor, a γ-secretase inhibitor, a glutamate receptor ligand (potentiator), a GSK-3 inhibitor, an HDAC activator, an HDAC inhibitor, a Hedgehog/Smoothened agonist, an IGF-IR inhibitor, an interleukin receptor inhibitor, an IRAK inhibitor, a JAK/STAT inhibitor, a MAO inhibitor, a MEK inhibitor, a mitophagy inhibitor, an NF-kB inhibitor, an NF-kB-AMPK-tyrosine kinase pathway inhibitor, a Notch-1 inhibitor, a P450 inhibitor, a PAEF inhibitor, a PDE inhibitor, a PPAR inhibitor, a TGF-β receptor inhibitor, a TGF-beta/Smad inhibitor, a TNF receptor inhibitor, and a Wnt/β-catenin pathway activator

As shown in the test examples herein, a compound selected as an active ingredient by the screening method of the present invention has the above-described primary action. If a compound has a primary action common to that of a compound selected as effective in the test examples herein, there is a high probability that the compound is selected as an effective compound by the screening method of the present invention. Therefore, it is preferable to subject the compound having the primary action described above to step A as a candidate substance in order to realize high-efficiency screening.

As the candidate substance, a known compound having the above-described primary action may be used. Since primary action points (targets) of the compounds are organized in various databases and registered in a searchable state, it is preferable to use the primary action points (targets). Examples of such a compound database include PubChem (http://pubchem.ncbi.nlm.nih.gov/), ChemBank (http://chembank.broadinstitute.org/welcome.htm), myPresto (http://presto.protein.osaka-u.ac.jp/myPresto4/), LigandBox (http://ligandbox.protein.osaka-u.ac.jp/ligandbox/), ZINC (http://zinc.docking.org/), ChEBI (http://www.ebi.ac.uk/chebi/), ChEMBL (https://www.ebi.ac.uk/chembl/), CTD Comparative Toxicogenomics Database (http://ctd.mdibl.org/), ChemSpider (http://www.chemspider.com/), ChemCupid (http://www.namiki-s.co.jp/chemcupid/), Nikkaji (http://nikkajiweb.jst.go.jp/nikkaji_web/pages/top.html), KEGG LIGAND (http://www.genome.jp/kegg/ligand.html), DrugBank (http://www.drugbank.ca/), SuperDrug (http://bioinformatics.charite.de/superdrug2/), SuperTarget (http://bioinf-apache.charite.de/supertarget/), SuperNatural (http://bioinformatics.charite.de/supernatural2/), PharmGKB (http://www.pharmgkb.org/), Het-PDB Navi, STITCH (http://hetpdbnavi.nagahama-i-bio.ac.jp/), GLIDA (http://pharminfo.pharm.kyoto-u.ac.jp/services/glida/), HIC-Up (http://xray.bmc.uu.se/hicup/), Dundee Prodrg2 (http://davapc1.bioch.dundee.ac.uk/prodrg/), and the like.

In one embodiment of the present invention, the candidate substance contains a substance presumed by in silico method to be one or more selected from the group A.

Since screening using cultured cells or experimental animals is costly and laborious, it is not realistic to provide all of the available compound libraries as candidate substances. Therefore, in silico screening using a computer is widely used in modern drug discovery. Also in the present invention, it is preferable to use, as a candidate substance, a compound that is presumed by in silico to be a compound having the primary action.

The presumption by the in silico method will be described in detail. In order to perform in silico drug discovery, it is necessary to model a phenomenon that occurs with respect to a medicine in vivo, in a cell, or in a test tube in the real world using some theory. When classified by the type of modeling theory used, it can be roughly classified into an informatics method and a simulation method.

The informatics method is also called a LBDD method (Ligand-Based Drug Discovery) mainly using structure-activity relationship information on a medicine (ligand) side, and is modeled by machine learning based on a similarity index and statistical theory. Machine learning is also an elemental technology of artificial intelligence.

The simulation method is a method of simulating an interaction between a protein and a medicine on a computer using structural information of the protein on the basis of theories such as classical molecular force field, quantum mechanics and molecular dynamics, and is called SBDD (structure-based drug discovery) because it is based on the structure of the protein.

The information on the three-dimensional structure of the protein is, for example, registered in a database such as PDBj (https://pdbj.org/), RCSB DB (https://www.rcsb.org/), BMRB (https://bmrb.io/), or PDBe (https://www.ebi.ac.uk/pdbe/), and thus can be used. In addition, the three-dimensional structure may be analyzed by newly performing NMR, X-ray crystal structure analysis, or the like. Moreover, the three-dimensional structure of the protein may be predicted from the amino acid sequence, and this may be used for the SBDD method.

In one embodiment of the present invention, the candidate substance contains a substance presumed by the LBDD method to be one or more selected from the group A. That is, a compound having a structure predicted to have the same primary action is used as a candidate substance with reference to structure-activity relationship information based on a plurality of compounds (ligands) already known to have the primary action of the group A. The structure-activity relationship information can be modeled by machine learning based on various similarity indexes (molecular weight, structure of functional group, electron density, hydrophobicity, hydrophilicity, skeleton, and the like) and statistical theory.

Drug discovery by the LBDD method has been widely implemented, and various types of software provided for free or for a fee can be used in the implementation of the present invention.

In one embodiment of the present invention, the candidate substance contains a substance presumed by the SBDD method to be one or more selected from the group A. That is, the three-dimensional structural coordinates of the protein to be the target of the primary action of the group A are prepared, a pocket is analyzed using the three-dimensional structural information using a computer, and a compound presumed to be bound thereto is selected as a candidate substance. At present, since a supercomputer with extremely high performance is available, it is preferable to implement the SBDD method using the supercomputer.

In one embodiment of the present invention, the candidate substance contains a substance presumed by the LBDD method and the SBDD method to be one or more selected from the group A.

The LBDD method (informatics method) and the SBDD method (simulation method) have a mutually complementary relationship. The informatics design has an advantage that the calculation time is short, and accuracy of the hit rate and the like is stable without depending on the skill of the modeling researcher to be used. On the other hand, there is a disadvantage that only a compound having the same binding site and binding mode as those of the known drugs used hits.

Using simulations, precise binding free energy according to the laws of physics can be predicted, there is an advantage that drug discovery can be developed for all possible binding sites of a protein, and there is a potential to compensate for disadvantages of informatics. However, in the simulation, there is a disadvantage that the calculation time is very long although it depends on the level, and there is a risk that the simulation ends in complete failure when the calculation setting is inappropriate.

From the above reasons, an embodiment in which a candidate substance is selected using both the LBDD method and the SBDD method is preferable.

By introducing transcription factors such as Oct3/4, Klf4, c-Myc, and Sox2 into somatic cells, induced pluripotent stem cells (iPS cells) can be obtained from the somatic cells. These four factors play a very important role in maintaining self-renewal ability and pluripotent properties of stem cells. It can be said that there is a high possibility that a compound capable of improving expression of these four factors can be used as a regenerative medicine. Actually, as shown in the test examples described later, it has been confirmed that an active ingredient selected by the screening method of the present invention has an action of improving the expression of these transcription factors.

From the above, in the present invention, the candidate substance is preferably a substance that positively controls expression of one or more selected from c-Myc, Sox2, Klf4, and Oct4. By adopting such an embodiment, efficient screening can be performed.

In the present embodiment, a substance known to positively control the expression of one or more selected from c-Myc, Sox2, Klf4, and Oct4 can be used as a candidate substance. A substance having such an expression control action can be selected by referring to a compound database.

In the present invention, a substance known in vitro to positively control the expression of one or more selected from c-Myc, Sox2, Klf4, and Oct4 can be used as a candidate substance.

In one embodiment of the present invention, a substance presumed by in silico method to positively control the expression of one or more selected from c-Myc, Sox2, Klf4, and Oct4 is used as a candidate substance.

As described above, many compounds that positively control the expression of one or more selected from c-Myc, Sox2, Klf4, and Oct4 are known, and information on their primary action points is accumulated in the compound database. In addition, a large number of three-dimensional structures of proteins as their primary action points are also registered in the protein database. When an in silico method such as an SBDD method or an LBDD method is applied on the basis of these information, it is possible to easily search for a substance presumed to exhibit the expression control action.

In one embodiment of the present invention, the candidate substance is an agonist of a factor that positively controls expression of one or more selected from c-Myc, Sox2, Klf4, and Oct4.

The factor can be easily grasped by referring to a protein database or the like in which known protein functions and the like are organized and stored. Then, the agonist of the factor can be easily grasped by referring to the above-described compound database.

By using such an agonist as the candidate substance, the active ingredient of the regenerative medicine can be efficiently screened.

In one embodiment of the present invention, the candidate substance is an antagonist of a factor that negatively controls the expression of one or more selected from c-Myc, Sox2, Klf4, and Oct4.

The factor can be easily grasped by referring to a protein database or the like in which known protein functions and the like are organized and stored. Then, the antagonist of the factor can be easily grasped by referring to the above-described compound database.

By using such an antagonist as the candidate substance, the active ingredient of the regenerative medicine can be efficiently screened.

Incidentally, a protein database in which known protein functions and the like are organized and stored is generally provided to be available. In such a database, information obtained by automatically extracting interaction information between proteins from publicly available academic papers and the like by text mining or information recorded manually is stored. In addition, a large amount of microarray data is collected, and information for associating proteins related to the expression status is stored. Examples of such a database include ASEdb (http://nic.ucsf.edu/asedb/), Bacteriome.org (http://www.compsysbio.org/bacteriome/), BioGRID (http://www.thebiogrid.org/), DACSIS (http://cib.cf.ocha.ac.jp/DACSIS/), DIP (http://dip.doe-mbi.ucla.edu/dip/), DroID (http://www.droidb.org/), HCPIN (http://nesg.org:9090/HCPIN/index.jsp), HPID (http://wilab.inha.ac.kr/hpid/), HPRD (http://www.hprd.org/), HUGE ppi (http://www.kazusa.or.jp/huge/ppi/), IntAct (http://www.ebi.ac.uk/intact/), Pathguide (http://www.pathguide.org/), POINT (http://point.bioinformatics.tw/Welcome.do), ProNIT (http://gibk26.bio.kyutech.ac.jp/jouhou/pronit/pronit.html) , Protein-Protein Interaction Panel (http://genome.gsc.riken.go.jp/ppi/), STRING (http://string.embl.de/), and the like.

In one embodiment of the present invention, the candidate substance is a substance presumed by a computer to be an agonist of a factor that positively controls the expression of one or more selected from c-Myc, Sox2, Klf4, and Oct4.

The present embodiment can be implemented by a combination of the above-described database that stores protein interaction information and the above-described in silico method by the LBDD method or the SBDD method. That is, a factor that positively controls the expression of one or more selected from c-Myc, Sox2, Klf4, and Oct4 is specified by referring to the above-described database storing protein interaction information. Next, a compound presumed to be an agonist of the factor by in silico method is selected as a candidate substance.

Such a candidate substance has potential as a regenerative medicine by positively controlling one or more selected from c-Myc, Sox2, Klf4, and Oct4. Therefore, according to the screening method of the present embodiment, the active ingredient can be efficiently selected.

In one embodiment of the present invention, the candidate substance is a substance presumed by a computer to be an antagonist of a factor that negatively controls the expression of one or more selected from c-Myc, Sox2, Klf4, and Oct4.

The present embodiment can be implemented by a combination of the above-described database that stores protein interaction information and the above-described in silico method by the LBDD method or the SBDD method. That is, a factor that negatively controls the expression of one or more selected from c-Myc, Sox2, Klf4, and Oct4 is specified by referring to the above-described database storing protein interaction information. Next, a compound presumed to be an antagonist of the factor by in silico method is selected as a candidate substance.

Such a candidate substance has potential as a regenerative medicine by positively controlling one or more selected from c-Myc, Sox2, Klf4, and Oct4. Therefore, according to the screening method of the present embodiment, the active ingredient can be efficiently selected.

In addition, in one embodiment of the present invention, the candidate substance is a substance presumed by a computer to have low toxicity to a living body.

In modern drug discovery, safety evaluation of compounds is widely performed by in silico method. In the present embodiment, since a substance that has undergone safety evaluation by the in silico method is used as a candidate substance, a possibility that the active ingredient selected as a result of screening can be provided as a medicine can be increased.

Notch signaling is an evolutionarily well-conserved pathway in multicellular organisms, which determines cell fate in the developmental process and maintains adult tissue homeostasis. A Notch pathway mediates cell-contact signaling. Within this transmission pathway, both signaling cells and receiving cells are affected by ligand and receptor crosstalk, thereby controlling a series of cell fate determination mechanisms in the development of the nervous, cardiac, immune, and endocrine systems. A Notch receptor is a single transmembrane protein and consists of a functional extracellular domain (NECD), transmembrane domain (TM), and intracellular domain (NICD). There are four classes 1 to 4 for the Notch receptor. The Notch receptor undergoes processing such as cleavage (S1 cleavage) and sugar chain modification in the endoplasmic reticulum and Golgi apparatus of the signal-receiving cell to generate a heterodimer stabilized by Ca²⁺, which is composed of TM-NICD inserted into the membrane and NECD non-covalently bound thereto. The processed Notch receptor is transported to the cell membrane by endosomes and subjected to control by Deltex and inhibition by NUMB, thereby allowing binding to the ligand.

Delta-like members (DLL1, DLL3, DLL4) and Jagged family (JAG1, JAG2) function as ligands for Notch signaling receptors. Upon ligand binding, NECD is cleaved from the TM-NICD domain by TACE (TNF-α ADAM metal protease converting enzyme) (S2 cleavage). NECD remains bound to the ligand and undergoes ubiquitination-dependent endocytosis/recycling by Mib within a signal-transmitting cell. In the signal-receiving cell, γ-secretase releases NICD from TM (S3 cleavage). This causes NICD to translocate into the nucleus, where it binds to CSL (CBF1/Su(H)/Lag-1) transcription factor complex and induces activation of Myc, p21, HES family, and the like, which are standard target genes for Notch.

Inhibition of Notch signal has been reported to inhibit self-renewal and sternness maintenance of cancer stem cells (Non Patent Literature 13 and Non Patent Literature 14). The Notch signaling pathway also plays a variety of roles during cardiac development, but there is also seemingly contradictory evidence of promoting or impairing cardiomyogenesis in vitro (Non Patent Literature 15) .

Notch signal is said to be essential for maintaining neural progenitor cells (NPCs) in the developing brain, and activation of the Notch signaling pathway maintains NPCs in a proliferative state, while loss-of-function mutations in key components of the pathway cause premature neuronal differentiation and depletion of NPCs (Non Patent Literature 16). A modulator of the Notch signal, e.g., Numb protein, can antagonize Notch effect, leading to cell cycle arrest and differentiation of NPCs (Non Patent Literature 17 and Non Patent Literature 18). Thus, the Notch signaling pathway controls NPC self-renewal and cell fate.

In addition, it is known that hyperactivation mutation of Notch signal causes T-cell acute lymphocytic leukemia, and activity-reducing mutation of Notch signal causes a type of basal cell cancer of skin cancer. Thus, the Notch signal has a double-sided nature capable of acting as both an oncogene and a tumor suppressor gene depending on the tissue on which the signal acts (Non Patent Literature 19).

As described above, it is known that activation of the Notch signal promotes proliferation of NPCs and suppresses differentiation. Also in blood cells, it is known that the activation of Notch signal is important for maintaining self-renewal and undifferentiation potency of hematopoietic stem cells. However, it is also known that the activation of Notch signal induces differentiation of epidermal cells (Non Patent Literature 20). That is, the Notch signal has a double-sided nature of inducing maintenance of self-renewal and undifferentiation potency of undifferentiated cells or conversely inducing differentiation depending on the situation in which the cells are placed.

It has also been reported that the Notch signal activates both expression of a gene and expression of a suppressor of the gene. This report suggests a possibility of having transient responsiveness after the activation of the Notch signal. Furthermore, since the targets of the Notch signaling pathway are composed of both positive regulator and negative regulator, it can be said that the cells are ready for both outcomes. This is considered to be a factor of double-sided nature (maintenance of self-renewal/undifferentiation potency of undifferentiated cells and differentiation induction) indicated by the Notch signal (Non Patent Literature 21).

Thus, the Notch signaling pathway has an important function of controlling maintenance and differentiation of undifferentiated cells. It is particularly important that the Notch signal has seemingly contradictory actions of maintaining the self-renewal/undifferentiation potency of undifferentiated cells and inducing differentiation depending on the situation in which the cells are placed. It is strongly presumed that the action of the active ingredient found in the screening method of the present invention that causes both self-amplification and differentiation induction of undifferentiated cells by a single agent is caused by the double-sided nature of the Notch signal. In fact, as shown in the test examples described later, a plurality of inhibitors of the Notch signaling pathway (Notch-1 inhibitor, γ-secretase inhibitor) have been screened as active ingredients by the screening method of the present invention. Therefore, by targeting the Notch signaling pathway, it is highly likely that more efficient screening of active ingredients can be realized.

That is, in one embodiment of the present invention, a substance that acts on the Notch signaling pathway or a presumed substance is used as a candidate substance.

In one embodiment of the present invention, a substance that is an inhibitor or is presumed to be an inhibitor of the Notch signaling pathway is used as a candidate substance.

Examples of such inhibitors include expression inhibitors of one or more Notch ligands selected from DLL1, DLL3, DLL4, JAG1, and JAG2, one or more Notch ligand inhibitors selected from DLL1, DLL3, DLL4, JAG1, and JAG2, inhibitors of one or more Notch receptors selected from Notch-1, Notch-2, Notch-3, and Notch-4, inhibitors of S1 cleavage (Furin (PACE) inhibitors), inhibitors of S2 cleavage (TACE inhibitors), inhibitors of S3 cleavage (γ-secretase inhibitors), inhibitors of formation of a transcription factor complex in the nucleus of NICD, and the like.

There are many known inhibitors, but they can be grasped by referring to a compound database. In addition, a compound presumed to be an inhibitor of the Notch signaling pathway can be easily grasped by an informatics method based on the known inhibitors. Further, based on the structure of the protein as a target of the inhibitor, a compound presumed to be an inhibitor of the Notch signaling pathway can be easily grasped by a simulation method.

As shown in the test examples described later, inhibitors of the Notch signaling pathway have been screened as active ingredients by the screening method of the present invention.

In one embodiment of the present invention, a substance that is an agonist or is presumed to be an agonist of the Notch signaling pathway is used as a candidate substance. Examples of such agonists include expression improvers for one or more Notch ligands selected from DLL1, DLL3, DLL4, JAG1, and JAG2, agonists for one or more Notch receptors selected from Notch-1, Notch-2, Notch-3, and Notch-4, and the like.

There are many known agonists, but they can be grasped by referring to a compound database. In addition, a compound presumed to be an agonist of the Notch signaling pathway can be easily grasped by an informatics method based on the known inhibitors. Further, based on the structure of the protein as a target of the inhibitor, a compound presumed to be an agonist of the Notch signaling pathway can be easily grasped by a simulation method.

Meanwhile, mTOR is a serine/threonine kinase that senses nutrient sources such as glucose and amino acids and plays a role as a regulator in cell proliferation, metabolism, and survival. This enzyme discovered as a target molecule of antibiotic rapamycin has recently been found to form two types of complexes, mTORC1 consisting of mTOR, Raptor, and mLST8 (GβL), and mTORC2 consisting of mTOR, Rictor, mLST8 (GβL), and Sin1, and to have an independent network.

The complex mTORC1 is activated by growth factors, hormones, stress, and the like, in addition to the aforementioned nutrient sources, phosphorylates molecules involved in protein synthesis and cell proliferation such as 4EBP1 and p70S6K, and is involved in mRNA translation, autophagy suppression, ribosome biosynthesis, and the like.

On the other hand, the complex mTORC2 is not regulated by a nutrient source, is activated by PI3K stimulated by a growth factor, phosphorylates Akt, SGK, and PKC, and is involved in suppression of apoptosis, cell growth, cytoskeletal control, and the like.

Among them, the PI3K/AKT/mTOR signaling pathway is activated in most cancers, and therefore has been deeply studied as a target of anticancer agents. PI3K is a unique intracellular lipid kinase family that phosphorylates 3'-hydroxyl group of inositol ring of phosphatidylinositide (PtdIns) and is classified into three classes. Most studied is class I PI3K, which promotes conversion of membrane-bound phosphatidylinositol-(4,5)-diphosphate (PIP2) to phosphatidylinositol-(3,4,5)-triphosphate (PIP3). PIP3 functions as a second messenger and promotes recruitment and activation of kinases with PH domains, such as PI3K-dependent kinase-1 (PDK1). PIP3 signaling is regulated by PTEN, which opposes PI3K activity. Serine/threonine kinase AKT, also known as protein kinase B (PKB), has a PH domain and is recruited to the cell membrane along with PDK1. Phosphorylation of amino acid residues T308 and S473 by PDK1 and mTORC2 is essential for complete activation of AKT.

Activated AKT phosphorylates many target proteins, particularly glycogen synthase kinase 3 (GSK3), tuberous sclerosis 2 (TSC2), caspase 9, and PRAS40 (AKT1S1), and exhibits a relatively broad spectrum of downstream effects that promote cell proliferation, differentiation, apoptosis, angiogenesis, and metabolism.

The study of this pathway in development was challenging, as lack of components of the PI3K/AKT/mTOR signaling pathway is often embryonic lethal. However, with the advent of ES and iPS cells, this problem has been alleviated to some extent. Consequently, the PI3K/AKT/mTOR signaling pathway has been found to be important for embryonic development and essential for iPS cell self-renewal. Although human ES cells and mouse ES cells greatly differ in the types of signals required for culture and proliferation, studies using kinase inhibitors and genetic approaches have revealed that both types of ES cells require activation of PI3K/AKT signals in order to maintain undifferentiated properties. When mouse ES cells are treated with a common PI3K inhibitor or a p 110β-specific inhibitor, their pluripotency is impaired, and pluripotency of mouse ES cells is lost even by deletion of PI3K gene. Similarly, inhibition of PI3K in human ES cells results in concomitant downregulation of pluripotency markers and upregulation of lineage-specific genes, both strongly suggesting loss of pluripotency. Supporting this, knockout of Pten in both mouse ES cells and human ES cells promotes proliferation and survival of these cells (Non Patent Literature 22).

In addition, LIF is added in culture of ES cells which are widely used in research, it is known that a LIF receptor activates a PI3K/AKT signaling pathway. LIF is a cytokine that was previously identified as one of IL-6 family, but is used as a differentiation inhibitor (maintenance of totipotency) in the culture of ES cells.

As described above, the PI3K/AKT/mTOR signaling pathway plays an important role in maintenance of self-renewal and pluripotency of stem cells and differentiation control. Therefore, the screening method of the present invention may be an embodiment targeting the PI3K/AKT/mTOR signaling pathway.

That is, in one embodiment of the present invention, a substance that acts on the PI3K/AKT/mTOR signaling pathway or a presumed substance is used as a candidate substance.

In one embodiment of the present invention, a substance that is an inhibitor or is presumed to be an inhibitor of the PI3K/AKT/mTOR signaling pathway is used as a candidate substance.

Such inhibitors include PI3K inhibitors, AKT inhibitors, mTOR inhibitors, mTORRC1 inhibitors, mTORC2 inhibitors, mTORC1/2 inhibitors, PI3K/mTOR inhibitors, and the like. There are many known inhibitors, but they can be grasped by referring to a compound database. In addition, a compound presumed to be an inhibitor of the PI3K/AKT/mTOR signaling pathway can be easily grasped by an informatics method based on the known inhibitors. Further, based on the structure of the protein as a target of the inhibitor, a compound presumed to be an inhibitor of the PI3K/AKT/mTOR signaling pathway can be easily grasped by a simulation method.

As shown in the test examples described later, inhibitors of the PI3K/AKT/mTOR signaling pathway have been screened as active ingredients by the screening method of the present invention.

In one embodiment of the present invention, a substance that is an agonist or is presumed to be an agonist of the PI3K/AKT/mTOR signaling pathway is used as a candidate substance. Such agonists include PTEN inhibitors, PI3K activators, and the like.

There are many known inhibitors and activators, but they can be grasped by referring to a compound database. In addition, a compound presumed to be an agonist of the PI3K/AKT/mTOR signaling pathway can be easily grasped by an informatics method based on the known inhibitors and activators. Further, based on the structure of the protein as a target of the inhibitor, a compound presumed to be an agonist of the PI3K/AKT/mTOR signaling pathway can be easily grasped by a simulation method.

A JAK/STAT signaling pathway is an information transmission system that transmits chemical signals from outside the cell to the cell nucleus and causes transcription and expression of DNA. It is involved in immunity, cell proliferation, differentiation, apoptosis, carcinogenesis, and the like. A JAK/STAT signal cascade is mainly composed of three elements: a receptor of cytokine such as interferon, interleukin, or a growth factor at the cell surface, JAK, and STAT.

The mechanism is that first upon binding of a ligand (interferon, interleukin, a growth factor, or the like), the receptor activates JAK and exhibits its kinase activity. The activated JAK phosphorylates tyrosine residues of the receptor and creates binding sites for proteins with SH2 domain of the receptor. STAT with SH2 domain binds to tyrosine sites phosphorylated by JAK, and STAT itself is phosphorylated by JAK. The activated STAT phosphorylated on tyrosine residues forms hetero or homodimers and migrates into the cell nucleus, causing transcription of the target gene. STATs can also receive tyrosine phosphorylation directly from receptor tyrosine kinases (such as epidermal growth factor receptor) or also receive from non-receptor intracytoplasmic tyrosine kinases (such as c-src).

This JAK/STAT signaling pathway is negatively controlled in multiple steps. Cytokine receptors and activated STATs are dephosphorylated and inactivated by protein tyrosine dephosphorylation enzyme. In addition, it is known that a suppressor of cytokine signaling (SOCS) binds to JAK, suppresses binding and phosphorylation of STAT of JAK, and competitively inhibits phosphorylated tyrosine of a cytokine receptor. STATs are negatively controlled in the nucleus by a protein inhibitor of activated STAT (PIAS). For example, PIAS1 and PIAS3 bind to and inhibit DNA, thereby inhibiting transcriptional activation by STAT1 and STAT3.

Recent studies have shown that important homeostatic processes of the germline and adult stem cells in Drosophila, as well as regeneration processes of several tissues including gonads, intestines, and appendages, are also controlled (Non Patent Literature 23). In addition, regulation of muller glial stem cell properties by JAK/STAT and MAPK signaling pathways in mammalian retinas has been reported (Non Patent Literature 24).

Further, LIF is added as a differentiation inhibitor in the culture of ES cells widely used in research, and it is known that the LIF receptor activates the JAK/STAT signaling pathway.

As described above, the JAK/STAT signaling pathway plays an important role in stem cell control. In addition, as described in the test examples described later, a plurality of JAK/STAT inhibitors have been screened by the screening method of the present invention.

Therefore, the screening method of the present invention may be an embodiment targeting the JAK/STAT signaling pathway.

That is, in one embodiment of the present invention, a substance that acts on the JAK/STAT signaling pathway or a presumed substance is used as a candidate substance.

In one embodiment of the present invention, a substance that is an inhibitor or is presumed to be an inhibitor of the JAK/STAT signaling pathway is used as a candidate substance.

Such inhibitors include JAK inhibitors, STAT inhibitors, and the like. There are many known inhibitors, but they can be grasped by referring to a compound database. In addition, a compound presumed to be an inhibitor of the JAK/STAT signaling pathway can be easily grasped by an informatics method based on the known inhibitors. Further, based on the structure of the protein as a target of the inhibitor, a compound presumed to be an inhibitor of the JAK/STAT signaling pathway can be easily grasped by a simulation method.

As shown in the test examples described later, inhibitors of the JAT/STAT signaling pathway have been screened as active ingredients by the screening method of the present invention.

In one embodiment of the present invention, a substance that is an agonist or is presumed to be an agonist of the JAK/STAT signaling pathway is used as a candidate substance. Such agonists include PIAS inhibitors, SOCS inhibitors, PTP inhibitors, and the like.

There are many known inhibitors, but they can be grasped by referring to a compound database. In addition, a compound presumed to be an agonist of the JAK/STAT signaling pathway can be easily grasped by an informatics method based on the known inhibitors. Further, based on the structure of the protein as a target of the inhibitor, a compound presumed to be an agonist of the JAK/STAT signaling pathway can be easily grasped by a simulation method.

Components of mitogen-activated protein kinase (MAPK) signaling pathway transmit and regulate extracellular stimuli for controlling and fine-tuning vital cellular functions including proliferation, cell division, metabolism, motility, innate immunity, cellular stress response, apoptosis, and survival functions in eukaryotes ranging from yeast to humans. The MAPK signaling pathway is known to include four major branched pathways and the presence of several types of MAPK enzymes, and is classified into at least seven different groups.

A MAPK cascade is characterized by sequential activation by three protein kinases via bispecific serine/threonine protein kinases (MAPK, MAPK activator (MEK, MKK, or MAPK kinase), and MEK activator (MEK kinase [MEKK] or MAPK kinase kinase). Activation of the classical MAPK pathway is initiated in the cell membrane, where it is activated by phosphorylation of MAPKKK by low molecular weight GTPases and various protein kinases. Subsequently, MAPKK is directly phosphorylated by MAPKKK, and activated MAPKK phosphorylates MAPK. The activated MAPK interacts with numerous intracytoplasmic substrates to phosphorylate and ultimately regulate transcription factors that induce context-specific gene expression.

In the pathway activated by growth factors, A-Raf, B-RAF, Mos, and Tpi-2 are known as MAPKKK, MEK1/2 is known as MAPKK, ERK1/2 is known as MAPK, and Elk-1, Ets-2, RSK, MNK, MSK, cPLA2, and the like are known as downstream factors thereof.

In the pathway activated by stress, cytokines, growth factors and the like, MLK3, TAK1, MEKK4, and ASK1 are known as MAPKKK, MKK3/6 is known as MAPKK, p38α/β/γ/5 is known as MAPK, and CHOP, ATF2, MNK, MSK, MEF2, and Elk-1 are known as downstream factors thereof.

In the pathway activated by stress, cytokines and growth factors, MEKK1/4, DLK, MLK1-4, LZK, TAK1, ASK1, and ZAK are known as MAPKKK, MKK4/7 is known as MAPKK, JKK1,2,3 are known as MAPK, and JUN, ATF2, RNPK, p53, NFAT4, Shc, and the like are known as downstream factors thereof.

In pathways activated by stress and growth factors, MEKK2/3 is known as MAPKKK, MEK5 is known as MAPKK, ERK5 is known as MAPK, and MEF2 is known as a downstream factor thereof.

In addition, LIF is added as a differentiation inhibitor in the culture of ES cells widely used in research, and it is known that the LIF receptor activates the MAPK signaling pathway (RAS-RAF-MEK-ERK pathway). It is known that this signaling pathway is essential for maintenance of self-renewal and totipotency.

As described above, the MAPK signaling pathway plays an important role in stem cell control. In addition, as described in the test examples described later, a plurality of MAPK signaling pathway inhibitors have been screened by the screening method of the present invention.

Therefore, the screening method of the present invention may be an embodiment targeting the MAPK signaling pathway.

That is, in one embodiment of the present invention, a substance that acts on the MAPK signaling pathway or a presumed substance is used as a candidate substance.

In one embodiment of the present invention, a substance that is an inhibitor or is presumed to be an inhibitor of the MAPK signaling pathway is used as a candidate substance.

Such inhibitors include one or more selected from A-Raf inhibitors, B-RAF inhibitors, Mos inhibitors, Tpi-2 inhibitors, MEK1/2 inhibitors, ERK1/2 inhibitors, Elk-1 inhibitors, Ets-2 inhibitors, RSK inhibitors, MNK inhibitors, MSK inhibitors, cPLA2 inhibitors, MLK3 inhibitors, TAK1 inhibitors, MEKK4 inhibitors, ASK1 inhibitors, MKK3/6 inhibitors, p38α/β/γ/5 inhibitors, CHOP inhibitors, ATF2 inhibitors, MNK inhibitors, MSK inhibitors, MEF2 inhibitors, Elk-1 inhibitors, MEKK1/4 inhibitors, DLK inhibitors, MLK1-4 inhibitors, LZK inhibitors, TAK1 inhibitors, ASK1 inhibitors, ZAK inhibitors, MKK4/7 inhibitors, JKK1,2,3 inhibitors, JUN inhibitors, FOS inhibitors, ATF2 inhibitors, RNPK inhibitors, p53 inhibitors, NFAT4 inhibitors, Shc inhibitors, MEKK2/3 inhibitors, MEK5 inhibitors, ERK5 inhibitors, and MEF2 inhibitors. There are many known inhibitors, but they can be grasped by referring to a compound database. In addition, a compound presumed to be an inhibitor of the MAPK signaling pathway can be easily grasped by an informatics method based on the known inhibitors. Further, based on the structure of the protein as a target of the inhibitor, a compound presumed to be an inhibitor of the MAPK signaling pathway can be easily grasped by a simulation method.

As shown in the test examples described later, inhibitors of the MAPK signaling pathway have been screened as active ingredients by the screening method of the present invention.

In one embodiment of the present invention, a substance that is an agonist or is presumed to be an agonist of the MAPK signaling pathway is used as a candidate substance. Such agonists include interleukin receptor activators and the like.

There are many known activators, but they can be grasped by referring to a compound database. In addition, a compound presumed to be an agonist of the MAPK signaling pathway can be easily grasped by an informatics method based on the known inhibitors. Further, based on the structure of the protein as a target of the inhibitor, a compound presumed to be an agonist of the MAPK signaling pathway can be easily grasped by a simulation method.

Transforming growth factor-β (TGF-β), Nodal, Activin, BMP, and the like are cytokines belonging to TGF-β superfamily, and regulate various functions of cells. Biological phenomena controlled by the TGF-β superfamily are diverse, such as inhibition of cell proliferation, differentiation, cell death, angiogenesis, immunity, extracellular matrix production, and senescence. Cells that have undergone stimulation of the TGF-β superfamily, which functions extracellularly, convert the stimulation to phosphorylation of transcription factor SMAD on the cell membrane. The TGF-β superfamily is roughly divided into a group consisting of TGF-β/Nodal/Activin and a group consisting of BMP, where stimulation of TGF-β/Nodal/Activin converts to phosphorylation of SMAD2 and SMAD3 (SMAD2/3) and stimulation of BMP converts to phosphorylation of SMAD1, SMAD5, and SMAD8 (SMAD1/5/8). These SMAD (SMAD1/2/3/5/8) are called receptor-regulated SMAD (R-SMAD). It is known that stimulus-dependent phosphorylated R-SMAD of the TGF-β superfamily forms a heterotrimer with SMAD4 called Co-SMAD (common-mediator SMAD), migrates into the nucleus, and regulates (activates or suppresses) various gene expression. In addition to R-SMAD and Co-SMAD, there is also I-SMAD (Inhibitory SMAD, SMAD6 SMAD7) that negatively controls the function of the TGF-β superfamily.

In the field of stem cells, BMP-4 is an important serum-derived factor that maintains mouse ES cells in an undifferentiated state together with LIF, and it has been understood that this action requires activation of specific intracellular signaling pathways (Smad pathways). However, as knowledge of other stem cells including human ES cells is accumulated, it has also been pointed out that BMPs often promote differentiation of stem cells.

As described above, the TGFβ/SMAD signaling pathway plays an important role in stem cell control. In addition, as described in the test examples described later, a plurality of TGFβ/SMAD inhibitors have been screened by the screening method of the present invention.

Therefore, the screening method of the present invention may be an embodiment targeting the TGFβ/SMAD signaling pathway.

That is, in one embodiment of the present invention, a substance acting on the TGFβ/SMAD signaling pathway or a presumed substance is used as a candidate substance.

In one embodiment of the present invention, a substance that is an inhibitor or is presumed to be an inhibitor of the TGFβ/SMAD signaling pathway is used as a candidate substance.

Such inhibitors include Tβ receptor inhibitors, Nodal receptor inhibitors, Activin receptor inhibitors, BMP receptor inhibitors, SMAD inhibitors, and the like. There are many known inhibitors, but they can be grasped by referring to a compound database. In addition, a compound presumed to be an inhibitor of the TGFβ/SMAD signaling pathway can be easily grasped by an informatics method based on the known inhibitors. Further, based on the structure of the protein as a target of the inhibitor, a compound presumed to be an inhibitor of the TGFβ/SMAD signaling pathway can be easily grasped by a simulation method.

As shown in the test examples described later, inhibitors of the TGFβ/SMAD signaling pathway have been screened as active ingredients by the screening method of the present invention.

In one embodiment of the present invention, a substance that is an agonist or is presumed to be an agonist of the TGFβ/SMAD signaling pathway is used as a candidate substance. Such agonists include Tβ receptor agonists, Nodal receptor agonists, Activin receptor agonists, BMP receptor agonists, SMAD agonists, and the like.

There are many known agonists, but they can be grasped by referring to a compound database. In addition, a compound presumed to be an agonist of the TGFβ/SMAD signaling pathway can be easily grasped by an informatics method based on the known agonists. Further, based on the structure of the protein as a target of the agonist, a compound presumed to be an agonist of the TGFβ/SMAD signaling pathway can be easily grasped by a simulation method.

In humans, 19 WNT genes have been identified so far. Some of the genes undergo alternative splicing, and the gene product Wnt protein has multiple isoforms. The Wnt protein mainly binds to a Frizzled receptor, which is a seven-transmembrane receptor protein, and transmits a signal into a cell. The Frizzled receptors are composed of about 10 protein families, and expression of these proteins varies depending on the cell type, and contributes to specificity of cell type and signal transduction.

The intracellular amount of β-catenin is maintained at low levels with Wnt signal off. In this state, β-catenin is incorporated into a degradation complex composed of Adenomatous Polyposis Coli (APC) or the like, is phosphorylated by Casein kinase 1 (CK1) or Glycogen synthase 3b (GSK3β) which are serine/threonine kinases, is then ubiquitinated by β-Transducin repeat containing protein (bTrCP), and is degraded by proteosomes.

On the other hand, in a situation where the Wnt signal is activated, Dishevelled recruits GBP/Frat-1 that releases GSK3β from the degradation complex, and acts to protect β-catenin. At this time, Frodo and β-Arrestin act with Dishevelled in a conjugate manner. On the other hand, Dapper acts as an antagonist of Dishevelled. In addition, LRP5/LRP6, which is a low-density lipoprotein receptor-related protein family, serves as a coupled receptor of canonical Wnt signaling cascade via β-catenin.

In the canonical cascade via β-catenin, when the Wnt signal is activated, β-catenin is stabilized and translocates into the nucleus. In the nucleus, transcription of target genes such as TCF, Lymphoid Enhancer D1, PPARd, and Twin is activated. On the other hand, in a state where the Wnt signal is off, TCF/LEF binds to a transcription regulator such as Groucho and is inactivated. Transcriptional regulatory activity of Groucho is mediated by histone deacetylases (Histone Deactylases; HDAC) that transcriptionally regulates genes.

ICAT and Duplin bind to β-catenin and inhibit the interaction between β-catenin and TCF/LEF, thereby negatively controlling the canonical cascade. Furthermore, β-catenin is also involved in cell adhesion, and binds to the intracellular domain of type I classical cadherin such as E-Cadherin and N-Cadherin, and further forms a complex with α-catenin. This cadherin/catenin complex is considered to interact with Actin cytoskeleton via other molecules.

In the signaling of PCP (Planar Cell Polarity) pathway, which is one of non-canonical cascades, polarity of cells is regulated by controlling the Actin cytoskeleton and asymmetric cytoskeleton formation. When Wnt binds to the Frizzled receptor, Dishevelled activates Rho and Rac which are small molecules GTPases. In signaling via Rho, Daam-1 forms a complex with Dishevelled and Rho to activate Rho kinase ROCK. On the other hand, in signal transduction via Rac, Dishevelled does not interact with Daam-1, and activates Jun kinase (JNK).

In Wnt/Calcium pathway, which is another non-canonical cascade, calcium is released into the cytoplasm when Wnt binds to the Frizzled receptor. This involves Knypek and Ror2, which are coupled receptors of Frizzled. In addition, heterotrimeric G protein, Phospholipase C (PLC), Protein Kinase C (PKC), and the like are known as intracellular second messengers of this pathway. It has been reported that the Wnt/Calcium pathway is important for intercellular adhesion and cell dynamics during gastrulation.

Antagonists of Wnt signaling are classified into two classes, secreted Frizzled Related Protein (sFRP) class and Dickkopf (Dkk) class. The sFRP classes include sFRP1-5, Wnt Inhibitory Factor-1 (WIF-1), and Cerberus, which are sFRP families. These antagonists bind directly to Wnt and inhibit the binding of Wnt to the receptor. On the other hand, Dkk1-4 included in the Dkk class binds to an extracellular domain of LRP5/LRP6 and suppresses the Wnt signal. Kremen binds to Dkk bound to LRP5/LRP6, and takes up the Dkk-LRP complex into the cell by endocytosis, thereby eliminating exposure of LRP on the cell surface. As a result, Wnt cannot bind to the receptor, and signal transduction is suppressed. Therefore, theoretically, an antagonist of the sFRP class inhibits both the canonical cascade via β-catenin and the non-canonical pathway in Wnt signaling, and an antagonist of the Dkk class selectively inhibits only the canonical cascade.

The isolated Wnt protein shows activity in various stem cells, such as neural stem cells, mammary stem cells, embryonic stem cells, and the like. In general, Wnt proteins act to maintain the undifferentiated state of stem cells (Non Patent Literature 25).

As described above, the Wnt signaling pathway plays an important role in stem cell control. In addition, as described in the test examples described later, a plurality of Wnt/β-catenins have been screened by the screening method of the present invention.

Therefore, the screening method of the present invention may be an embodiment targeting the Wnt signaling pathway.

That is, in one embodiment of the present invention, a substance acting on the Wnt signaling pathway or a presumed substance is used as a candidate substance.

In one embodiment of the present invention, a substance that is an inhibitor or is presumed to be an inhibitor of the Wnt signaling pathway is used as a candidate substance.

Such inhibitors include Wnt inhibitors and Frizzled receptor inhibitors. There are many known inhibitors, but they can be grasped by referring to a compound database. In addition, a compound presumed to be an inhibitor of the Wnt signaling pathway can be easily grasped by an informatics method based on the known inhibitors. Further, based on the structure of the protein as a target of the inhibitor, a compound presumed to be an inhibitor of the Wnt signaling pathway can be easily grasped by a simulation method.

As shown in the test examples described later, inhibitors of the Wnt signaling pathway have been screened as active ingredients by the screening method of the present invention.

In one embodiment of the present invention, a substance that is an agonist or is presumed to be an agonist of the Wnt signaling pathway is used as a candidate substance. Such agonists include GSK-3 inhibitors, CK1 inhibitors, bTrCP inhibitors, Pan-Proteasome inhibitors, and the like. There are many known inhibitors, but they can be grasped by referring to a compound database. In addition, a compound presumed to be an agonist of the Wnt signaling pathway can be easily grasped by an informatics method based on the known inhibitors. Further, based on the structure of the protein as a target of the inhibitor, a compound presumed to be an agonist of the Wnt signaling pathway can be easily grasped by a simulation method.

The Notch signaling pathway, the PI3K/AKT/mTOR signaling pathway, the JAK/STAT signaling pathway, the MAPK signaling pathway, and the TGFβ/SMAD signaling pathway are generally said to promote maintenance of self-renewal and totipotency of stem cells. However, as shown in the test examples described later, a compound group that induces an increase in both undifferentiated cells and differentiated cells in vivo includes inhibitors and activators of these signaling pathways. From this, it can be said that a substance acting on these pathways is highly likely to induce an increase in both undifferentiated cells and differentiated cells in vivo, regardless of whether the substance is an inhibitor or an activator.

Therefore, in a preferred embodiment of the present invention, a substance that acts (including inhibition and activation) on one or more signaling pathways selected from the Notch signaling pathway, the PI3K/AKT/mTOR signaling pathway, the JAK/STAT signaling pathway, the MAPK signaling pathway, the TGFβ/SMAD signaling pathway, and the Wnt signaling pathway is used as a candidate substance for screening.

For the same reason, in a preferred embodiment of the present invention, a substance presumed by in silico method to act (including inhibition and activation) on one or more signaling pathways selected from the Notch signaling pathway, the PI3K/AKT/mTOR signaling pathway, the JAK/STAT signaling pathway, the MAPK signaling pathway, the TGFβ/SMAD signaling pathway, and the Wnt signaling pathway is used as a candidate substance for screening.

Also, in one embodiment, a compound included in general formula (I), general formula (II), or general formula (III) described later or a salt thereof or a hydrate of the compound or salt is administered to an animal as a candidate substance.

The salt is preferably a pharmacologically acceptable salt. Examples of the salt include inorganic acid salts, organic acid salts, inorganic base salts, organic base salts, acidic or basic amino acid salts, and the like.

Preferred examples of the inorganic acid salt include hydrochlorides, hydrobromides, sulfates, nitrates, phosphates, and the like, and preferred examples of the organic acid salt include acetates, succinates, fumarates, maleates, tartrates, citrates, lactates, stearates, benzoates, mandelates, methanesulfonates, ethanesulfonates, p-toluenesulfonates, benzenesulfonates, and the like.

Preferred examples of the inorganic base salt include alkali metal salts such as sodium salts and potassium salts, alkaline earth metal salts such as calcium salts and magnesium salts, aluminum salts, ammonium salts, and the like, and preferred examples of the organic base salt include diethylamine salts, diethanolamine salts, meglumine salts, N,N'-dibenzylethylenediamine salts, and the like.

Preferred examples of the acidic amino acid salt include aspartates, glutamates, and the like, and preferred examples of the basic amino acid salt include arginine salts, lysine salts, ornithine salts, and the like.

Hereinafter, the general formulae (I) to (III) will be described in detail.

### [1-1-2-1] General formula (I)

In one embodiment of the present invention, a compound included in the following general formula (I) or a salt thereof or a hydrate of the compound or salt is administered to an animal as a candidate substance.

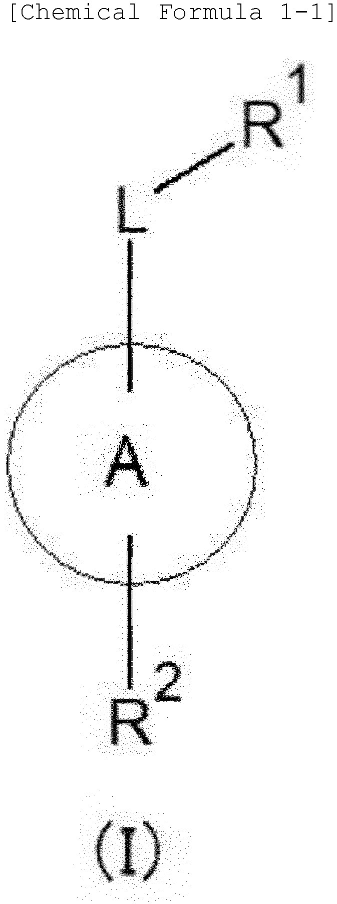

In the general formula (I), ring A may be a monocyclic ring or a fused bicyclic ring.

When ring A is a monocyclic ring, ring A is preferably a 3- to 8-membered ring, more preferably a 4- to 6-membered ring, more preferably a 5- to 6-membered ring, and more preferably a 5-membered ring.

When ring A is a fused bicyclic ring, ring A is preferably a 4- to 8-membered ring, more preferably a 5- to 8-membered ring, more preferably a 5- to 7-membered ring, and more preferably a 5- to 6-membered ring.

In a preferred embodiment, ring A is a monocyclic ring.

Ring A may be saturated or unsaturated. When unsaturated, the number of double bonds contained in ring A is preferably 1 to 3, and more preferably 1 to 2.

Preferably, ring A is unsaturated. More preferably, ring A is an aromatic ring.

Ring A may contain a heteroatom.

The number of heteroatoms contained in ring A is preferably 1 to 4, more preferably 1 to 3, more preferably 2 to 3, and more preferably 2.

Examples of the hetero atom include an oxygen atom, a nitrogen atom, and a sulfur atom, and more preferably include a nitrogen atom.

The number of heteroatoms independently selected from an oxygen atom, a nitrogen atom, and a sulfur atom is 1 to 4, more preferably 1 to 3, more preferably 2 to 3, and more preferably 2.

Ring A is preferably an aromatic heterocyclic ring containing a hetero atom, more preferably an aromatic heterocyclic ring containing a nitrogen atom, and more preferably a monocyclic aromatic heterocyclic ring containing a nitrogen atom.

Specific examples of ring A include aziridine, azirine, oxirane, oxylene, phosphirane, phosphylene, thiirane, thiirene, diaziridine, diazirine, oxaziridine, dioxirane, azetidine, azete, oxetane, oxete, thietane, thiete, diazetidine, diazete, dioxetane, dioxete, dithietane, dithiete, pyrrolidine, pyrrole, tetrahydrofuran, furan, tetrahydrothiophene, thiophene, imidazolidine, pyrazolidine, imidazole, imidazoline, pyrazole, oxazolidine, isoxazolidine, oxazole, oxazoline, isoxazole, thiazolidine, isothiazolidine, thiazole, isothiazole, dioxolane, dithiolane, triazole, furazan, oxadiazole, thiadiazole, dioxazole, dithiazole, tetrazole, oxatetrazole, thiatetrazole, pentazole, piperidine, pyridine, pyridinium cation, tetrahydropyran, pyran, pyrylium cation, thiane, thiopyran, thiopyrylium cation, piperazine, diazine, morpholine, oxazine, thiomorpholine, thiazine, dioxane, dioxine, dithiane, dithiine, hexahydro-1,3,5-triazine, triazine, trioxane, trithiane, tetrazine, pentazine, azepane, azepine, oxepane, oxepine, thiepane, thiepine, diazepane, diazepine, thiazepine, azocane, azocine, oxocane, oxocine, thiocane, thiocine, azonane, azonine, oxonane, oxonine, thionane, thionine, and the like.

More preferred embodiments of ring A include pyrrole, furan, thiophene, imidazole, imidazoline, pyrazole, pyrazoline, oxazole, oxazoline, isoxazole, thiazole, thiazoline, isothiazole, triazole, furazan, oxadiazole, thiadiazole, dioxazole, dithiazole, tetrazole, oxatetrazole, thiatetrazole, pentazole, pyridine, pyran, thiopyran, diazine, oxazine, thiazine, dioxine, dithiin, triazine, and tetrazine.

More preferred embodiments of ring A include pyrrole, imidazole, imidazoline, pyrazole, pyrazoline, triazole, pyridine, diazine, triazine, and tetrazine.

More preferred embodiments of ring A include imidazole, pyrazole, triazole, pyridine, diazine, and triazine.

Still more preferably, ring A is pyrazole.

When ring A is a pyrazole, R² is preferably bonded to any one of 3 to 5-positions, and more preferably 4-position of the pyrazole ring.

In addition, the group consisting of -L-R¹ is bonded to preferably 1, 3, or 5-position, and more preferably 1 or 3-position of the pyrazole ring.

The number of the pyrazole ring is according to the example shown below.

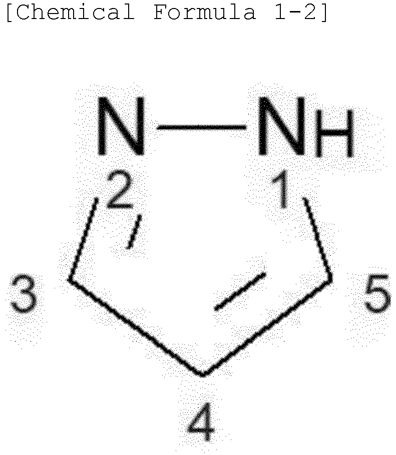

Ring A may be further substituted with a C1-3 alkyl group or halogen atom.

The C1-3 alkyl group as used herein may be linear or branched. Specific examples thereof include a methyl group, an ethyl group, a propyl group, a methylethyl group, and the like.

In addition, the halogen atom as used herein preferably includes a fluorine atom, a chlorine atom, a bromine atom, an iodine atom, and the like.

Preferably 1 to 3, more preferably 1 to 2 and more preferably 1 of hydrogen atoms of ring A may be substituted.

In the general formula (I), L is a C1-10 saturated or unsaturated hydrocarbon chain which may be substituted with substituent R³.

The hydrocarbon chain is preferably C1-9, more preferably C1-8, more preferably C1-7, more preferably C1-6, more preferably C1-5, more preferably C1-4, more preferably C1-3, and more preferably C1-2.

Specific examples of the hydrocarbon chain include-CH₂-, -CH₂CH₂-, -CH=CH-, -CH₂CH₂CH₂-, -CH=CHCH₂-, -CH₂CH=CH-, -CH₂CH₂CH₂CH₂-, -CH=CHCH₂CH₂-, -CH₂CH=CHCH₂-, -CH₂CH₂CH=CH-,-CH=CHCH=CH-, and the like.

The hydrocarbon chain may be substituted with the substituent R³.

The substituent R³ is a C1-10 saturated or unsaturated hydrocarbon group which may be substituted with a halogen atom and may have a cyclic structure.

The halogen atom as used herein preferably includes a fluorine atom, a chlorine atom, a bromine atom, an iodine atom, and the like.

The substituent R³ is preferably C1-10, more preferably C2 to 9, more preferably C3 to 8, and more preferably C4 to 6.

The substituent R³ may be linear or branched.

Also, the substituent R³ may have a cyclic structure. In this case, the ring is preferably a 3- to 10-membered ring, more preferably a 4- to 8-membered ring, more preferably a 4- to 7-membered ring, more preferably a 4- to 6-membered ring, more preferably a 5- to 6-membered ring, and more preferably a 5-membered ring.

In the general formula (I), L may not be present.

In the general formula (I), R¹ is R¹⁻¹ or R¹⁻² as defined below.
R¹⁻¹: -C=N, -COOH, -CHO, -COOCH₃, -NO₂, or halogen atom
R¹⁻²: a 3- to 8-membered
monocyclic or fused bicyclic group,
which may contain 1 to 4 heteroatoms independently selected from an oxygen atom, a nitrogen atom, and a sulfur atom,
is saturated or unsaturated, and
may be further substituted with a C1-3 alkyl group or a halogen atom.

R¹⁻¹ is more preferably -C=N or -NO₂.

R¹⁻² may be a monocyclic ring or a fused bicyclic ring.

When R¹⁻² is a monocyclic ring, R¹⁻² is preferably a 3- to 8-membered ring, more preferably a 4- to 6-membered ring, more preferably a 5- to 6-membered ring, and more preferably a 6-membered ring.

When R¹⁻² is a fused bicyclic ring, R¹⁻² is preferably a 4- to 8-membered ring, more preferably a 5- to 8-membered ring, more preferably a 5- to 7-membered ring, and more preferably a 5- to 6-membered ring.

In a preferred embodiment, R¹⁻² is a monocyclic ring.

R¹⁻² may be saturated or unsaturated. When unsaturated, the number of double bonds contained in R¹⁻² is preferably 1 to 3, and more preferably 2 to 3.

Preferably, R¹⁻² is unsaturated. More preferably, R¹⁻² is an aromatic ring.

R¹⁻² may contain a heteroatom.

The number of heteroatoms contained in R¹⁻² is preferably 1 to 4, more preferably 1 to 3, more preferably 1 to 2, and more preferably 1.

Examples of the hetero atom include an oxygen atom, a nitrogen atom, and a sulfur atom, and more preferably include a nitrogen atom.

The number of heteroatoms independently selected from an oxygen atom, a nitrogen atom, and a sulfur atom is 1 to 4, more preferably 1 to 3, more preferably 1 to 2, and more preferably 1.

R¹⁻² is preferably an aromatic heterocyclic ring containing a hetero atom, more preferably an aromatic heterocyclic ring containing a nitrogen atom, and more preferably a monocyclic aromatic heterocyclic ring containing a nitrogen atom.

Specific examples of R¹⁻² include aziridine, azirine, oxirane, oxylene, phosphirane, phosphylene, thiirane, thiirene, diaziridine, diazirine, oxaziridine, dioxirane, azetidine, azete, oxetane, oxete, thietane, thiete, diazetidine, diazete, dioxetane, dioxete, dithietane, dithiete, pyrrolidine, pyrrole, tetrahydrofuran, furan, tetrahydrothiophene, thiophene, imidazolidine, pyrazolidine, imidazole, imidazoline, pyrazole, oxazolidine, isoxazolidine, oxazole, oxazoline, isoxazole, thiazolidine, isothiazolidine, thiazole, isothiazole, dioxolane, dithiolane, triazole, furazan, oxadiazole, thiadiazole, dioxazole, dithiazole, tetrazole, oxatetrazole, thiatetrazole, pentazole, piperidine, pyridine, pyridinium cation, tetrahydropyran, pyran, pyrylium cation, thiane, thiopyran, thiopyrylium cation, piperazine, diazine, morpholine, oxazine, thiomorpholine, thiazine, dioxane, dioxine, dithiane, dithiine, hexahydro-1,3,5-triazine, triazine, trioxane, trithiane, tetrazine, pentazine, azepane, azepine, oxepane, oxepine, thiepane, thiepine, diazepane, diazepine, thiazepine, azocane, azocine, oxocane, oxocine, thiocane, thiocine, azonane, azonine, oxonane, oxonine, thionane, thionine, and the like.

More preferred embodiments of R¹⁻² include pyrrole, furan, thiophene, imidazole, imidazoline, pyrazole, pyrazoline, oxazole, oxazoline, isoxazole, thiazole, thiazoline, isothiazole, triazole, furazan, oxadiazole, thiadiazole, dioxazole, dithiazole, tetrazole, oxatetrazole, thiatetrazole, pentazole, pyridine, pyran, thiopyran, diazine, oxazine, thiazine, dioxine, dithiin, triazine, and tetrazine.

More preferred embodiments of R¹⁻² include pyrrole, imidazole, imidazoline, pyrazole, pyrazoline, triazole, pyridine, diazine, triazine, and tetrazine.

More preferred embodiments of R¹⁻² include imidazole, pyrazole, triazole, pyridine, diazine, and triazine.

Still more preferably, R¹⁻² is pyridine.

R¹⁻² may be further substituted with a C1-3 alkyl group or halogen atom.

The C1-3 alkyl group as used herein may be linear or branched. Specific examples thereof include a methyl group, an ethyl group, a propyl group, a methylethyl group, and the like.

In addition, the halogen atom as used herein preferably includes a fluorine atom, a chlorine atom, a bromine atom, an iodine atom, and the like.

Preferably 1 to 3, more preferably 1 to 2 and more preferably 1 of hydrogen atoms of R¹⁻² may be substituted.

In a preferred embodiment, when R¹ is R¹⁻¹, it is preferable that L is present.

Also, when R¹ is R¹⁻², it is preferable that L is not present or is not substituted by the R³.

In the general formula (I), R² is preferably a 3- to 20-membered ring, more preferably a 4- to 18-membered ring, more preferably a 6- to 16-membered ring, more preferably a 6- to 14-membered ring, more preferably a 6- to 12-membered ring, and more preferably a 8- to 12-membered ring.

R² may be monocyclic or fused bicyclic.

When R² is a monocyclic ring, R² is preferably a 3-to 12-membered ring, more preferably a 4- to 10-membered ring, more preferably a 5- to 8-membered ring, and more preferably a 5- to 6-membered ring.

When R² is a fused bicyclic ring, R² is preferably a 5- to 20-membered ring, more preferably a 6- to 18-membered ring, more preferably a 8- to 16-membered ring, more preferably a 8- to 12-membered ring, more preferably a 8-to 10-membered ring, and more preferably a 9- to 10-membered ring.

When R² is a fused bicyclic ring, R² is preferably a fused bicyclic ring of a 4-membered ring and a 4-membered ring, a 4-membered ring and a 5-membered ring, a 4-membered ring and a 6-membered ring, a 5-membered ring and a 5-membered ring, a 5-membered ring and a 6-membered ring, or a 6-membered ring and a 6-membered ring.

R² may be saturated or unsaturated. When unsaturated, the number of double bonds is preferably 1 to 6, more preferably 2 to 6, more preferably 3 to 6, and more preferably 4 to 5.

R² is preferably an aromatic ring, and more preferably a fused bicyclic aromatic ring.

R² may contain a heteroatom.

The number of heteroatoms contained in R² is preferably 1 to 6, more preferably 1 to 5, more preferably 1 to 4, and more preferably 1 to 3.

Examples of the hetero atom include an oxygen atom, a nitrogen atom, and a sulfur atom, and more preferably include a nitrogen atom.

The number of heteroatoms independently selected from an oxygen atom, a nitrogen atom, and a sulfur atom is 1 to 6, more preferably 1 to 5, more preferably 1 to 4, and more preferably 1 to 3.

R² is preferably an aromatic heterocyclic ring containing a hetero atom, more preferably an aromatic heterocyclic ring containing a nitrogen atom, and more preferably a fused bicyclic aromatic heterocyclic ring containing a nitrogen atom.

R² preferably has any of the following structures.

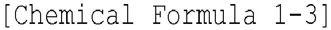

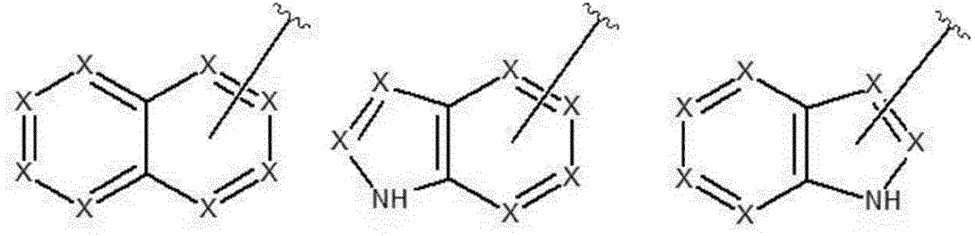

X in the above formula represents a carbon atom or a nitrogen atom. Among the atoms represented by X, the total number of nitrogen atoms is 1 to 6, more preferably 1 to 5, more preferably 1 to 4, and more preferably 1 to 3, and the remainder is carbon atoms.

### [1-1-2-2] General formula (II)

### [1] Compound of present invention

In one embodiment of the present invention, a compound included in the following general formula (II) or a salt thereof or a hydrate of the compound or salt is administered to an animal as a candidate substance.

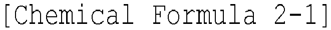

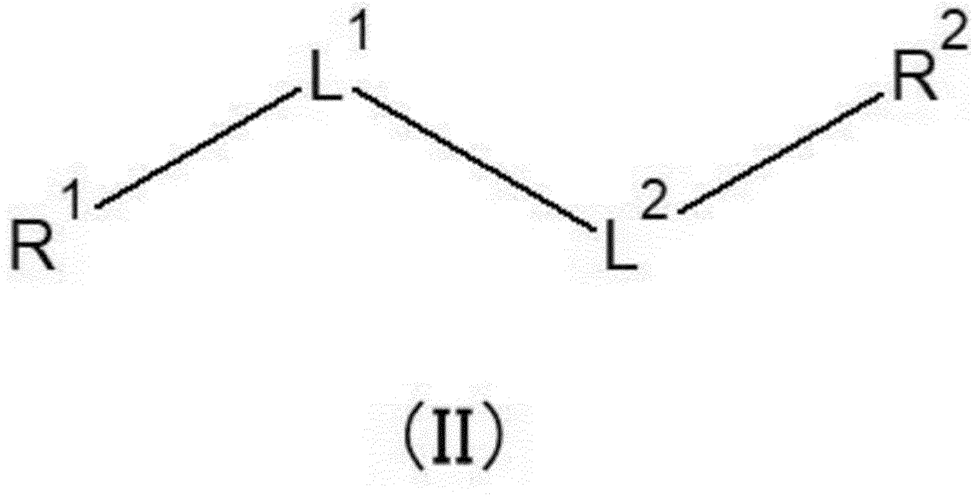

In the general formula (II), R¹ is a hydrogen atom, a halogen atom, or a C1-20 hydrocarbon group.

Here, examples of the halogen atom preferably include a fluorine atom, a chlorine atom, a bromine atom, and an iodine atom.

Hereinafter, a case where R¹ is a hydrocarbon group will be described in more detail.

Here, R¹ is preferably C1-18, more preferably C1-16, more preferably C1-14, more preferably C1-12, and more preferably C1-10.

R¹ may be saturated or unsaturated. Also, R¹ may be linear or branched. R¹ may contain a heteroatom. The heteroatom is preferably selected from an oxygen atom, a nitrogen atom, and a sulfur atom. More preferably, R¹ contains a nitrogen atom. R¹ may contain 1 to 4, more preferably 1 to 3, more preferably 1 to 2 heteroatoms independently selected from oxygen, nitrogen, and sulfur atoms.

Further, R¹ may have a cyclic structure. The cyclic structure is preferably a 3- to 8-membered ring, more preferably a 4- to 7-membered ring, and more preferably a 5- to 6-membered ring.

R¹ is preferably R¹⁻¹ or R¹⁻² below.

R¹⁻¹: a C1-20 hydrocarbon group which is saturated or unsaturated, linear or branched, and may be substituted with a halogen atom.

R¹⁻²: a 3- to 8-membered ring which is saturated or unsaturated, may be substituted with a halogen atom or a C1-3 hydrocarbon group, may contain 1 to 4 heteroatoms independently selected from an oxygen atom, a nitrogen atom, and a sulfur atom.

R¹⁻¹ is more preferably C1-18, more preferably C1-16, more preferably C1-14, more preferably C1-12, more preferably C1-10, more preferably C1-8, more preferably C1-6, more preferably C1-4, more preferably C1-3, and more preferably C1-2.

In addition, R¹⁻¹ may be substituted with a fluorine atom, a chlorine atom, a bromine atom, or an iodine atom.

The number of substitutions by halogen atoms can be set to preferably 1 to 3, more preferably 1 to 2, and more preferably 1.

R¹⁻² is preferably a monocyclic ring or a fused bicyclic ring, and more preferably a monocyclic ring.

When R¹⁻² is a monocyclic ring, R¹⁻² is preferably a 3- to 8-membered ring, more preferably a 4- to 6-membered ring, more preferably a 5- to 6-membered ring, and more preferably a 6-membered ring.

When R¹⁻² is a fused bicyclic ring, R¹⁻² is preferably a 4- to 8-membered ring, more preferably a 5- to 8-membered ring, more preferably a 5- to 7-membered ring, and more preferably a 5- to 6-membered ring.

R¹⁻² may be saturated or unsaturated. When unsaturated, the number of double bonds contained in the ring is preferably 1 to 3, more preferably 2 to 3, and more preferably 3.

Preferably, R¹⁻² is unsaturated. More preferably, R¹⁻² is an aromatic ring.

R¹⁻² may contain a heteroatom.

The number of heteroatoms contained in R¹⁻² is preferably 1 to 4, more preferably 1 to 3, more preferably 1 to 2, and more preferably 1.

Examples of the hetero atom include an oxygen atom, a nitrogen atom, and a sulfur atom, and more preferably include a nitrogen atom.

The number of heteroatoms independently selected from an oxygen atom, a nitrogen atom, and a sulfur atom is 1 to 4, more preferably 1 to 3, more preferably 1 to 2, and more preferably 1.

R¹⁻², in one embodiment, is preferably an aromatic heterocyclic ring containing a hetero atom, more preferably an aromatic heterocyclic ring containing a nitrogen atom, and more preferably a monocyclic aromatic heterocyclic ring containing a nitrogen atom.

Also, in another embodiment, R¹⁻² is an aromatic ring containing no heteroatom, and more preferably a monocyclic aromatic ring containing no heteroatom.

Specific examples of R¹⁻² include cyclopropane, cyclopropene, cyclobutane, cyclobutene, cyclobutadiene, cyclopentene, cyclopentadiene, cyclohexene, cyclohexadiene, benzene, cyclopentane, cycloheptene, cycloheptadiene, cycloheptatriene, cyclooctane, cyclooctene, cyclooctadiene, cyclooctatriene, aziridine, azirine, oxirane, oxylene, phosphirane, phosphylene, thiirane, thiirene, diaziridine, diazirine, oxaziridine, dioxirane, azetidine, azete, oxetane, oxete, thietane, thiete, diazetidine, diazete, dioxetane, dioxete, dithietane, dithiete, pyrrolidine, pyrrole, tetrahydrofuran, furan, tetrahydrothiophene, thiophene, imidazolidine, pyrazolidine, imidazole, imidazoline, pyrazole, oxazolidine, isoxazolidine, oxazole, oxazoline, isoxazole, thiazolidine, isothiazolidine, thiazole, isothiazole, dioxolane, dithiolane, triazole, furazan, oxadiazole, thiadiazole, dioxazole, dithiazole, tetrazole, oxatetrazole, thiatetrazole, pentazole, piperidine, pyridine, pyridinium cation, tetrahydropyran, pyran, pyrylium cation, thiane, thiopyran, thiopyrylium cation, piperazine, diazine, morpholine, oxazine, thiomorpholine, thiazine, dioxane, dioxine, dithiane, dithiine, hexahydro-1,3,5-triazine, triazine, trioxane, trithiane, tetrazine, pentazine, azepane, azepine, oxepane, oxepine, thiepane, thiepine, diazepane, diazepine, thiazepine, azocane, azocine, oxocane, oxocine, thiocane, thiocine, azonane, azonine, oxonane, oxonine, thionane, thionine, and the like.

More preferred embodiments of R¹⁻² include cyclopentene, cyclopentadiene, cyclohexene, cyclohexadiene, benzene, cyclopentane, cycloheptene, cycloheptadiene, cycloheptatriene, cyclooctane, cyclooctene, cyclooctadiene, cyclooctatriene, pyrrole, furan, thiophene, imidazole, imidazoline, pyrazole, pyrazoline, oxazole, oxazoline, isoxazole, thiazole, thiazoline, isothiazole, triazole, furazan, oxadiazole, thiadiazole, dioxazole, dithiazole, tetrazole, oxatetrazole, thiatetrazole, pentazole, pyridine, pyran, thiopyran, diazine, oxazine, thiazine, dioxine, dithiin, triazine, and tetrazine.

More preferred embodiments of R¹⁻² include benzene, pyrrole, imidazole, imidazoline, pyrazole, pyrazoline, triazole, pyridine, diazine, triazine, and tetrazine.

More preferred embodiments of R¹⁻² include benzene, imidazole, pyrazole, triazole, pyridine, diazine, and triazine.

Still more preferably, R¹⁻² is benzene or pyridine.

R¹⁻² may be further substituted with a C1-3 alkyl group or halogen atom.

The C1-3 alkyl group as used herein may be linear or branched. Specific examples thereof include a methyl group, an ethyl group, a propyl group, a methylethyl group, and the like.

In addition, the halogen atom as used herein preferably includes a fluorine atom, a chlorine atom, a bromine atom, an iodine atom, and the like.

Preferably 1 to 3, more preferably 1 to 2 and more preferably 1 of hydrogen atoms of the ring composed of R¹⁻² may be substituted.

In the general formula (II), L¹ is any of the following divalent groups.

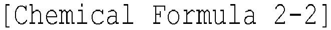

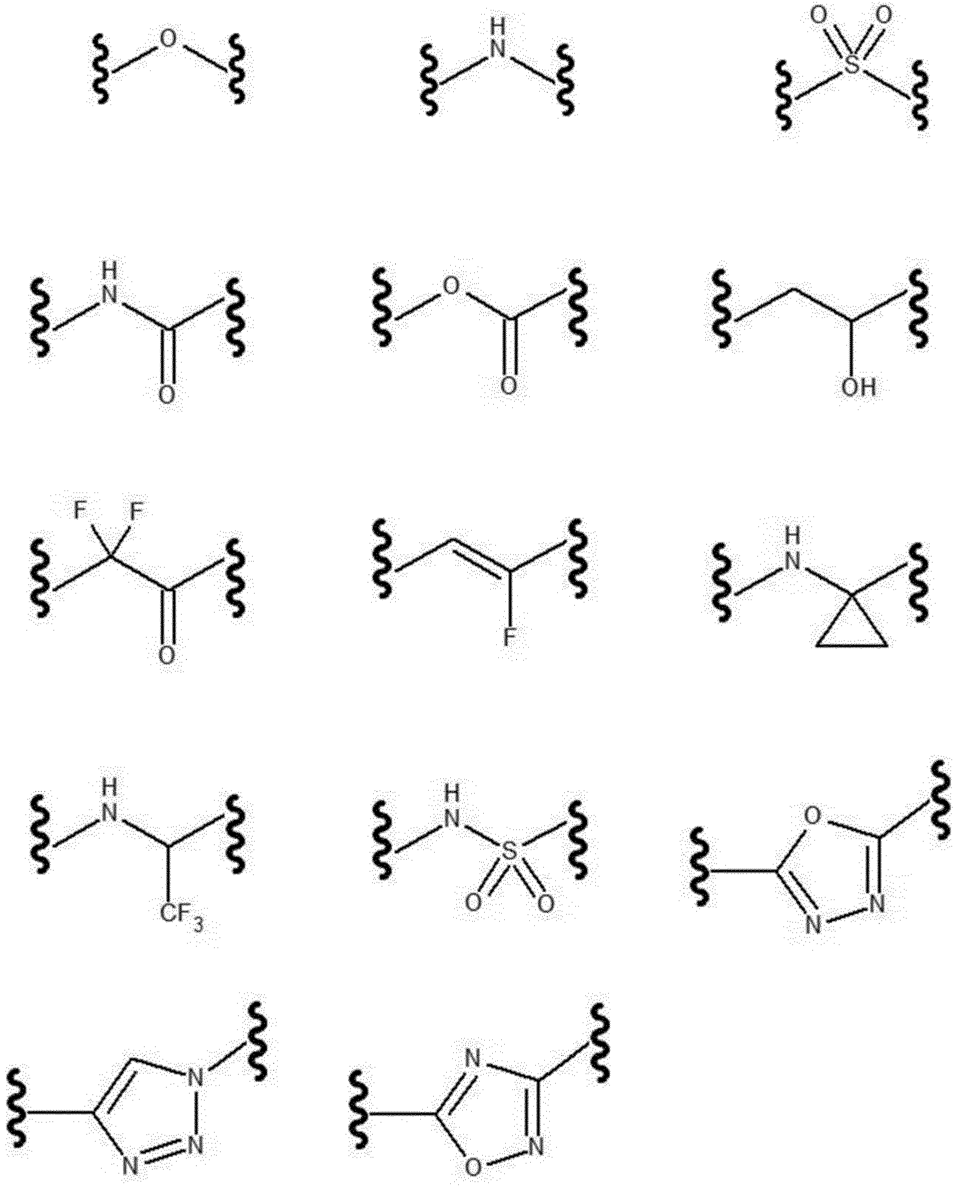

More preferably, L¹ is any of the divalent groups listed below. These are in a bioisostere relationship with each other.

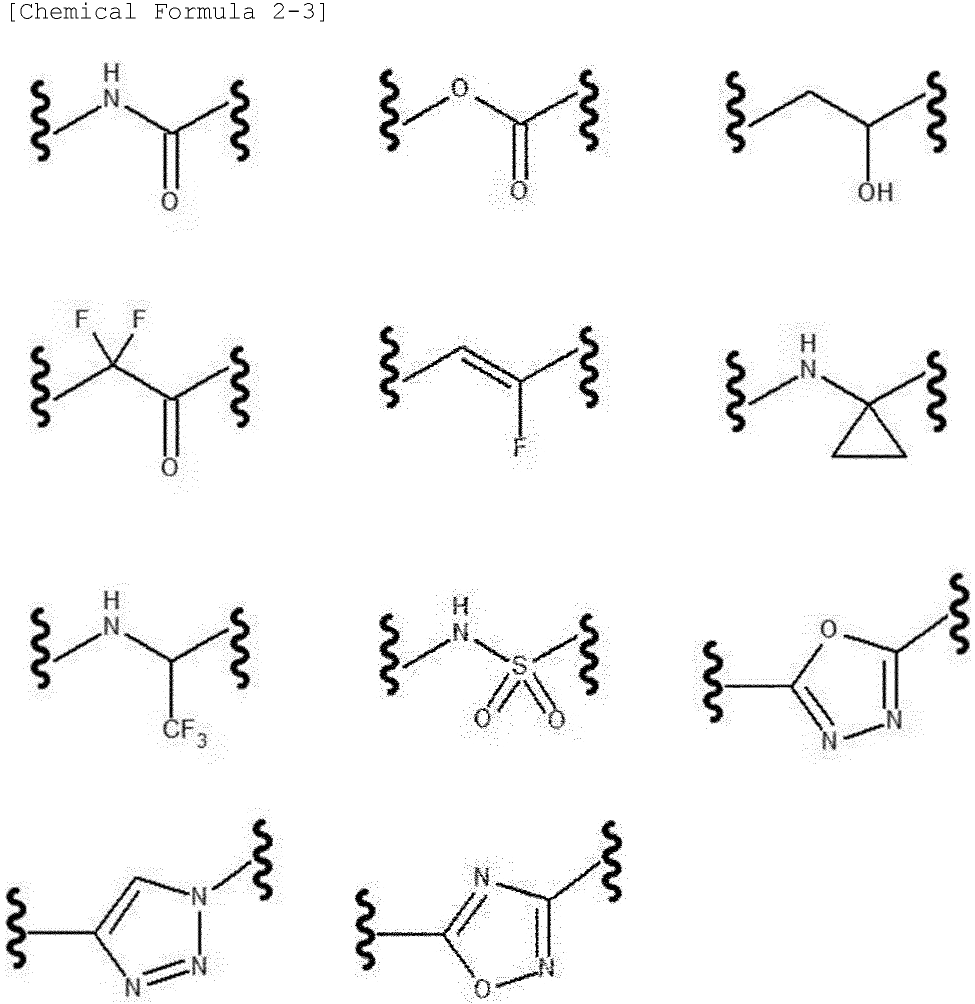

More preferably, L¹ is any of the divalent groups listed below.

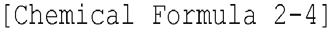

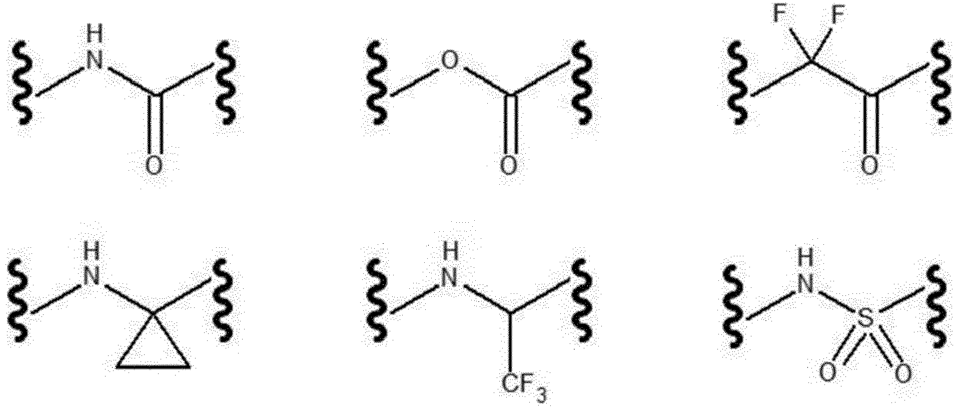

More preferably, L¹ is the following divalent group.

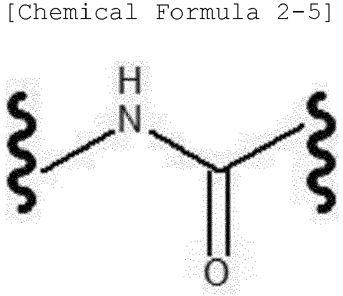

In the general formula (II), L² is a C1-30 hydrocarbon group. L² is more preferably C1-28, more preferably C1-26, more preferably C1-24, more preferably C1-22, more preferably C1-20, more preferably C1-18, more preferably C1-16, more preferably C1-14, more preferably C1-12, more preferably C1-10, and more preferably C1-8.

L² is linear. Further, L² may be saturated or unsaturated.

Furthermore, L² may be substituted with one or more substituents listed below.
- C1-3 Hydrocarbon group
- Hydroxyl group which may be substituted with C1-3 hydrocarbon group
- Amino group which may be substituted with C1-3 hydrocarbon group
- Sulfuric acid group which may be substituted with C1-3 hydrocarbon group
- Phosphoric acid group which may be substituted with C1-3 hydrocarbon group
- Halogen atom

More specifically, L² may be substituted with one or more substituents listed below.
- Methyl group, ethyl group, propyl group, or isopropyl group
- Hydroxyl group, methoxy group, ethoxy group, propoxy group, or isopropoxy group
- Amino group, or alkylamino group in which one or two hydrogen atoms of amino group are each independently substituted with methyl group, ethyl group, propyl group, or isopropyl group
- Sulfate group, methyl sulfate group, ethyl sulfate group, propyl sulfate group, or isopropyl sulfate group
- Phosphoric acid group, or alkyl phosphoric acid group in which one or two hydrogen atoms of phosphoric acid group are each independently substituted with methyl group, ethyl group, propyl group, or isopropyl group

More preferably, L² may be substituted with one or more substituents selected from a methyl group, an ethyl group, a propyl group, an isopropyl group, a hydroxyl group, an amino group, a sulfate group, and a phosphate group.

More preferably, L² may be substituted with one or more substituents selected from a methyl group, an ethyl group, a propyl group, an isopropyl group, a hydroxyl group, an amino group, a sulfate group, and a phosphate group.

More preferably, L² may be substituted with one or more substituents selected from a methyl group, an ethyl group, a propyl group, an isopropyl group, a hydroxyl group, and an amino group.

More preferably, L² may be substituted with one or more substituents selected from a methyl group, an ethyl group, a propyl group, an isopropyl group, and an amino group.

More preferably, L² may be substituted with an amino group.

The number of substitutions of L² is preferably 1 to 3, more preferably 1 to 2, and more preferably 1.

In the general formula (II), R² is any of the groups listed below.
- Carboxyl group which may be substituted with C1-3 hydrocarbon group
- Hydroxyl group which may be substituted with C1-3 hydrocarbon group
- Hydroxamic acid group which may be substituted with C1-3 hydrocarbon group
- Sulfo group which may be substituted with C1-3 hydrocarbon group
- Boronic acid group which may be substituted with C1-3 hydrocarbon group
- Carbamoyl group which may be substituted with C1-3 hydrocarbon group
- Sulfamoyl group which may be substituted with C1-3 hydrocarbon group
- Sulfoximine group which may be substituted with C1-3 hydrocarbon group
- Cyano group
- Tetrazolyl group

More preferably, R² is any of the groups listed below.
- Carboxyl group or group in which hydrogen atom of carboxy group is substituted with methyl group, ethyl group, propyl group, or isopropyl group
- Hydroxyl group, methoxy group, ethoxy group, propoxy group, or isopropoxy group
- Hydroxamic acid group or group in which hydrogen atom of hydroxamic acid group is substituted with methyl group, ethyl group, propyl group, or isopropyl group
- Sulfo group or group in which hydrogen atom of sulfo group is substituted with methyl group, ethyl group, propyl group, or isopropyl group
- Boronic acid group, or group in which one or two hydrogen atoms of boronic acid group are each independently substituted with methyl group, ethyl group, propyl group, or isopropyl group
- Carbamoyl group, or group in which one or two hydrogen atoms of carbamoyl group are each independently substituted with methyl group, ethyl group, propyl group, or isopropyl group
- Sulfamoyl group, or group in which one or two hydrogen atoms of sulfamoyl group are each independently substituted with methyl group, ethyl group, propyl group, or isopropyl group
- Sulfoximine group or group in which hydrogen atom of sulfoximine group is substituted with methyl group, ethyl group, propyl group, or isopropyl group
- Cyano group
- Tetrazolyl group

In one embodiment, R² is a carboxyl group, a hydroxyl group, a hydroxamic acid group, a sulfo group, a boronic acid group, a carbamoyl group, a sulfamoyl group, a sulfoximine group, a cyano group, or a tetrazolyl group.

More preferably, R² is a carboxyl group or a droxamic acid group.

In one embodiment, R² is a carboxyl group which may be substituted with a C1-3 hydrocarbon group or a hydroxamic acid group which may be substituted with a C1-3 hydrocarbon group.

### [1-1-2-3] General formula (III)

In one embodiment of the present invention, a compound included in the following general formula (III) or a salt thereof or a hydrate of the compound or salt is administered to an animal as a candidate substance.

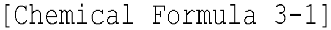

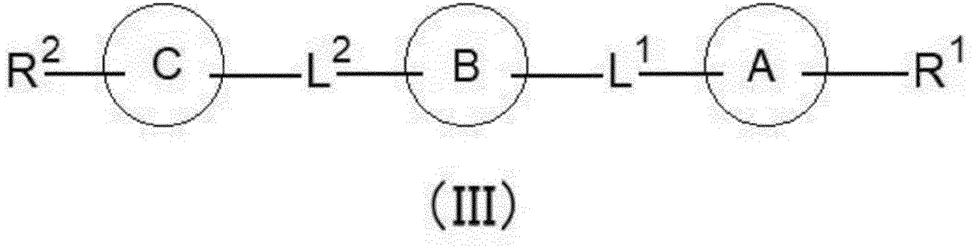

In the general formula (III), ring A, ring B, and ring C are 3- to 8-membered rings, more preferably 4- to 6-membered rings, more preferably 5- to 6-membered rings, and more preferably 6-membered rings.

Ring A, ring B, and ring C may be saturated or unsaturated. When unsaturated, the number of double bonds contained in the ring is preferably 1 to 3, more preferably 2 to 3, and more preferably 3.

Preferably, at least one, more preferably at least two, and more preferably all of ring A, ring B, and ring C are unsaturated. More preferably, ring A, ring B, and ring C are aromatic rings.

Ring A, ring B, and ring C may be substituted with one or more substituents listed below.
- Halogen atom
- C1-3 Hydrocarbon group
- Hydroxyl group which may be substituted with C1-3 hydrocarbon group
- Amino group which may be substituted with C1-3 hydrocarbon group
- Sulfuric acid group which may be substituted with C1-3 hydrocarbon group
- Phosphoric acid group which may be substituted with C1-3 hydrocarbon group

More specifically, ring A, ring B, and ring C may be substituted with one or more substituents listed below.
- Fluorine atom, chlorine atom, bromine atom, or iodine atom
- Methyl group, ethyl group, propyl group, or isopropyl group
- Hydroxyl group, methoxy group, ethoxy group, propoxy group, or isopropoxy group
- Amino group, or alkylamino group in which one or two hydrogen atoms of amino group are each independently substituted with methyl group, ethyl group, propyl group, or isopropyl group
- Sulfate group, methyl sulfate group, ethyl sulfate group, propyl sulfate group, or isopropyl sulfate group
- Phosphoric acid group, or alkyl phosphoric acid group in which one or two hydrogen atoms of phosphoric acid group are each independently substituted with methyl group, ethyl group, propyl group, or isopropyl group

Ring A, ring B, and ring C may contain a hetero atom.

The number of heteroatoms contained in ring A, ring B, and ring C is preferably 1 to 4, more preferably 1 to 3, more preferably 1 to 2, and more preferably 2.

Examples of the hetero atom include an oxygen atom, a nitrogen atom, and a sulfur atom, and more preferably include a nitrogen atom.

The number of heteroatoms independently selected from an oxygen atom, a nitrogen atom, and a sulfur atom is 1 to 4, more preferably 1 to 3, more preferably 1 to 2, and more preferably 1.

In one embodiment, any of ring A, ring B, and ring C is preferably an aromatic heterocyclic ring containing a hetero atom, and more preferably an aromatic heterocyclic ring containing a nitrogen atom.

Also, in another embodiment, any of ring A, ring B, and ring C is an aromatic ring containing no heteroatom.

In one embodiment, all of ring A, ring B, and ring C are aromatic rings, of which ring B is an aromatic heterocyclic ring containing a heteroatom, and ring A and ring C are aromatic rings containing no heteroatom.

In one embodiment, ring A is cyclohexane, cyclohexene, cyclohexadiene, or benzene, which may be further substituted with a C1-3 hydrocarbon group or a halogen atom.

In one embodiment, ring B is a 6-membered monocyclic heterocyclic ring which contains 1 to 3 nitrogen atoms, is saturated or unsaturated, and may further be substituted with a C1-3 hydrocarbon group or a halogen atom.

In one embodiment, ring C is cyclohexane, cyclohexene, cyclohexadiene, or benzene, which may be further substituted with a C1-3 hydrocarbon group, a halogen atom, or a hydroxyl group which may be substituted with a C1-3 hydrocarbon group.

Specific examples of ring A, ring B, and ring C include cyclopropane, cyclopropene, cyclobutane, cyclobutene, cyclobutadiene, cyclopentene, cyclopentadiene, cyclohexene, cyclohexadiene, benzene, cyclopentane, cycloheptene, cycloheptadiene, cycloheptatriene, cyclooctane, cyclooctene, cyclooctadiene, cyclooctatriene, aziridine, azirine, oxirane, oxylene, phosphirane, phosphylene, thiirane, thiirene, diaziridine, diazirine, oxaziridine, dioxirane, azetidine, azete, oxetane, oxete, thietane, thiete, diazetidine, diazete, dioxetane, dioxete, dithietane, dithiete, pyrrolidine, pyrrole, tetrahydrofuran, furan, tetrahydrothiophene, thiophene, imidazolidine, pyrazolidine, imidazole, imidazoline, pyrazole, oxazolidine, isoxazolidine, oxazole, oxazoline, isoxazole, thiazolidine, isothiazolidine, thiazole, isothiazole, dioxolane, dithiolane, triazole, furazan, oxadiazole, thiadiazole, dioxazole, dithiazole, tetrazole, oxatetrazole, thiatetrazole, pentazole, piperidine, pyridine, pyridinium cation, tetrahydropyran, pyran, pyrylium cation, thiane, thiopyran, thiopyrylium cation, piperazine, diazine, morpholine, oxazine, thiomorpholine, thiazine, dioxane, dioxine, dithiane, dithiine, hexahydro-1,3,5-triazine, triazine, trioxane, trithiane, tetrazine, pentazine, azepane, azepine, oxepane, oxepine, thiepane, thiepine, diazepane, diazepine, thiazepine, azocane, azocine, oxocane, oxocine, thiocane, thiocine, azonane, azonine, oxonane, oxonine, thionane, thionine, and the like.

More preferred embodiments of ring A, ring B, and ring C include cyclopentene, cyclopentadiene, cyclohexene, cyclohexadiene, benzene, cyclopentane, cycloheptene, cycloheptadiene, cycloheptatriene, cyclooctane, cyclooctene, cyclooctadiene, cyclooctatriene, pyrrole, furan, thiophene, imidazole, imidazoline, pyrazole, pyrazoline, oxazole, oxazoline, isoxazole, thiazole, thiazoline, isothiazole, triazole, furazan, oxadiazole, thiadiazole, dioxazole, dithiazole, tetrazole, oxatetrazole, thiatetrazole, pentazole, pyridine, pyran, thiopyran, diazine, oxazine, thiazine, dioxine, dithiin, triazine, and tetrazine.

More preferred embodiments of ring A, ring B, and ring C include benzene, pyrrole, imidazole, imidazoline, pyrazole, pyrazoline, triazole, pyridine, diazine, triazine, and tetrazine.

More preferred embodiments of ring A, ring B, and ring C include benzene, imidazole, pyrazole, triazole, pyridine, diazine, and triazine. As the diazine, pyrimidine is preferred.

Still more preferably, ring A, ring B, and ring C are benzene or pyrimidine. Still more preferably, ring A and ring C are benzene, and ring B is pyrimidine.

In one embodiment, ring A is cyclohexane, cyclohexene, cyclohexadiene, or benzene. More preferably, ring A is benzene.

In this case, it is preferable to have a mode in which R¹ and L¹ are preferably bonded to carbon atoms at 1 and 2-positions of ring A, respectively.

In one embodiment, ring B is a 6-membered monocyclic heterocyclic ring which contains 1 to 3 nitrogen atoms, is saturated or unsaturated, and is substituted with a halogen atom. More preferably, ring B is a pyrimidine substituted with a halogen atom. More preferably, ring B is a pyrimidine substituted with a chlorine atom.

In this case, it is preferable to have a mode in which L¹, L², and a chlorine atom are bonded to carbon atoms at 4-position, 2-position, and 5-position of ring B, respectively.

In one embodiment, ring C is cyclohexane, cyclohexene, cyclohexadiene, or benzene substituted with a C1-3 alkoxy group. More preferably, ring C is cyclohexane, cyclohexene, cyclohexadiene, or benzene substituted with a methoxy group. More preferably, ring C is benzene substituted with a methoxy group.

In this case, it is preferable to have a mode in which L², R², and a methoxy group are bonded to 1-position, 4-position, and 2-position of ring C, respectively.

In the general formula (III), R¹ is a group selected from the following.

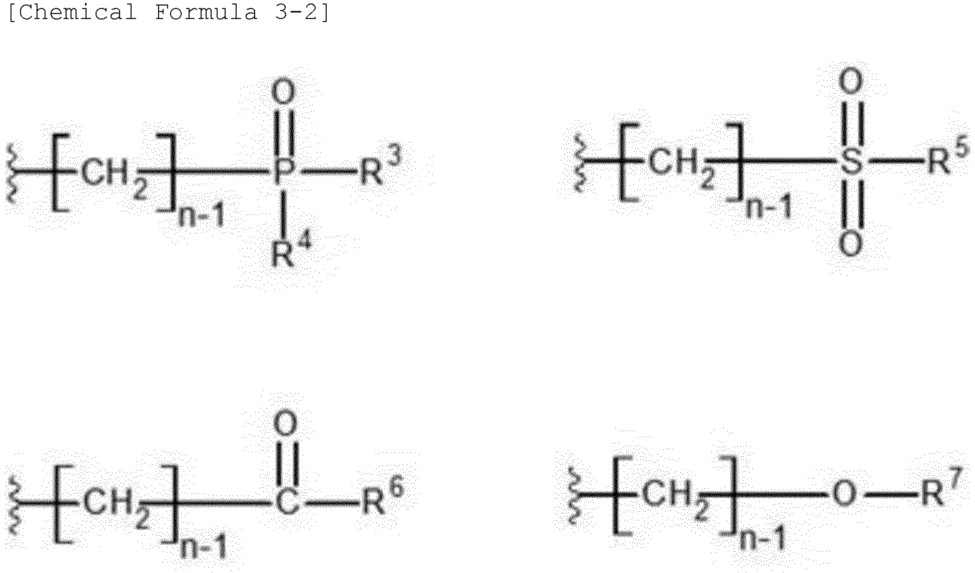

R¹ is more preferably a group selected from the following.

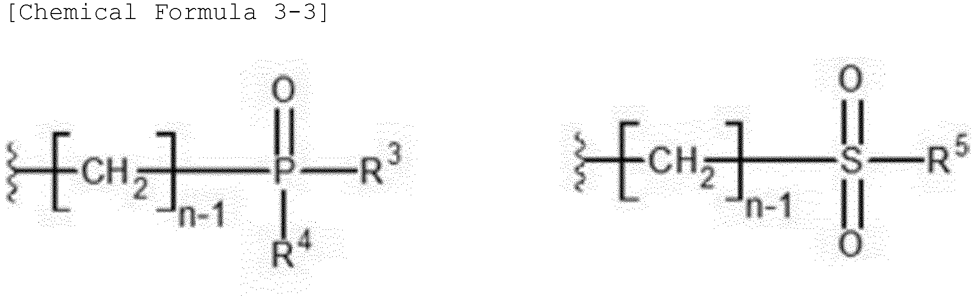

Here, R³ to R⁶ are each independently any of the following groups.
- Hydrogen atom
- C1-3 Hydrocarbon group
- Hydroxyl group which may be substituted with C1-3 hydrocarbon group
- Amino group which may be substituted with C1-3 hydrocarbon group

More preferably, R³ to R⁶ are each independently any of the following groups.
- Hydrogen atom
- Methyl group, ethyl group, propyl group, or isopropyl group
- Hydroxyl group, methoxy group, ethoxy group, propoxy group, or isopropoxy group
- Amino group, or alkylamino group in which one or two hydrogen atoms of amino group are each independently substituted with methyl group, ethyl group, propyl group, or isopropyl group

More preferably, R³ to R⁶ are each independently any of the following groups.
- Methyl group, ethyl group, propyl group, or isopropyl group
- Amino group, or alkylamino group in which one or two hydrogen atoms of amino group are each independently substituted with methyl group, ethyl group, propyl group, or isopropyl group

More preferably, R³ to R⁶ are each independently a methyl group or a monomethylamino group.

R⁷ is a hydrogen atom, a C1-3 hydrocarbon group, or an amino group which may be substituted with a C1-3 hydrocarbon group.

R⁷ is more preferably a methyl group, an ethyl group, a propyl group, an isopropyl group, a monomethylamino group, a monoethylamino group, a monopropylamino group, or a monoisopropylamino group.

R⁷ is more preferably a methyl group or a monomethylamino group.

n represents an integer of 1 to 4, more preferably an integer of 1 to 3, more preferably an integer of 1 to 2, and more preferably 1.

In the general formula (III), L¹ and L² are each independently a C1-3 alkylene group or alkenylene group, - N(H)-, or -O-.

More preferably, L¹ and L² are each independently a C1-2 alkylene group or alkenylene group, -N(H)-, or -O-.

More preferably, L¹ and L² are each independently a C1-2 alkylene group or alkenylene group or -N(H)-.

Among the above-described divalent groups, a group other than -O-, that is, a C1-3 alkylene group or alkenylene group and -N(H)- may be further substituted with a C1-30 hydrocarbon group. The C1-30 hydrocarbon group may be saturated or unsaturated. Also, the C1-30 hydrocarbon group may be linear or branched. Further, the C1-30 hydrocarbon group may have a cyclic structure or may be substituted with a halogen atom. Furthermore, the C1-30 hydrocarbon group may contain 1 to 6 heteroatoms independently selected from an oxygen atom, a nitrogen atom, and a sulfur atom.

In the general formula (III), R² is a C1-30, more preferably C2 to 28, more preferably C2 to 26, more preferably C2 to 24, more preferably C2 to 22, and more preferably C2 to 20 hydrocarbon group.

R² may be saturated or unsaturated. Also, R² may be linear or branched.

R² may have a cyclic structure. The number of ring structures contained in the structure of R² is preferably 1 to 3, and more preferably 1 to 2. The ring structure can be a 3- to 8-membered ring, preferably a 4- to 7-membered ring, and more preferably a 5- to 6-membered ring.

R² may be substituted with a halogen atom. Preferable examples of the halogen atom include a fluorine atom, a chlorine atom, a bromine atom, and an iodine atom. The number of halogen atoms to be substituted is preferably 1 to 4, more preferably 1 to 3, more preferably 1 to 2, and more preferably 1.

R² may contain 1 to 6, preferably 1 to 5, more preferably 1 to 4 heteroatoms independently selected from an oxygen atom, a nitrogen atom, and a sulfur atom. Preferable examples of the hetero atom include a nitrogen atom.

R² preferably has the following structure.

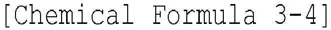

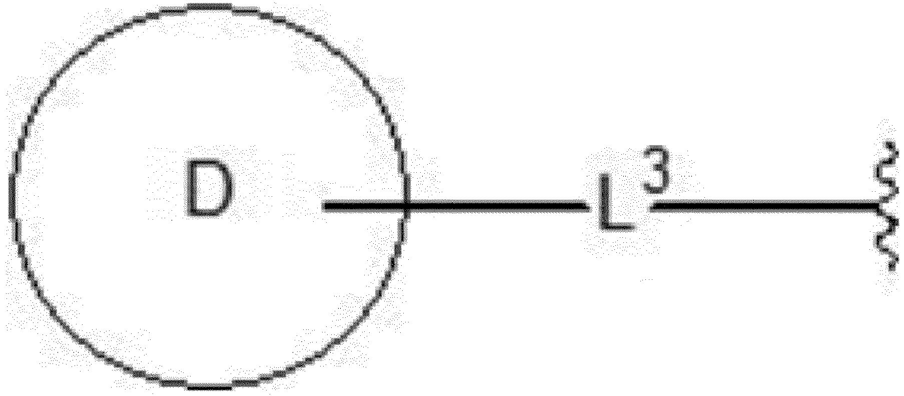

Ring D is a 3- to 8-membered ring, more preferably a 4- to 6-membered ring, more preferably a 5- to 6-membered ring, and more preferably a 6-membered ring.

Ring D may be saturated or unsaturated. When unsaturated, the number of double bonds contained in the ring is preferably 1 to 3, more preferably 2 to 3, and more preferably 3. Preferably, ring D is saturated.

Ring D may be substituted with one or more substituents listed below.
- Halogen atom
- C1-3 Hydrocarbon group
- Hydroxyl group which may be substituted with C1-3 hydrocarbon group
- Amino group which may be substituted with C1-3 hydrocarbon group
- Sulfuric acid group which may be substituted with C1-3 hydrocarbon group
- Phosphoric acid group which may be substituted with C1-3 hydrocarbon group

More specifically, ring D may be substituted with one or more substituents listed below.
- Fluorine atom, chlorine atom, bromine atom, or iodine atom
- Methyl group, ethyl group, propyl group, or isopropyl group
- Hydroxyl group, methoxy group, ethoxy group, propoxy group, or isopropoxy group
- Amino group, or alkylamino group in which one or two hydrogen atoms of amino group are each independently substituted with methyl group, ethyl group, propyl group, or isopropyl group
- Sulfate group, methyl sulfate group, ethyl sulfate group, propyl sulfate group, or isopropyl sulfate group
- Phosphoric acid group, or alkyl phosphoric acid group in which one or two hydrogen atoms of phosphoric acid group are each independently substituted with methyl group, ethyl group, propyl group, or isopropyl group

Ring D may contain a heteroatom.

The number of heteroatoms contained in ring D is preferably 1 to 4, more preferably 1 to 3, more preferably 1 to 2, and more preferably 2.

Examples of the hetero atom include an oxygen atom, a nitrogen atom, and a sulfur atom, and more preferably include a nitrogen atom.

The number of heteroatoms independently selected from an oxygen atom, a nitrogen atom, and a sulfur atom is 1 to 4, more preferably 1 to 3, more preferably 1 to 2, and more preferably 1.

Specific examples of ring D include cyclopropane, cyclopropene, cyclobutane, cyclobutene, cyclobutadiene, cyclopentene, cyclopentadiene, cyclohexene, cyclohexadiene, benzene, cyclopentane, cycloheptene, cycloheptadiene, cycloheptatriene, cyclooctane, cyclooctene, cyclooctadiene, cyclooctatriene, aziridine, azirine, oxirane, oxylene, phosphirane, phosphylene, thiirane, thiirene, diaziridine, diazirine, oxaziridine, dioxirane, azetidine, azete, oxetane, oxete, thietane, thiete, diazetidine, diazete, dioxetane, dioxete, dithietane, dithiete, pyrrolidine, pyrrole, tetrahydrofuran, furan, tetrahydrothiophene, thiophene, imidazolidine, pyrazolidine, imidazole, imidazoline, pyrazole, oxazolidine, isoxazolidine, oxazole, oxazoline, isoxazole, thiazolidine, isothiazolidine, thiazole, isothiazole, dioxolane, dithiolane, triazole, furazan, oxadiazole, thiadiazole, dioxazole, dithiazole, tetrazole, oxatetrazole, thiatetrazole, pentazole, piperidine, pyridine, pyridinium cation, tetrahydropyran, pyran, pyrylium cation, thiane, thiopyran, thiopyrylium cation, piperazine, diazine, morpholine, oxazine, thiomorpholine, thiazine, dioxane, dioxine, dithiane, dithiine, hexahydro-1,3,5-triazine, triazine, trioxane, trithiane, tetrazine, pentazine, azepane, azepine, oxepane, oxepine, thiepane, thiepine, diazepane, diazepine, thiazepine, azocane, azocine, oxocane, oxocine, thiocane, thiocine, azonane, azonine, oxonane, oxonine, thionane, thionine, and the like.

Ring D is preferably piperazine substituted with a C1-3 hydrocarbon group, and more preferably piperazine substituted with a methyl group.

L³ is a C1-20 divalent hydrocarbon group, more preferably a C1-18, more preferably a C1-16, more preferably a C1-14, more preferably a C1-12, more preferably a C1-10, and more preferably a C1-8 divalent hydrocarbon group.

L³ may be saturated or unsaturated. Also, L³ may be linear or branched.

L³ may have a cyclic structure. The number of ring structures contained in the structure of L³ is preferably 1 to 2, and more preferably 1. The ring structure can be a 3- to 8-membered ring, preferably a 4- to 7-membered ring, more preferably a 5- to 6-membered ring, and more preferably a 6-membered ring.

L³ may be substituted with a halogen atom. Preferable examples of the halogen atom include a fluorine atom, a chlorine atom, a bromine atom, and an iodine atom. The number of halogen atoms to be substituted is preferably 1 to 4, more preferably 1 to 3, more preferably 1 to 2, and more preferably 1.

L³ may contain 1 to 6, preferably 1 to 5, more preferably 1 to 4 heteroatoms independently selected from an oxygen atom, a nitrogen atom, and a sulfur atom. Preferable examples of the hetero atom include a nitrogen atom.

L³ is preferably a divalent group having the following structure.

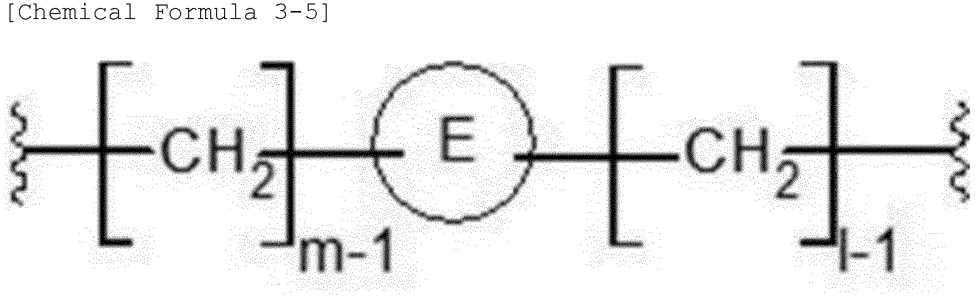

Here, m and l each independently represent an integer of 1 to 4, preferably 1 to 3, and more preferably 1 to 2. More preferably, m and l are 1.

Ring E is a 3- to 8-membered ring, more preferably a 4- to 6-membered ring, more preferably a 5- to 6-membered ring, and more preferably a 6-membered ring.

Ring E may be saturated or unsaturated. When unsaturated, the number of double bonds contained in the ring is preferably 1 to 3, more preferably 1 to 2, and more preferably 1. Preferably, ring E is a saturated ring.

Ring E may be a heterocycle. In this case, the number of heteroatoms contained in ring E is preferably 1 to 4, more preferably 1 to 3, more preferably 1 to 2, and more preferably 1.

Examples of the hetero atom include an oxygen atom, a nitrogen atom, and a sulfur atom, and more preferably include a nitrogen atom.

The number of heteroatoms independently selected from an oxygen atom, a nitrogen atom, and a sulfur atom is 1 to 4, more preferably 1 to 3, more preferably 1 to 2, and more preferably 1.

Specific examples of ring E include cyclopropane, cyclopropene, cyclobutane, cyclobutene, cyclobutadiene, cyclopentene, cyclopentadiene, cyclohexene, cyclohexadiene, benzene, cyclopentane, cycloheptene, cycloheptadiene, cycloheptatriene, cyclooctane, cyclooctene, cyclooctadiene, cyclooctatriene, aziridine, azirine, oxirane, oxylene, phosphirane, phosphylene, thiirane, thiirene, diaziridine, diazirine, oxaziridine, dioxirane, azetidine, azete, oxetane, oxete, thietane, thiete, diazetidine, diazete, dioxetane, dioxete, dithietane, dithiete, pyrrolidine, pyrrole, tetrahydrofuran, furan, tetrahydrothiophene, thiophene, imidazolidine, pyrazolidine, imidazole, imidazoline, pyrazole, oxazolidine, isoxazolidine, oxazole, oxazoline, isoxazole, thiazolidine, isothiazolidine, thiazole, isothiazole, dioxolane, dithiolane, triazole, furazan, oxadiazole, thiadiazole, dioxazole, dithiazole, tetrazole, oxatetrazole, thiatetrazole, pentazole, piperidine, pyridine, pyridinium cation, tetrahydropyran, pyran, pyrylium cation, thiane, thiopyran, thiopyrylium cation, piperazine, diazine, morpholine, oxazine, thiomorpholine, thiazine, dioxane, dioxine, dithiane, dithiine, hexahydro-1,3,5-triazine, triazine, trioxane, trithiane, tetrazine, pentazine, azepane, azepine, oxepane, oxepine, thiepane, thiepine, diazepane, diazepine, thiazepine, azocane, azocine, oxocane, oxocine, thiocane, thiocine, azonane, azonine, oxonane, oxonine, thionane, thionine, and the like.

Preferably, ring E is cyclohexane or piperidine, and more preferably piperidine.

Also, in one embodiment, L³ is not present.

### [1-1-2-4] Derivative of specific compound

In one embodiment of the present invention, a derivative of any one of Compounds 1 to 7 below or a salt thereof or a hydrate of the compound or salt is administered to an animal as a candidate substance.
Compound 1: 3-Cyclopentyl-3-[4-(7H-pyrrolo[2,3-d]pyrimidin-4-yl)-1H-pyrazol-1-yl]propanenitrile
Compound 2: 4-[3-(6-Methylpyridin-2-yl)-1H-pyrazol-4-yl]quinoline
Compound 3: 2-Amino-5-(ethylamino)-5-oxopentanoic acid
Compound 4: N¹-Hydroxy-N⁸-phenyloctanediamino
Compound 5: 4-[(5-Bromopyridin-2-yl)amino]-4-oxobutyric acid
Compound 6: 5-Chloro-2-N-{4-[4-(dimethylamino)piperidin-1-yl]-2-methoxyphenyl}-4-N-[2-(dimethylphosphoryl)phenyl]pyrimidine-2,4-diamine
Compound 7: 2-[[5-Chloro-2-[2-methoxy-4-(4-methylpiperazin-1-yl)anilino]pyrimidin-4-yl]amino]-N-methylbenzenesulfonamide

The structures of these seven compounds are shown below.

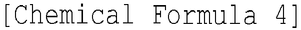

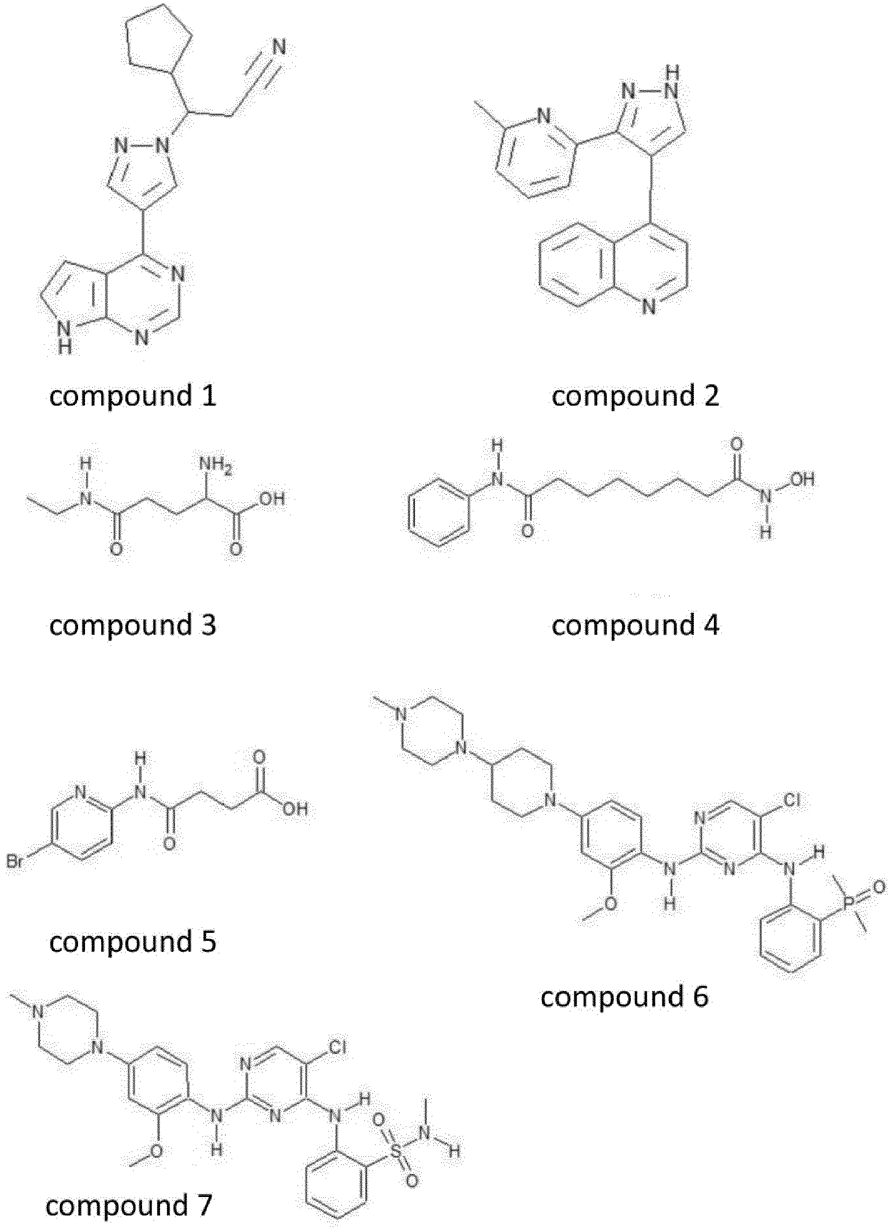

These seven compounds have been confirmed to have a therapeutic or preventive effect on the disease or the like of the nervous system in Examples described later. Therefore, a derivative obtained by adding an arbitrary group to the structure of these seven compounds, substituting a part of the structure with an arbitrary structure or deleting a part of the structure is highly likely to have a therapeutic or preventive effect on the disease or the like of the nervous system as with the seven compounds.

Therefore, by using derivatives of these seven compounds as candidate substances, it is possible to efficiently screen active ingredients that exhibit a therapeutic or preventive effect on the disease or the like of the nervous system.

When derivatives of these seven compounds are used as candidate substances, an embodiment including the following step A', step B and/or step C, and step D' may be adopted.
[step A'] a step of selecting any of the seven compounds as a lead compound and administering a derivative of the lead compound as a candidate substance to an animal (excluding human);
[step B] a step of observing neural undifferentiated cells in the animal that has undergone step A;
[step C] a step of observing the nervous system differentiated cells in the animal that has undergone step A; and
[step D'] a step of selecting the candidate substance as the active ingredient when the candidate substance has a proliferation action of neural undifferentiated cells and/or nervous system differentiated cells as compared with the case of administering the lead compound.

By adopting such an embodiment, optimization can be performed using any of the seven compounds as the lead compound.

### [1-2] Step B

Step B is a step of observing undifferentiated cells in the animal that has undergone step A.

In the present specification, the "undifferentiated cell" includes both a stem cell and a precursor cell.

Means for observing undifferentiated cells is not particularly limited. Suitable examples thereof include means for observing a marker of undifferentiated cells and means for observing a reporter gene specifically expressed in the undifferentiated cell. Hereinafter, details of each means will be described.

First, the means for observing markers of undifferentiated cells will be described. Cells have different morphological and functional characteristics for each developmental process. The reason why the characteristics are changed in the developmental process as described above is that expression of various genes is temporally and spatially controlled in each developmental process. Specifically, in the most undifferentiated stem cells, many genes necessary for suppressing differentiation and performing self-renewal while maintaining totipotency are expressed. As differentiation proceeds, the expression of genes maintaining totipotency is suppressed, while many genes required for taking a certain specific embodiment and exhibiting a certain specific function are expressed.

In embryology, as a method for tracking this developmental process, it is widely conducted to observe expression of "marker gene" specifically expressed in a certain developmental process.

In a preferred embodiment of the present invention, the marker specifically expressed in the undifferentiated cell is observed in step B. Hereinafter, the types of marker genes will be outlined.

Examples of markers of neural stem cells (neuroepithelial cells) include Nestin, SOX2, Notch1, HES1, HES3, Occludin, E-cadherin, SOX10, and the like.

In addition, when zebrafish are used as a screening tool, Her-5 can be preferably exemplified as a neural stem cell marker (Development 133(21): 4293-303 • December 2006) .

Preferable examples of markers of Schwann precursor cells include SOX10, GAP43, BLBP, MPZ, Dhh, P75NTR, and the like.

Examples of markers of radial glial cells include Vimentin, PAX6, HES1, HES5, GFAP, EAAT1/GLAST, BLBP, TN-C, N-cadherin, Nestin, SOX2, and the like.

Examples of markers of oligodendrocyte progenitor cells include PDGFRA and NG2.

Examples of markers of intermediate progenitor cells include TBR2 and MASH1.

Examples of markers of retinal stem cells or retinal progenitor cells include Pax6, Sox2, Nestin, vimentin, musashi, and Chx10 (Vsx2).

Examples of markers of cardiac undifferentiated cells include Mesp1, Nkx2.5, and the like.

Examples of markers of pancreatic undifferentiated cells include Pdx1, Ptfla, and the like.

Some of the above-described markers are also expressed in differentiated cells. The present invention may be an embodiment in which a gene expressed in both undifferentiated cells and differentiated cells as described above is used as a marker. This case corresponds to a mode in which step B and step C are simultaneously performed (Fig. 5(c)).

Preferred examples of the means for observing these markers include immunostaining using an antibody against a protein which is each gene product. The specific method of immunostaining is not particularly limited, and immunostaining can be performed by a conventional method.

Alternatively, the marker may be observed by in situ hybridization using hybridization between mRNA that is a transcript of each gene and a single-stranded nucleic acid molecule (probe) having a complementary base sequence. A specific method of the in situ hybridization is not particularly limited, and the hybridization can be performed by a conventional method.

Next, a method using a transgenic animal into which a reporter gene specifically expressed in the undifferentiated cell has been introduced will be described.

The reporter gene refers to a foreign gene for visualizing expression of a certain gene in a cell. By inserting a base sequence encoding a fluorescent protein or the like under an expression control region of a gene whose expression is desired to be visualized, the cell is designed to emit fluorescence in synchronization with the expression of the gene.

In one embodiment of the present invention, a transgenic animal into which a reporter gene specifically expressed in the undifferentiated cell has been introduced is used as a screening tool. In this case, the expression of the reporter gene specifically expressed in the undifferentiated cell in step B is observed.

Such transgenic animals can be prepared by conventionally used known gene editing techniques.

In a case where a promoter that specifically and positively controls expression in undifferentiated cells is known, a base sequence in which a reporter gene sequence is connected downstream of the promoter is inserted into a genome, whereby a transgenic animal that specifically expresses in a specific cell can be prepared.

In addition, since the marker gene of the undifferentiated cell is known as described above, a transgenic animal can be prepared by introducing a reporter gene into a locus of the marker gene. As a result, the reporter gene is expressed in synchronization with the expression of the marker gene.

Examples of the reporter genes include fluorescent proteins such as Sirius, EBFP, ECFP, mTurquoise, TagCFP, AmCyan, mTFP1, MidoriishiCyan, CFP, GFP, TurboGFP, AcGFP, TagGFP, Azami-Green, ZsGreen, EmGFP, EGFP, GFP2, HyPer, TagYFP, EYFP, Venus, YFP, PhiYFP, PhiYFP-m, TurboYFP, ZsYellow, KusabiraOrange, mOrange, TurboRFP, DsRed-Express, DsRed2, TagRFP, DsRed-Monomer, AsRed2 Red, mStrawberry, TurboFP602, mRFP1, JRed, KillerRed, mCherry, KeimaRed, mRasberry, mPlum, PS-CFP, Dendra2, Kaede, EosFP, and KikumeGR, and genes encoding proteins that catalyze a color reaction such as LacZ.

Although a specific method for designing reporter gene introduction is not limited, it is preferable to use a transgenic animal designed to introduce a cDNA sequence of a reporter gene into an arbitrary position of an open reading frame of a marker gene, more preferably an arbitrary position that does not destroy the function or topology of a protein that is a product of the marker gene, and express a fusion protein of the marker gene and the reporter gene.

Such transgenic lines into which a reporter gene specifically expressed in the undifferentiated cell has been introduced have been variously established, and any available line may be used. Hereinafter, some examples of transgenic lines into which a reporter gene specifically expressed in a neural undifferentiated cell has been introduced will be described, but it goes without saying that the embodiment of the present invention is not limited thereto.

- Her5-GFP transgenic zebrafish line (Development. 2003 Sep; 130(18): 4307-23.)
- Nestin-GFP transgenic zebrafish line (Developmental Dynamics 2009 Feb; 238(2): 475-86.)
- Gfap-GFP transgenic zebrafish line (Developmental Dynamics 2009 Feb; 238(2): 475-86.)
- Nestin-GFP transgenic mouse line (The Journal of comparative neurology, Volume 469, Issue 39 February 2004, Pages 311-324)

When the reporter gene is a fluorescent protein, fluorescence emitted by irradiating the transgenic animal with light at an excitation wavelength of the fluorescent protein is observed.

Fluorescence can be observed with a fluorescence microscope. Based on the brightness and area of the fluorescent portion, proliferation of undifferentiated cells in the body of the animal can be quantified.

When the reporter gene is a fluorescent protein, it is preferable because undifferentiated cells can be visualized temporally and dynamically in a living state of the transgenic animal as a screening tool.

In order to maximize such an effect, the transgenic animal is preferably an animal having a light body color or being transparent to translucent. Such animals include zebrafish.

In the case of using zebrafish embryos, it is preferable to add a melanin production inhibitor (for example, phenylthiourea) to the culture solution in step A in order to suppress production of melanin pigment.

### [1-3] Step C

Step C is a step of observing differentiated cells in the animal that has undergone step A. More specifically, a quantitative parameter and a qualitative parameter of the differentiated cell are observed. Examples of the quantitative parameter include the number of cells, the size and range of tissues, and the like. Examples of the qualitative parameter include the function and shape of the tissue composed of differentiated cells, and the like. When the function of the tissue is observed, the function to be observed is selected according to the type of the tissue to be observed. For example, when the heart is observed, the cardiac function can be evaluated by observing stroke volume and the like. In addition, in the case of observing the pancreas, the function of the pancreas can be evaluated by observing the amount of insulin, which is a secretion of the pancreas, and the like.

In the present specification, the "differentiated cell" means a cell having no self-renewal ability.

The term "nervous system differentiated cell" includes various neurons and glial cells. In addition, in the present specification, immature neural cells are divided into dividing neural progenitor cells and non-dividing neural progenitor cells. The former is included in neural undifferentiated cells, and the latter is included in nervous system differentiated cells.

Means for observing differentiated cells is not particularly limited. Suitable examples thereof include means for observing a marker of a differentiated cell and means for observing a reporter gene specifically expressed in the differentiated cell. Hereinafter, details of each means will be described.

In a preferred embodiment of the present invention, the marker specifically expressed in the differentiated cell is observed in step C. Hereinafter, the types of marker genes will be outlined.

Among immature neurons, those classified as non-dividing neural progenitor cells do not undergo further cell division unlike neural stem cells and radial glial cells which are cells of the immature nervous system. Those cells are cells that travel through the nervous system to reach a destination and then extend neurites to make synaptic connection, and ultimately become a member of the neural network.

Examples of markers of immature neurons classified as non-dividing neural progenitor cells include Doublecortin, NeuroD1, TBR1, Beta III tubulin, Stathmin 1, and the like.

Acetylated tubulin is a marker of stabilized microtubule. Moreover, microtubules present near cell bodies of elongated axons in neurons are composed of acetylated tubulin that is stable and has a long lifespan.

Thus, acetylated tubulin is useful as a marker of elongated mature neurons of the axon.

In addition, examples of the markers of mature neurons include NeuN, MAP2, Beta III tubulin, 160 kD Neurofilament, 200 kD Neurofilament, NSE, PSD93, PSD95, and the like.

More specifically, examples of markers of glutamatergic neurons include vGluT1, vGluT2, Glutaminase, Glutamine synthetase, NMDAR1, NMDAR2B, and the like.

Examples of markers of GABAergic neurons include GABA transporter 1, GABAB Receptor 1, GABAB Receptor 2, GAD65, GAD67, ABAT, and the like.

Examples of markers of dopaminergic neurons include Tyrosine Hydroxylase, dopamine transporter, FOXA2, GIRK2, LMX1B, Nurr1, and the like.

Examples of markers of serotonergic neurons include Tryptophan Hydroxylase, Serotonin Transporter, Pet1, and the like.

Examples of markers of cholinergic neurons include Acetylcholinesterase, ChAT, VAChT, and the like.

Also, specific examples of markers of glial cells include the following.

Examples of markers of myelinated Schwann cells include SOX10, S100, EGR2, MBP, MPZ, and the like.

Examples of markers of non-myelinated Schwann cells include SOX10, S100, GAP43, NCAM, P75NTR, and the like.

Examples of markers of oligodendrocytes include Olig1, Olig2, Olig3, OSP, MBP, MOG, SOX10, and the like.

Examples of markers of astrocytes include GFAP, EAAT1/GLAST, EAAT2/GLT-1, Glutamine synthetase, S100 beta, ALDH1L1, and the like.

Examples of markers of retinal ganglion cells include Thy-1, Sdk1, Sdk2, and the like.

Further, examples of markers of cardiomyocytes include GATA-4, α-Sarcomeric Actin, α-Sarcomeric Actinin, Slow Myosin Heavy Chain, Troponin T-C, MYL-2, and the like.

Examples of mature pancreatic β cell markers include insulin, C-peptide, MAFA, NKX6.1, PDX1, Fltp, and the like.

Some of the above-described markers are also expressed in undifferentiated cells. The present invention may be an embodiment in which a gene expressed in both undifferentiated cells and differentiated cells as described above is used as a marker. This case corresponds to a mode in which step B and step C are simultaneously performed (Fig. 5(c)).

Suitable examples of the means for observing these markers include immunostaining and in situ hybridization. The observation of differentiated cells by these observation means can be performed by a conventional method.

In one embodiment of the present invention, a transgenic animal into which a reporter gene specifically expressed in the differentiated cell has been introduced is used as a screening tool. In this case, the expression of the reporter gene specifically expressed in the differentiated cell is observed in step C.

Such transgenic animals can be prepared by conventionally used known gene editing techniques in the same manner as described in the description item of step B above. The type of reporter gene is also similar to that described in the description item of step B above.

Transgenic lines into which a reporter gene specifically expressed in the differentiated cell has been introduced have been variously established, and any available line may be used. Some examples of transgenic lines into which a reporter gene specifically expressed in the nervous system differentiated cell has been introduced will be described, but it goes without saying that the embodiment of the present invention is not limited thereto.

- Huc-GFP transgenic zebrafish line (Developmental Biology 227, 279-293 (2000))
- Ggaf-GFP transgenic zebrafish line (Developmental Dynamics 2009 Feb; 238(2): 475-86.)

When the reporter gene is a fluorescent protein, fluorescence emitted by irradiating the transgenic animal with light at an excitation wavelength of the fluorescent protein is observed.

Fluorescence can be observed with a fluorescence microscope. Based on the brightness of the fluorescence, proliferation of differentiated cells in the body of the animal can be quantified.

When the reporter gene is a fluorescent protein, it is preferable because differentiated cells can be visualized temporally and dynamically in a living state of the transgenic animal as a screening tool.

In order to maximize such an effect, the transgenic animal is preferably an animal having a light body color or being transparent to translucent. Such animals include zebrafish.

In the case of using zebrafish embryos, it is preferable to add a melanin production inhibitor (for example, phenylthiourea) to the culture solution in step A in order to suppress production of melanin pigment.

### [1-4] Embodiment in which both step B and step C are performed

In the present item, an embodiment in which both step B and step C are performed (Fig. 4) will be further described. As described with reference to Fig. 5, the order of step B and step C is not particularly limited, and these steps may be performed simultaneously.

In addition, step B and step C may be performed on the same animal individual, or may be performed on another animal individual. In the latter case, the experimental conditions for the animal individuals to be subjected to each of step B and step C are as uniform as possible.

In a preferred mode of the present invention, step B and step C are performed on the same animal individual. Hereinafter, an embodiment in which step B and step C are performed on the same animal individual will be described.

In one mode of the present invention, the undifferentiated cell marker is observed in step B, and the differentiated cell marker is also observed in step C.

In such a form, it is preferable to have an embodiment in which the animal that has undergone step A is fixed for staining, and multiple staining is performed with an antibody/probe against an undifferentiated cell marker and a differentiated cell marker. By multiple staining, undifferentiated cells and differentiated cells can be simultaneously observed.

When multiple staining is performed, labeling of the antibody/probe for the undifferentiated cell marker and the differentiated cell marker is designed so as not to be confused, according to a conventional method. In the case of a fluorescent label, a label with an excitation wavelength and a fluorescent wavelength different from each other is used.

In one mode of the present invention, a transgenic animal into which a reporter gene specifically expressed in the undifferentiated cell has been introduced is used. Then, the expression of the reporter gene is observed in step B, and the differentiated cell marker is observed in step C.

In this case, it is preferable that step B (fluorescence observation or the like) in which the animal can be observed in a living state is performed first, and then step C (immunostaining or the like) is performed.

By adopting such a form, it is possible to follow and observe self-renewal of undifferentiated cells over time and evaluate an effect of proliferation of differentiated cells as a result of the proliferation of undifferentiated cells.

In one mode of the present invention, a transgenic animal into which a reporter gene specifically expressed in the differentiated cell has been introduced is used. Then, the undifferentiated cell marker is observed in step B, and the expression of the reporter gene specifically expressed in the differentiated cell is observed in step C.

In this case, it is preferable that step C (fluorescence observation or the like) in which the animal can be observed in a living state is performed first, and then step B (immunostaining or the like) is performed.

By adopting such a form, it is possible to follow and observe proliferation of differentiated cells over time and evaluate an effect of self-renewal of undifferentiated cells as a cause of the proliferation of differentiated cells.

In another mode of the present invention, the animal used as a screening tool is a transgenic animal into which both a reporter gene specifically expressed in the undifferentiated cell and a reporter gene specifically expressed in the differentiated cell have been introduced. Then, the expression of the reporter gene specifically expressed in the undifferentiated cell is observed in step B, and the expression of the reporter gene specifically expressed in the differentiated cell is observed in step C.

By using a transgenic animal into which two of the reporter gene for undifferentiated cell and the reporter gene for differentiated cell are introduced as described above, both step B and step C can be performed temporally and dynamically in a living state of the animal. In addition, since step B and step C can be performed simultaneously, the burden on the animal is also small.

When the reporter gene is a fluorescent protein gene, the reporter gene for undifferentiated cell and the reporter gene for differentiated cell are preferably genes encoding fluorescent proteins having excitation wavelengths and fluorescence wavelengths different from each other.

### [1-5] Step D

Step D is a step of selecting an active ingredient by evaluating the observation results of step B and/or step C. Specifically, step D is a step of selecting, as an active ingredient, a candidate substance that provides quantitative and/or qualitative improvement of undifferentiated cells and/or differentiated cells, as compared with a case where the candidate substance is not administered.

In the case of performing step B, in step D, a candidate substance that improves the amount of undifferentiated cells as compared with the case where the candidate substance is not administered is selected as an active ingredient.

In the case of performing step C, in step D, a candidate substance that improves the amount of differentiated cells as compared with the case where the candidate substance is not administered is selected as an active ingredient. In addition, a candidate substance that improves the function of the tissue composed of differentiated cells as compared with the case where the candidate substance is not administered is selected as an active ingredient.

The effect of promoting proliferation of undifferentiated cells and/or differentiated cells can be appropriately evaluated according to the specific embodiments of step B and step C.

For example, when immunostaining or in situ hybridization is performed in step B and/or step C, a proliferation effect of the cells can be evaluated by using intensity and range of fluorescence or color in a stained image as an index. That is, when the intensity and range of fluorescence or color in the stained image are strong or wide, it can be discriminated that the candidate substance has an effect of promoting proliferation of undifferentiated cells and/or differentiated cells.

The proliferation effect of undifferentiated cells and/or differentiated cells is preferably evaluated by using the result of a control experiment performed under the same conditions as a comparison target except that the candidate substance is not administered.

The control experiment may be performed simultaneously with steps A to D, or the result of the control experiment performed once may be recorded and evaluated with reference to the recorded result.

### [1-6] Two-stage screening

As described above, the present invention includes step A, step B and/or step C, and step D. An embodiment in which steps A to D are performed twice or more may be employed. That is, the active ingredients selected by performing steps A to D as primary screening may be further subjected to steps A to D as secondary screening as candidate substances. In this case, it is preferable to change conditions of the primary screening and the secondary screening.

For example, an embodiment using an embryo in the primary screen and using an adult in the secondary screen may be employed.

In addition, an animal more advanced than the animal used in the primary screening may be used in the secondary screening. Specific examples include embodiments in which fish are used in the primary screening and mammals are used in the secondary screening.

In one embodiment of the present invention, the following step A₁ and step A₂ are included as step A, the following step B₁ and step B₂ are included as step B, the following step C₁ and step C₂ are included as step C, and the following step D₁ and step D₂ are included as step D. Either one or both of step B₁ and step C₁ may be performed, and either one or both of step B₂ and step C₂ may be performed.
[step A₁] a step of administering a candidate substance to an embryo of an animal;
[step B₁] a step of observing undifferentiated cells in the embryo of an animal that has undergone step A₁;
[step C₁] a step of observing differentiated cells in the embryo of an animal that has undergone step A₁;
[step D₁] a step of selecting, as an active ingredient, as a result of step B₁ and/or step C₁, a candidate substance that improves the amount of undifferentiated cells and/or differentiated cells, as compared with a case where the candidate substance is not administered;
[step A₂] a step of administering the active ingredient selected in step D₁ to an adult of an animal;
[step B₂] a step of observing undifferentiated cells in the adult of an animal that has undergone step A₂;
[step C₂] a step of observing differentiated cells in the adult of an animal that has undergone step A₂; and
[step D₂] a step of selecting, as an active ingredient, as a result of step B₂ and/or step C₂, a candidate substance that improves the amount of undifferentiated cells and/or differentiated cells, as compared with a case where the candidate substance is not administered.

Steps A₁ to D₁ are primary screening using an embryo and the later steps A₂ to D₂ are secondary screening using an adult animal. By performing two-stage screening in this manner, it is possible to more accurately screen active ingredients.

Preferably, steps A₁ to D₁ are primary screening using fish embryos and the later steps A₂ to D₂ are secondary screening using adult mammals. In the primary screening, it is possible to easily perform the primary screening in a large amount by using fish embryos. Then, by using adult mammals in the secondary screening, it is possible to select an active ingredient that is highly likely to be effective in humans.

Preferably, steps A₁ to D₁ are primary screening using zebrafish embryos and the later steps A₂ to D₂ are secondary screening using adult mice. In the primary screening, it is possible to easily perform the primary screening in a large amount by using zebrafish embryos. Then, by using adult mice in the secondary screening, it is possible to select an active ingredient that is highly likely to be effective in humans.

In one embodiment of the present invention, the following step A₁ and step A₂ are included as step A, the following step B₁ and step B₂ are included as step B, the following step C₁ and step C₂ are included as step C, and the following step D₁ and step D₂ are included as step D. Either one or both of step B₁ and step C₁ may be performed, and either one or both of step B₂ and step C₂ may be performed.
[step A₁] a step of administering a candidate substance to an embryo of fish;
[step B₁] a step of observing undifferentiated cells in the embryo of an animal that has undergone step A₁;
[step C₁] a step of observing differentiated cells in the embryo of an animal that has undergone step A₁;
[step D₁] a step of selecting, as an active ingredient, as a result of step B₁ and/or step C₁, a candidate substance that improves the amount of undifferentiated cells and/or differentiated cells, as compared with a case where the candidate substance is not administered;
[step A₂] a step of administering the active ingredient selected in step D₁ to mammals;
[step B₂] a step of observing undifferentiated cells in the mammals that have undergone step A₂;
[step C₂] a step of observing differentiated cells in the mammals that have undergone step A₂; and
[step D₂] a step of selecting, as an active ingredient, as a result of step B₂ and/or step C₂, a candidate substance that improves the amount of undifferentiated cells and/or differentiated cells, as compared with a case where the candidate substance is not administered.

Steps A₁ to D₁ are primary screening using fish embryos and the later steps A₂ to D₂ are secondary screening using mammals. By performing two-stage screening in this manner, it is possible to more accurately screen active ingredients. In step A₂, an embodiment using either adults or embryos of mammals may be employed.

In the primary screening, it is possible to easily perform the primary screening in a large amount by using fish embryos. Then, by using mammals in the secondary screening, it is possible to select an active ingredient that is highly likely to be effective in humans.

### [2] Second screening method

Next, an embodiment of the second screening method will be described.

The second screening method includes the following steps E to G.
[step E] a step of administering a candidate substance to a model animal (excluding human) of a disease, disorder, or illness;
[step F] a step of determining a therapeutic effect of a disease, disorder, or illness in the animal that has undergone step E; and
[step G] a step of selecting a candidate substance having a therapeutic effect as the active ingredient.

### [2-1] Step E

In the second screening method, a model animal of a disease or the like is used (step E). Preferably, a model animal of a disease of nervous system, heart, or pancreas is used. It is also preferable to use a model animal for cancer.

For a specific embodiment of step E, the above-described description of step A is appropriate, and in particular, the description regarding the model animal of a disease or the like is appropriate as it is.

The model animal for cancer is not particularly limited, and not only one in which a cancer suppressor gene is deleted by genetic engineering technique but also a model animal prepared by transplanting tumor cells or tumor tissues into an immunodeficient animal can be used. Various types of cancer model animals are available for a fee or free, and these can be used without limitation according to the purpose.

Examples of the model animal of hematological cancer, particularly leukemia include a T-cell acute lymphocytic leukemia model mouse (p53null, Ezh2 conditional KO) (Non Patent Literature 26), a Cre/lox regulatory transgenic zebrafish model having conditional myc-induced T-cell acute lymphoblastic leukemia (Non Patent Literature 27), and the like.

Model animals of diabetes include type 1 diabetes model mice such as NOD/ShiJcl, and type 2 diabetes model mice such as KK/TaJcl, KK-A^{y}/TaJcl, BKS.Cg-m⁺/⁺Lepr^{db}/Jcl, GK/Jcl, SDT/Jcl, and SDT fatty/Jcl. In addition, drug-induced diabetes model animals such as type 1 diabetes models of rats, mice, and zebrafish induced with streptozotocin (STZ) may also be used.

Model animals of cardiomyopathy include mice, rats, and zebrafish in which cardiomyopathy is induced by doxorubicin (DOX) which causes cardiac toxicity, particularly left ventricular dysfunction.

In step E, it is preferable to adopt a mode in which the candidate substance is locally administered to a site which is damaged or functionally declined by suffering from a disease or the vicinity thereof. In addition, the candidate substance may be administered in the form of oral administration, transdermal administration, enteral administration, or the like. The dosage form can be appropriately designed depending on the type of disease.

In the case of administration to a glaucoma model, it is preferable to drop or apply a candidate substance to the retina.

In the case of administration to a spinal cord injury model, it is preferable to administer a candidate substance to a spinal cord injury site.

In the case of administration to a hematological cancer model, a diabetes model, or a cardiomyopathy model, it is preferable to inject and administer a candidate substance to a blood vessel.

### [2-2] Step F

In step F, the therapeutic effect of a disease, disorder, or illness in the animal that has undergone step E is determined.

The method for determining the therapeutic effect includes observation of appearance and behavior of the disease model animal, anatomical and pathological diagnosis, and the like, and can be appropriately designed in accordance with properties of the disease or the like with which the disease model animal is affected.

Specific examples of observation of appearance and behavior of the disease model include the following.

For example, the therapeutic effect in a depression model can be determined by forced swim test (mouse, rat) or tail suspension test (mouse).

The therapeutic effect in an anxiety model can be determined by Vogel type conflict test (rat) and social interaction test (rat).

The therapeutic effect in a spinal cord injury model can be determined by using the presence or absence of recovery of motor function lost due to spinal cord injury as an index.

The therapeutic effect in a glaucoma model can be determined by a two-choice visual discrimination task using an escape reaction from water as an index (Prusky, West, & Douglas (2000)), a discrimination task in a craving situation using food as a reward (Gianfranceschi, Fiorentini, & Maffei (1999)), a test using a visual movement reaction (Douglas, Alam, Silver, Mcgill, Tschetter, & Prusky, 2005), and the like.

The therapeutic effect in a diabetes model can be determined based on indices such as measurement of insulin secretion level and measurement of blood glucose level.

The therapeutic effect in a cardiomyopathy model can determine recovery of the myocardial function weakened by the disorder, that is, recovery of the cardiac function, based on an index such as the stroke volume.

The therapeutic effect on cancer can be determined by using a tumor reduction effect, a tumor marker leaked into blood, or the like as an index.

In addition, the active ingredient selected by the above-described first screening method exhibits an anticancer effect by forcibly differentiating cancer cells into normal cells. Therefore, the therapeutic effect can be determined by using, as an index, whether or not differentiation of cancer cells into normal cells is observed.

The active ingredient selected in the above-described first screening method promotes forced differentiation of cancer cells into vascular endothelial cells when applied to hematological cancer such as leukemia. This detoxifies harmful cancer cells. Therefore, the therapeutic effect particularly on hematological cancer, for example, leukemia, is to observe the presence or absence of thickening or tumorigenesis of vascular endothelium. In addition, the presence or absence of thickening or tumorigenesis of vascular endothelium or lymphatic endothelium is also observed for cancer exhibiting humoral metastasis such as hematogenous metastasis or lymphatic metastasis.

Specific examples of the anatomical and pathological diagnosis may include the following.

The therapeutic effect in the glaucoma model can be determined by observing the presence or absence of an increase in damaged retinal ganglion cells. For a specific embodiment of the observation method of the presence or absence of an increase in retinal ganglion cells, the description in the item of the first screening method above is appropriate as it is.

The therapeutic effect in the spinal cord injury model can be determined by observing crushed spinal cord with a microscope, MRI, or the like and using the regeneration effect as an index.

In order to determine the therapeutic effect more accurately, it is preferable to perform a control experiment. The control experiment is preferably performed under the same conditions except that the candidate substance is not administered. The control experiment may be performed simultaneously with step E and step F, but the result of the control experiment acquired once is recorded, and the therapeutic effect may be determined in step F with reference to the recorded result.

### [2-3] Step G

Step G is a step of selecting a candidate substance determined to have a therapeutic effect as the active ingredient in step F.

### [3] Two-stage screening method

The present invention also relates to a two-stage screening method in which primary screening is performed by a first screening method, and a candidate substance selected as an active ingredient in the primary screening is subjected to a second screening method.

Embodiments of the first screening method and the second screening method are as described above.

Preferably, in step A of the first screening method, a disease model animal is not used, and a normal animal is used. That is, in the first screening method, the primary screening is performed on a large amount of candidate substances at low cost using a normal animal that can be acquired in a large amount at low cost. Then, after the number of candidate substances is reduced by the primary screening, the secondary screening using the disease model is performed. By performing two-stage screening in this manner, it is possible to efficiently and economically screen active ingredients effective for the disease or the like of the nervous system.

In one embodiment of the present invention, an embryo is used as a screening tool in the first screening.

In one embodiment of the present invention, fish embryos, more preferably zebrafish embryos are used as a screening tool in the first screening, and mammals, more preferably mice or rats are used as a screening tool in the second screening.

In one embodiment of the present invention, as a result of the first screening, a candidate substance selected as an active ingredient may be subjected to the second screening method. In this case, the second screening method may be performed on the basis of the result of the first screening performed by another institution. That is, the practitioner of the present invention may perform only the second screening method.

### [4] Production method

The present invention also relates to a method for producing a pharmaceutical composition, including a formulation step of mixing and formulating a substance selected as an active ingredient and a pharmaceutical additive by the above-described screening method.

The practitioner of the production method of the present invention may perform the above-described screening method by himself/herself. In addition, the invention of the production method may be implemented on the basis of a result of performing the above-described screening method by another person.

That is, one embodiment of the present invention includes a screening step of screening active ingredients by the above-described screening method. The embodiment of the screening step is as described above.

Further, in one embodiment of the present invention, the invention of the production method is performed using a substance already determined to be an active ingredient by the above-described screening method.

Hereinafter, the formulation step of mixing and formulating a substance selected as an active ingredient in screening and a pharmaceutical additive will be described in detail.

The dosage form and composition of the pharmaceutical composition can be appropriately designed depending on the disease to be treated or prevented or the like.

The pharmaceutical composition contains the above-described active ingredients and any pharmaceutical additive. When the pharmaceutical composition is in the form of a solid preparation, a dosage form of granule, dry syrup, fine granule, tablet, powder, capsule, or pill can be appropriately selected. A formulation method of these dosage forms is performed by a conventional method.

When the pharmaceutical composition is in the form of a solid preparation, a stabilizer may be contained. The stabilizing agent includes sodium chloride and potassium chloride, formic acid, oxalic acid, acetic acid, citric acid, ascorbic acid and fumaric acid, miglyols (medium chain fatty acid triglycerides), triethyl citrate and polyoxyethylene sorbitan monooleate, triacetin (glyceryl triacetate), glycerin fatty acid esters, acylglycerols, monoglyceride derivatives, and polyglycerin fatty acid esters.

When the pharmaceutical composition is in the form of a solid preparation, an excipient may be contained. Examples of the excipient include polysaccharides such as isomalt, erythritol, D-mannitol, xylitol, sorbitol, reduced maltose starch syrup (maltitol), lactitol, oligosaccharide alcohol, xylose, glucose, fructose, maltose, lactose, sucrose, fructose, trehalose, isomerized sugar, starch syrup, purified sucrose, sucrose, purified sucrose spherical granules, anhydrous lactose, sucrose and starch spherical granules, oligosaccharides, dextrins, and starch, partially digested starch, glucose hydrate, crystalline cellulose, microcrystalline cellulose, pullulan, β-cyclodextrin, aminoethyl sulfonic acid, maltose syrup powder, sodium chloride, citric acid, sodium citrate, glycine, calcium gluconate, L-glutamine, tartaric acid, potassium hydrogen tartrate, ammonium carbonate, dextran 40, dextrin, calcium lactate, povidone, macrogol (polyethylene glycol) 1500, macrogol 1540, macrogol 4000, macrogol 6000, anhydrous citric acid, DL-malic acid, sodium hydrogen phosphate, potassium dihydrogen phosphate, sodium dihydrogen phosphate, L-aspartic acid, alginic acid, carmellose sodium, hydrated silicon dioxide, crospovidone, calcium glycerophosphate, magnesium aluminosilicate, calcium silicate, magnesium silicate, light anhydrous silicic acid, synthetic aluminum silicate, wheat flour, wheat starch, wheat germ powder, wheat germ oil, rice flour, rice starch, cellulose acetate phthalate, titanium oxide, magnesium oxide, dihydroxyaluminum aminoacetate, calcium triphosphate, talc, calcium carbonate, magnesium carbonate, precipitated calcium carbonate, natural aluminum silicate, corn starch, corn starch granulated product, potato starch, hydroxypropylcellulose, hydroxypropyl starch, anhydrous calcium hydrogen phosphate, anhydrous calcium hydrogen phosphate granulated product, calcium dihydrogen phosphate, and the like.

It is also possible to form the pharmaceutical composition into the form of a solid preparation, suspend the solid preparation in water, and take the suspension. In this case, suspending agents include cellulose-based polymers such as carmellose, carmellose sodium, crystalline cellulose-carmellose sodium, hydroxypropylcellulose, hypromellose (hydroxypropyl methylcellulose), methylcellulose, carboxymethyl ethylcellulose, hydroxyethylcellulose, hydroxyethyl methylcellulose, hydroxypropyl methylcellulose acetate succinate, hydroxypropyl methylcellulose phthalate, and a mixture of fumaric acid, stearic acid, polyvinyl acetal diethylaminoacetate, and hydroxypropyl methylcellulose, acrylic polymers such as ethyl acrylate-methyl methacrylate copolymer dispersion, aminoalkyl methacrylate copolymer, methacrylate copolymer, 2-methyl-5-vinylpyridine methylacrylate-methacrylate copolymer, dry methacrylate acid copolymer, and dimethylamino ethyl methacrylate-methyl methacrylate copolymer, vinyl-based polymers such as polyvinylpyrrolidone, crospovidone, carboxyvinyl polymer, polyvinyl acetal diethylaminoacetate, polyvinyl alcohol, a polyvinyl alcohol-methyl methacrylate-acrylate copolymer, and a polyvinyl alcohol copolymer, sodium alginate, carrageenan, a carboxyvinyl polymer, a dried aluminum hydroxide gel, xanthan gum, magnesium aluminum silicate, sodium polyphosphate, macrogol 4000, macrogol 6000, and the like, and carmellose, carmellose sodium, crystalline cellulose-carmellose sodium, hydroxypropylcellulose, hypromellose, and polyvinylpyrrolidone can be preferably used, and hypromellose and the like can be more preferably used.

When the pharmaceutical composition is in the form of a solid preparation, a lubricant may be added to improve lubricity. Examples of the lubricant include light anhydrous silicic acid, hydrated silicon dioxide, sucrose fatty acid ester, stearyl alcohol, stearic acid, magnesium stearate, calcium stearate, sodium stearyl fumarate, talc, and the like.

When the pharmaceutical composition is a solid preparation, a flavoring substance may be added to a poor taste drug such as a bitter taste to correct the taste. Examples of the flavoring substance include ascorbic acid, aspartic acid, aspartame, sucralose, glycine, sodium chloride, magnesium chloride, hydrochloric acid, dilute hydrochloric acid, citric acid and its salts, anhydrous citric acid, L-glutamic acid and its salts, succinic acid and its salts, acetic acid, tartaric acid and its salts, sodium bicarbonate, fumaric acid and its salts, malic acid and its salts, glacial acetic acid, disodium inosinate, honey, reduced maltose syrup (maltitol), licorice, and the like.

When the pharmaceutical composition is a solid preparation, a binder may be contained. Examples of the binder include hydroxypropylcellulose, corn starch, pregelatinized starch, partially pregelatinized starch, gum arabic, gum arabic powder, gelatin, agar, dextrin, pullulan, polyvinylpyrrolidone, polyvinyl alcohol, crystalline cellulose, methylcellulose, ethylcellulose, carboxymethyl ethylcellulose, carmellose, carmellose sodium, hydroxyethylcellulose, hydroxyethyl methylcellulose, hydroxypropylcellulose, hypromellose, and the like.

When the pharmaceutical composition is a solid preparation, a disintegrant may be contained. Examples of the disintegrant include croscarmellose sodium, crospovidone, carmellose calcium, sodium carboxymethyl starch, low-substituted hydroxypropylcellulose, and the like.

When the pharmaceutical composition is a solid preparation, a colorant may be contained. Examples of the colorant include iron oxide, tar dyes, natural dyes, and the like. Examples of the iron oxide include iron sesquioxide, yellow iron oxide, yellow iron sesquioxide, black iron oxide, and the like.

When the pharmaceutical composition is a solid preparation, a flavoring agent or a sweetening agent may be further added as necessary.

Specific examples of the flavoring agent include orange essence, orange oil, caramel, camphor, cinnamon oil, spearmint oil, strawberry essence, chocolate essence, cherry flavor, spruce oil, pine oil, peppermint oil, vanilla flavor, strawberry flavor, bitter essence, fruit flavor, peppermint essence, mix flavor, mint flavor, menthol, lemon powder, lemon oil, rose oil, and the like.

Specific examples of the sweetening agent include aspartame, reduced maltose starch syrup (maltitol), licorice, xylitol, glycerin, saccharin, sucralose, D-sorbitol, acesulfame potassium, stevia, taumatin, adopantame, and the like.

In addition, the pharmaceutical composition can be a dosage form of a parenteral administration agent. For example, the dosage form can be a flowable dosage form such as intravenous injection, intracerebral injection, intrathecal injection, intramuscular injection, intraperitoneal injection, subcutaneous injection, suppository, enema, oral intestinal solvent, eye drop, ophthalmic ointment, or ointment. Such a flowable dosage form is advantageous when the active ingredient is directly administered to the affected area.

The liquid agent is preferably in the form of an injection or an eye drop. Also, the ointment is preferably in the form of an ophthalmic ointment.

When the pharmaceutical composition is an injection, examples of an aqueous medium include water (that is, water for injection) and a mixture of a water-miscible solvent and water. Examples of such a water-miscible solvent include alcohols (for example, aliphatic alcohols (for example, methanol, ethanol, and the like) or aryl alcohols), polyols, esters, alkyl halides, ethers, cyanides/nitriles, ketones, aldehydes, amines, amides, formamides, sulfides, sulfoxides, and carboxylic acids.

When the pharmaceutical composition is an injection, a pH adjusting agent may be added. As the pH adjusting agent, a pH adjusting agent usually used for injections can be used. Specifically, an acidic substance such as acetic acid, lactic acid, phosphoric acid, tartaric acid, citric acid, ascorbic acid, hydrochloric acid, gluconic acid, or sulfuric acid can be used in the case of being inclined to the acidic side, and a basic substance such as potassium hydroxide, sodium hydroxide, calcium hydroxide, magnesium hydroxide, monoethanolamine, diethanolamine, or triethanolamine can be used in the case of being inclined to the basic side.

When the pharmaceutical composition is an injection, a tonicity agent can be added. Examples of the tonicity agent include saccharides and sugar alcohols such as glucose, maltose, α-trehalose, sorbitol, and mannitol, polyhydric alcohols such as glycerin, propylene glycol, and polyethylene glycol, electrolytes such as sodium chloride, and the like.

When the pharmaceutical composition is an injection, a buffer can be added. Examples of the buffer include citrate buffers, acetate buffers (for example, sodium acetate hydrate), phosphate buffers, tartrate buffers, and the like.

When the pharmaceutical composition is used as an injection, a blending component usually used for an injection can be added. Examples of such a blending component include diluents, soothing agents, antiseptics, and the like.

The pharmaceutical composition may be formed in the form of a lyophilized injection capable of preparing an injection by adding an aqueous medium. The pharmaceutical composition for an injection in a lyophilized form can be produced by freeze-drying the pharmaceutical composition for an injection in an aqueous solution form described above by a conventional method.

More specifically, the pharmaceutical composition is preferably in the form of an injection for intracerebral administration, an injection for intracerebroventricular administration, or an injection for spinal cord administration.

The pharmaceutical composition can be in the form of an eye drop. In the form of an eye drop, a saccharide such as glucose, fructose, galactose, mannose, ribose, ribulose, arabinose, xylose, lyxose, deoxyribose, maltose, trehalose, sucrose, cellobiose, lactose, pullulan, lactulose, raffinose, or maltitol may be added.

When the pharmaceutical composition is in the form of an eye drop, nonionic surfactants such as a polyoxyethylene-polyoxypropylene block copolymer adduct of ethylenediamine (for example, poloxamine), POE sorbitan fatty acid esters such as POE (20) sorbitan monolaurate (polysorbate 20) and POE (20) sorbitan monooleate (polysorbate 80), POE hydrogenated castor oil such as POE (60) hydrogenated castor oil, POE alkyl ethers such as POE (9) lauryl ether, POE • POP alkyl ethers such as POE (20) POP (4) cetyl ether, and POE alkylphenyl ethers such as POE (10) nonylphenyl ether; amphoteric surfactants such as glycine type such as alkyldiaminoethylglycine, betaine acetate type such as lauryldimethylaminoacetate betaine, and imidazoline type; anionic surfactants such as POE alkyl ether phosphates such as POE (10) sodium lauryl ether phosphate and its salts, N-acyl amino acid salts such as sodium lauroyl methyl alanine, alkyl ether carboxylates, N-acyl taurine salts such as sodium N-cocoyl methyl taurine, sulfonates such as sodium tetradecene sulfonate, alkyl sulfates such as sodium lauryl sulfate, POE alkyl ether sulfates such as sodium POE (3) lauryl ether sulfate, and α-olefin sulfonates; cationic surfactants such as alkylamine salts, alkyl quaternary ammonium salts (benzalkonium chloride, benzethonium chloride, and the like), alkylpyridinium salts (cetylpyridinium chloride, cetylpyridinium bromide, and the like), and the like may be added.

When the pharmaceutical composition is an eye drop, an antiseptic, a disinfectant or an antibacterial agent may be added. Specific examples include sorbic acid or its salts (sorbic acid, potassium sorbate, sodium sorbate, triclocarban sorbate, and the like), paraoxybenzoic acid esters (methyl parahydroxybenzoate, ethyl parahydroxybenzoate, propyl parahydroxybenzoate, butyl parahydroxybenzoate, and the like), acrinol, methylrosanilinium chloride, benzalkonium chloride, benzethonium chloride, cetylpyridinium chloride, cetylpyridinium bromide, chlorhexidine, polyhexamethylene biguanide, alkylpolyaminoethylglycine, benzyl alcohol, phenethyl alcohol, chlorobutanol, isopropanol, ethanol, phenoxyethanol, a carrier such as silver of zirconium phosphate, thimerosal, dehydroacetic acid, chloroxylenol, chlorophene, resorcin, thymol, hinokitiol, sulfamine, lysozyme, lactoferrin, triclosan, 8-hydroxyquinoline, undecylenic acid, caprylic acid, propionic acid, benzoic acid, propionic acid, halocarban, thiabendazole, polymyxin B, 5-chloro-2-methyl-4-isothiazolin-3-one, 2-methyl-4-isothiazolin-3-one, polylysine, hydrogen peroxide, polidronium chloride, Glokill (trade name: for example, Glokill PQ, manufactured by Rhodia), polydiallyldimethylammonium chloride, poly[oxyethylene (dimethyliminio) ethylene-(dimethyliminio)ethylene dichloride, polycondensation products of polyethylenepolyamine-dimethylamine epichlorohydrin (trade name: for example, Busan 1157, manufactured by Buckman Laboratories, Inc.), and the like.

When the pharmaceutical composition is in the form of an eye drop, a pH adjusting agent may be added. Examples of the pH adjusting agent include inorganic acids (hydrochloric acid, sulfuric acid, phosphoric acid, polyphosphoric acid, boric acid, and the like), organic acids (lactic acid, acetic acid, citric acid, tartaric acid, malic acid, succinic acid, oxalic acid, gluconic acid, fumaric acid, propionic acid, acetic acid, aspartic acid, epsilon-aminocaproic acid, glutamic acid, aminoethylsulfonic acid, and the like), gluconolactone, ammonium acetate, inorganic bases (sodium bicarbonate, sodium carbonate, potassium hydroxide, sodium hydroxide, calcium hydroxide, magnesium hydroxide, and the like), organic bases (monoethanolamine, triethanolamine, diisopropanolamine, triisopropanolamine, lysine, and the like), borax, and the like.

When the pharmaceutical composition is an eye drop, a tonicity agent may be added. Examples of the isotonic agent include saccharides such as glucose, mannitol, and sorbitol, and inorganic salts such as sodium chloride, potassium chloride, sodium carbonate, sodium bicarbonate, calcium chloride, magnesium sulfate, sodium hydrogen phosphate, disodium hydrogen phosphate, dipotassium hydrogen phosphate, sodium thiosulfate, and sodium acetate.

When the pharmaceutical composition is used as an ophthalmic ointment, it can be prepared using any ointment base. The ointment base is not particularly limited, but fats and oils, a wax, a hydrocarbon compound, or the like as a hydrophobic base can be generally used. Specific examples thereof include mineral bases such as yellow petrolatum, white petrolatum, paraffin, liquid paraffin, plastibase, and silicone, animal and vegetable bases such as beeswax and animal and vegetable oils and fats, and the like.

In addition, it is known that there is a route in which an administered substance directly migrates to a cerebrospinal fluid (CSF) or a brain through a nasal mucosal layer without passing through blood in the nasal cavity (Ilium L., Eur. J. Pharm. Sci., 11, 1-18 (2000)., Frey W.H., Drug Deliv. Technol., 2, 46-49 (2002)., Lochhead J.J., Thorne G., Adv. Drug Deliv. Rev., 64, 614-628 (2012)., Djupesland P.G., Messina J.C., Mahmoud R.A., Ther. Deliv., 5, 709-733 (2014).).

Therefore, the pharmaceutical composition can be in the form of a nasal administration preparation in order to allow the active ingredient to reach the brain. Examples of the nasal administration preparation include dosage forms such as nasal drops, liquid nasal sprays, powder nasal sprays, nasal mucosal injections, gel preparations, and ointments.

When the pharmaceutical composition is a nasal drop, a surfactant may be added. Examples of the surfactant include nonionic surfactants such as polyoxyethylene alkyl ether, polyoxyethylene polypropylene alkyl ether, sorbitan fatty acid ester, polyoxyethylene sorbitan fatty acid ester, glycerin fatty acid ester, polyglycerin fatty acid ester, polyoxyethylene glycerin fatty acid ester, polyethylene glycol fatty acid ester, sucrose fatty acid ester, polyoxyethylene castor oil, polyoxyethylene hydrogenated castor oil, and polyoxyethylene polyoxypropylene polymer; amphoteric surfactants such as alkyl betaine, alkyl amide betaine, alkyl sulfobetaine, and imidazoline; anionic surfactants such as saturated higher fatty acid salts, alkyl sulfonates, alkyl ether sulfonates, alkyl ether sulfonates, and polyoxyethylene alkyl ether phosphates; and the like.

When the pharmaceutical composition is a nasal drop, a thickener may be added. Examples of the thickener include celluloses such as hydroxypropylcellulose, hydroxypropylmethylcellulose, carmellose, croscarmellose, and methylcellulose, processed starch such as partially gelatinized starch, polyvinyl alcohol, polyvinyl pyrrolidone, crospovidone, polyethylene glycol, carboxyvinyl polymer, acrylic acid-alkyl methacrylate copolymer, xanthan gum, carrageenan, alginic acid and its salts, gum arabic, guar gum, locust bean gum, pullulan, gelatin, sodium polyacrylate, and the like.

When the pharmaceutical composition is a nasal drop, an oily component may be added. Examples of the oily component include unsaturated aliphatic alcohols such as palmitoleyl alcohol, oleyl alcohol, eicosonyl alcohol, elaidyl alcohol, and linoleyl alcohol; unsaturated fatty acids such as oleic acid, elaidic acid, linoleic acid, undecylenic acid, myristoleic acid, palmitoleic acid, linderic acid, lauroleic acid, tsuzuic acid, petroselinic acid, vaccenic acid, and gondoic acid; unsaturated fatty acid esters such as glycerol monooleic acid ester, glycerol dioleic acid ester, octyldodecyl oleate, and oleyl oleate; saturated fatty acid esters such as cetyl octanoate, isopropyl myristate, and myristyl myristate; hydrocarbons such as oleyl alcohol, elaidyl alcohol, liquid paraffin, vaseline, and microcrystalline wax; silicon oils such as methylpolysiloxane, methylphenylpolysiloxane, and dimethylcyclopolysiloxane; waxes such as beeswax, higher alcohols such as cetyl alcohol and stearyl alcohol; sterols such as cholesterol; metal soaps such as aluminum stearate and magnesium stearate, avocado oil, palm oil, beef tallow, jojoba oil; and the like.

When the pharmaceutical composition is a nasal drop, a tonicity agent may be added. Examples of the tonicity agent include saccharides such as sorbitol, glucose, and mannitol, polyhydric alcohols such as glycerin, polyethylene glycol, and propylene glycol, inorganic salts such as sodium chloride and potassium chloride, and the like.

When the pharmaceutical composition is a nasal drop, a pH adjusting agent may be added. Examples of the pH adjusting agent include acetic acid, formic acid, lactic acid, tartaric acid, oxalic acid, glycolic acid, malic acid, citric acid, succinic acid, fumaric acid, phosphoric acid, hydrochloric acid, nitric acid, sulfuric acid, and salts thereof, sodium hydroxide, potassium hydroxide, calcium hydroxide, arginine, methylamine, ethylamine, propylamine, dimethylamine, diethylamine, dipropylamine, trimethylamine, triethylamine, tripropylamine, monomethanolamine, monoethanolamine, monopropanolamine, dimethanolamine, diethanolamine, dipropanolamine, trimethanolamine, triethanolamine, isopropanolamine, diisopropanolamine, tripropanolamine, aqueous ammonia, guanidine carbonate, sodium bicarbonate, ammonium carbonate, and the like.

When the pharmaceutical composition is a nasal drop, a buffer may be added. Examples of the buffer include boric acid and its salts, phosphates, acetates, amino acid salts, and the like.

When the pharmaceutical composition of the present invention is a nasal drop, a chelating agent may be added. Examples of the chelating agent include edetic acid, oxalic acid, citric acid, pyrophosphoric acid, hexametaphosphoric acid, gluconic acid, salts thereof, and the like.

When the pharmaceutical composition is a nasal drop, a flavoring agent/cooling agent may be added. Examples of the flavoring agent/cooling agent include flavoring agents such as peppermint oil, peppermint white oil, cinnamon oil, clove oil, fennel oil, castor oil, turpentine oil, eucalyptus oil, orange oil, lavender oil, lemon oil, rose oil, lemongrass oil, star anise oil, thyme oil, chenopodium oil, yamajin oil, legume oil, bergamot oil, citronella oil, camphor oil, rosemary, and sage; and cooling agents such as l-menthol, camphor, thymol, N-ethyl-p-menthane-carboxamide, p-menthane-3,8-diol, l-isopulegol, and l-menthyl glyceryl ether.

When the pharmaceutical composition is a nasal drop, an antioxidant may be added. Examples of the antioxidant include ascorbic acid, propyl gallate, butylhydroxyanisole, dibutylhydroxytoluene, nordihydroguaiaretic acid, tocopherol, tocopherol acetate, and the like.

When the pharmaceutical composition is a nasal drop, an antiseptic may be added. Examples of the antiseptic include thymol, isopropylmethylphenol, benzoic acid and salts thereof, methyl benzoate, ethyl parahydroxybenzoate, propyl parahydroxybenzoate, butyl parahydroxybenzoate, benzyl alcohol, benzalkonium chloride, benzethonium chloride, and the like.

When the pharmaceutical composition is a nasal drop, an absorption promoting agent may be added. Examples of the absorption promoting agent include diisopropyl adipate, lecithin, squalane, squalene, l-menthol, polyethylene glycol, isopropyl myristate, dimethyl sulfoxide, peppermint oil, eucalyptus oil, d-limonene, dl-limonene, and the like.

When the pharmaceutical composition is a nasal drop, a suspending agent may be added. Examples of the suspending agent include celluloses such as methyl cellulose, sodium carboxymethyl cellulose, and hydroxypropyl methyl cellulose; synthetic polymer compounds such as polyvinyl alcohol, polyvinyl pyrrolidone, and carboxyvinyl polymer; nonionic surfactants such as polyoxyethylene alkyl ether, polyoxyethylene polypropylene alkyl ether, sorbitan fatty acid ester, polyoxyethylene sorbitan fatty acid ester, glycerin fatty acid ester, polyglycerin fatty acid ester, polyoxyethylene glycerin fatty acid ester, polyethylene glycol fatty acid ester, sucrose fatty acid ester, polyoxyethylene castor oil, polyoxyethylene hydrogenated castor oil, and polyoxyethylene polyoxypropylene polymer; amphoteric surfactants such as alkyl betaine, alkyl amide betaine, alkyl sulfobetaine, and imidazoline; anionic surfactants such as saturated higher fatty acid salts, alkyl sulfonates, alkyl ether sulfonates, alkyl ether sulfonates, and polyoxyethylene alkyl ether phosphates; and the like.

The pharmaceutical composition may be formed into a dosage form of a liquid agent, and the liquid agent may be charged in a sprayer to form a liquid nasal spray preparation. In this case, the sprayer for charging the pharmaceutical composition of the present invention is not particularly limited, and for example, a sprayer adopted in a liquid nasal spray preparation for treatment of allergic rhinitis can be used. In addition, as a specific form of the liquid agent to be charged into a sprayer, the above description of nasal drop is appropriate.

In addition, the pharmaceutical composition may be formed into a powder form, and the powder form may be charged in a sprayer to form a powder nasal spray preparation. In this case, the sprayer for charging the pharmaceutical composition of the present invention is not particularly limited, and for example, a sprayer adopted in a powder nasal spray preparation for treatment of allergic rhinitis can be used. In addition, the powder of the pharmaceutical composition to be charged in the sprayer can be produced by adopting a freeze drying method, a spray drying method, or the like.

When the pharmaceutical composition is a powder nasal spray preparation, any excipient may be added. Examples of the excipient include glucose, sucrose, lactose, and fructose; starches or starch derivatives; oligosaccharides such as dextrin, cyclodextrin, and derivatives thereof; polyvinylpyrrolidone, alginic acid, tylose, silicic acid, cellulose, cellulose derivatives (for example, cellulose ethers); sugar alcohols such as mannitol, sorbitol, arabinose, ribose, mannose, sucrose, trehalose, maltose, and dextran; calcium carbonate, calcium phosphate, lactose, lactitol, dextrates, dextrose, maltodextrin, and the like.

When the pharmaceutical composition is in the form of a nasal cavity mucosal injection which is a preparation for nasal administration, the description of the specific mode of the injection described above is appropriate for the specific mode.

When the pharmaceutical composition is in the form of an ointment which is a preparation for nasal administration, the description of the specific mode of the eye ointment described above is appropriate for the specific mode.

When the pharmaceutical composition of the present invention is a gel-like preparation, it can be gelated by adding a thickener. For other specific modes, the description of the specific mode of the nasal drop described above is appropriate.

### [5] Design method

The present invention also relates to a method for designing a pharmaceutical composition, including the step of selecting a pharmaceutical additive to be combined with a substance selected as an active ingredient by the above-described screening method.

The practitioner of the design method of the present invention may perform the above-described screening method by himself/herself. In addition, the invention of the design method may be implemented on the basis of a result of performing the above-described screening method by another person.

That is, one embodiment of the present invention includes a screening step of screening active ingredients by the above-described screening method. The embodiment of the screening step is as described above.

Further, in one embodiment of the present invention, the invention of the design method is performed using a substance already determined to be an active ingredient by the above-described screening method.

According to the present invention, a regenerative medicine and/or an anticancer agent can be designed.

In a preferred mode of the present invention, the method includes a step of selecting an indication of the pharmaceutical composition. Examples of the indication include diseases, disorders, or illnesses of nervous system, heart, or pancreas, or symptoms thereof, or cancer. The specific contents of these indications are as described in detail above.

In a preferred mode of the present invention, the method includes a step of selecting a dosage form of the pharmaceutical composition. In a preferred mode of the present invention, a liquid agent is selected as a dosage form, and an aqueous medium to be mixed with an active ingredient is selected. In the present invention, an injection can be selected as a dosage form. In the present invention, an eye drop can be selected as a dosage form. In the present invention, a lyophilized preparation can be selected as a dosage form. In the present invention, an ointment is selected as a dosage form, and a base material to be mixed with an active ingredient is selected. An ophthalmic ointment can also be selected as the ointment. In the present invention, a preparation for nasal administration can be selected as a dosage form. In the present invention, a preparation for oral administration can be selected as a dosage form.

The description of the production method of the present invention above can be directly applied to a pharmaceutical additive to be combined with a substance selected as an active ingredient in screening, a dosage form of the pharmaceutical composition, and the like.

### [6] Method for clinical trials

The present invention also relates to a method including designing a pharmaceutical composition by the method described above, preparing a substance selected as an active ingredient by the above-described screening method, producing a pharmaceutical composition by the production method described above, and statistically processing a data set obtained from persons to whom the pharmaceutical composition was administered in a clinical trial.

The implementation of the method of the present invention is essential for the medicine containing an active ingredient screened in the above-described screening method to be commercialized upon approval from regulatory authorities.

In one embodiment of the present invention, a data set obtained from persons to whom the pharmaceutical composition was administered in a clinical trial (test group) and persons to whom a control drug was administered (control group) is compared and statistically processed.

The data set obtained from each of the test group and the control group is statistically processed, and the presence or absence of a statistically significant difference is determined between both groups.

In one embodiment of the present invention also relates to a method including statistically processing a data set obtained from healthy persons to whom the pharmaceutical composition was administered. This embodiment relates to a case of conducting a clinical trial called "Phase 1 clinical trial" in Japanese pharmaceutical practice.

Examples of the obtained data set include data sets such as dose of medicine, the number of administrations, administration time, administration period, blood concentration of an active ingredient after administration or its temporal transition, and concentration of an active ingredient or its metabolite in urine.

One embodiment of the present invention also relates to a method including statistically processing a data set obtained from a patient with indication of the pharmaceutical composition to which the pharmaceutical composition has been administered. This embodiment relates to "Phase 2 clinical trial" or "Phase 3 clinical trial" in Japanese pharmaceutical practice.

The obtained data set includes physiological data on dose of medicine, the number of administrations, administration time, administration period, blood concentration of an active ingredient after administration or its temporal transition, and concentration of an active ingredient or its metabolite in urine, or safety, side effects, and efficacy against the indication.

The statistical process is preferably performed by a computer. In addition, a specific method of the statistical processing can be appropriately selected based on a test plan of the clinical trial.

### [7] Pharmaceutical composition

The present invention also relates to a pharmaceutical composition containing an active ingredient screened by the above-described screening method, a pharmaceutical composition produced by the above-described production method, and a pharmaceutical composition designed by the above-described design method.

The pharmaceutical composition of the present invention will be described in detail below.

An animal as a subject of the pharmaceutical composition of the present invention is not particularly limited, but is preferably a vertebrate. Specifically, the present invention is applicable to any species of mammals, birds, reptiles, amphibians, and fish. More specifically, the subject of the present invention includes, for example, a human or a non-human animal excluding a human. Examples of the non-human animal include fish such as zebrafish, non-human mammals such as mouse, rat, rabbit, dog, sheep, horse, cat, goat, monkey, and guinea pig, and the like.

The pharmaceutical composition of the present invention has an action of promoting self-renewal of undifferentiated cells. Therefore, the pharmaceutical composition of the present invention can be used for self-renewing undifferentiated cells.

The pharmaceutical composition of the present invention is used for self-renewing adult stem cells. The pharmaceutical composition of the present invention is used for self-renewal of adult stem cells of one or more tissues selected from the nervous system, heart, and pancreas.

In addition, the pharmaceutical composition of the present invention has an action of inducing differentiation of undifferentiated cells into various cells. Therefore, the pharmaceutical composition of the present invention can be used for inducing differentiation of undifferentiated cells.

Further, the pharmaceutical composition of the present invention can be used for inducing differentiation of adult stem cells.

Furthermore, the pharmaceutical composition of the present invention can be used for inducing differentiation of adult stem cells of one or more tissues selected from the nervous system, heart, and pancreas.

The pharmaceutical composition of the present invention is used for any one or both of two applications of "self-renewal of undifferentiated cells" and "differentiation induction of undifferentiated cells".

In a preferred embodiment of the present invention, it is used for both applications of "self-renewal of undifferentiated cells" and "differentiation induction of undifferentiated cells".

In the conventional regenerative medicine process, the self-renewal step and the differentiation inducing step are performed outside the body (on a petri dish or in a test tube).

The pharmaceutical composition of the present invention can achieve these two steps in vivo. That is, the present invention can be used for self-renewal and differentiation induction of undifferentiated cells in vivo.

In one embodiment of the present invention, it is a regenerative medicine and/or an anticancer agent.

In one embodiment of the present invention, it is a regenerative medicine by self-renewal of undifferentiated cells and differentiation induction of undifferentiated cells.

In one embodiment of the present invention, the pharmaceutical composition is used for inducing differentiation of cancer cells into normal cells. In a more preferred embodiment, the pharmaceutical composition is for treating cancer by inducing differentiation of cancer cells into normal cells.

According to the pharmaceutical composition of the present invention, it is possible to promote self-renewal of neural undifferentiated cells, and subsequently promote differentiation of amplified neural undifferentiated cells. That is, according to the present invention, it is possible to realize regeneration or enhancement of the nervous system that has been considered to be impossible or very difficult to regenerate.

As described above, the pharmaceutical composition of the present invention can induce differentiation into various cells constituting the nervous system. That is, the pharmaceutical composition of the present invention can induce differentiation of neural undifferentiated cells into neural cells and glial cells.

The pharmaceutical composition of the present invention can be used for proliferation of mature neurons such as glutaminergic neurons, GABAergic neurons, dopaminergic neurons, serotonergic neurons, and cholinergic neurons by inducing differentiation of neural undifferentiated cells.

In addition, the pharmaceutical composition of the present invention can be used for proliferation of retinal ganglion cells or Purkinje cells.

The pharmaceutical composition of the present invention can be used for proliferation of glial cells such as astrocytes, oligodendrocytes, ependymal cells, Schwann cells (sheath cells), and satellite cells by inducing differentiation of neural undifferentiated cells.

The pharmaceutical composition of the present invention may be used for either the central nervous system or the peripheral nervous system. For any nervous system, the pharmaceutical composition of the present invention exhibits an action of self-renewal and/or differentiation induction of neural undifferentiated cells.

Once damaged, the central nervous system does not regenerate/is extremely difficult to regenerate. Therefore, the pharmaceutical composition of the present invention is preferably used for self-renewal and/or differentiation induction of neural undifferentiated cells of the central nervous system.

Examples of the central nervous system in which the present invention can be suitably used include any of the brain, spinal cord, optic nerve, and olfactory nerve. In particular, since disorders of the brain, spinal cord, and optic nerve lead to serious diseases significantly lowering QOL of patients, it is preferable to use the pharmaceutical composition of the present invention for self-renewal and/or differentiation induction of neural undifferentiated cells of the brain, spinal cord, and optic nerve.

As described above, the pharmaceutical composition of the present invention exhibits an effect of self-renewal and/or differentiation induction of neural undifferentiated cells. This enables the present invention to achieve regeneration of the nervous system, which is said to be impossible or extremely difficult to regenerate.

By this action, the present invention is applied to a disease, disorder, or illness of the nervous system, or symptoms thereof, thereby exhibiting a therapeutic or preventive effect thereof.

That is, the pharmaceutical composition of the present invention is preferably used for the treatment or prevention of a disease, disorder, or illness of the nervous system, or symptoms thereof.

The present invention is effective for the disease or the like of the nervous system of any of the central nervous system and the peripheral nervous system.

Examples of the disease or the like of the central nervous system include diseases or the like of the brain, spinal cord, optic nerve, and/or olfactory nerve.

Examples of the disease or the like of the peripheral nervous system include diseases or the like of the somatic nervous system and the autonomic nervous system.

Examples of the disease or the like of the central nervous system include Parkinson's disease; Alzheimer's disease; Creutzfeldt-Jakob disease; corticobasal degeneration; amyotrophic lateral sclerosis; multiple sclerosis; progressive motor weakness; immune neuropathies; central nervous system damage such as brain damage, spinal cord injury, optic nerve damage, olfactory nerve damage; Alzheimer's disease with parkinsonism; bradykinesia; akinesia; movement disorders that impair detailed motion control and finger dexterity; hypophonia; monotonous utterance; stiffness; dystonia; inflammation associated with Parkinson's disease; tremor of face, chin, tongue, posture; parkinsonian walking; shuffling; brachybasia; festination; mood, cognitive, sensory, sleep disorders; dementia; depression; drug-induced parkinsonism; vascular parkinsonism; multiple system atrophy; progressive supranuclear palsy; disorders with primary tau lesion; corticobasal ganglionic degeneration; parkinsonism with dementia; hyperactivity disorders; chorea; Huntington's disease; dystonia; Wilson's disease; Tourette's syndrome; essential tremor; myoclonus; delayed movement disorder; schizophrenia; bipolar disorder and autism spectrum disorder, and the like.

Examples of the disease or the like of the peripheral nervous system include movement disorders in which symptoms such as "weakness in the hands and feet", "often drops object", "cannot walk or run well", "cannot stand up well", or "easily trips due to toe drop" appear; sensory disorders in which symptoms such as "stinging", "tingling", or "loss of sensation" of the hands and feet appear; autonomic nerve disorders in which symptoms such as "the skin of the hand or foot is cold" and "the lower body does not sweat" appear; and the like

The pharmaceutical composition of the present invention has an action of inducing regeneration of the nervous system by promoting self-renewal and/or differentiation induction of neural undifferentiated cells. Therefore, the pharmaceutical composition of the present invention is effective for diseases and the like caused by damage or functional decline of the nervous system.

In addition, the pharmaceutical composition of the present invention is effective for diseases and the like caused by damage or functional decline of neurons or glial cells.

Further, the pharmaceutical composition of the present invention is effective for diseases and the like caused by damage or functional decline of cell bodies, myelin sheaths, axons, and neuromuscular junctions.

The pharmaceutical composition of the present invention is effective for diseases and the like caused by damage or functional decline of mature neurons such as glutaminergic neurons, GABAergic neurons, dopaminergic neurons, serotonergic neurons, and cholinergic neurons.

Furthermore, the pharmaceutical composition of the present invention is effective for diseases and the like caused by damage or functional decline of retinal ganglion cells or Purkinje cells.

The pharmaceutical composition of the present invention is effective for diseases and the like caused by damage or functional decline of glial cells such as astrocytes, oligodendrocytes, ependymal cells, Schwann cells (sheath cells), and satellite cells.

The term "functional decline" as used herein includes a state in which the function of the cell itself is deteriorated, and also includes a case in which functional decline of the entire tissue is observed due to a decrease in the number of cells.

Hereinafter, glaucoma and spinal cord injury among indications of the present invention will be described in more detail.

The pharmaceutical composition of the present invention is effective for treatment or prevention of glaucoma. The present invention is effective for both high intraocular pressure glaucoma and normal intraocular pressure glaucoma.

High intraocular pressure glaucoma is a disease in which the optic nerve of the retina gradually shrinks or swells and is damaged due to high intraocular pressure, retinal ganglion cells, which are a type of neurons existing in the retina, die by apoptosis, then the optic disc part is depressed, the visual field gradually narrows, and eventually the visual acuity is lost. Normal intraocular pressure glaucoma is clinically a disease in which all clinical findings of five categories of (1) intraocular pressure within a normal value range of 10 to 21 mmHg, (2) optic disc rim narrowing and depression, (3) retinal optic nerve fiber layer deficiency, (4) optic nerve deformation and posterior deviation at the site of the cribriform plate (cribriform orbital plate), and (5) reduction in optic nerve ganglion cells and glial cells are comprehensively observed, and specific optic nerve lesions are caused.

The pharmaceutical composition of the present invention exhibits a therapeutic effect by regenerating the damaged or reduced optic nerve (retinal ganglion cells, glial cells) seen in both high intraocular pressure glaucoma and normal intraocular pressure glaucoma.

The spinal cord does not regenerate spontaneously. Therefore, once the spinal cord is damaged, it has been impossible to completely cure the damage.

The pharmaceutical composition of the present invention exhibits a regeneration effect of the nervous system. Therefore, the pharmaceutical composition of the present invention is highly suitable for treatment of spinal cord injury. According to the pharmaceutical composition of the present invention, regeneration of nerves at an injury site of the spinal cord is brought about, and movement disorders and the like accompanying the spinal cord injury can be ameliorated or completely cured.

The spinal cord injury to which the pharmaceutical composition of the present invention can be applied includes injuries caused by accidents such as traffic accidents, sports accidents, and falls, and also includes illnesses such as spinal cord tumors and hernias.

It is preferable that the pharmaceutical composition of the present invention is administered to a site where neural undifferentiated cells are present in a living body or the vicinity thereof for use. By adopting such an administration form, self-renewal and differentiation induction of neural undifferentiated cells are efficiently caused by the effect of the pharmaceutical composition of the present invention.

The term "vicinity" refers to a range in which the active ingredient contained in the pharmaceutical composition can reach the location of the neural undifferentiated cells by diffusion or dispersion by a body fluid.

It is preferable that the pharmaceutical composition of the present invention is administered to a site where neural undifferentiated cells of the central nervous system are present in a living body or the vicinity thereof for use. By adopting such an administration form, self-renewal and differentiation induction of neural undifferentiated cells of the central nervous system are efficiently caused by the effect of the pharmaceutical composition of the present invention.

The pharmaceutical composition of the present invention is preferably administered to the brain, spinal cord, or eyeball for use. By adopting such an administration form, regeneration or neogenesis of the brain, spinal cord, or optic nerve can be realized.

Incidentally, stem cells responsible for neurogenesis in the adult brain are called adult neural stem cells, and are known to exist in at least two positions of the subventricular zone around the lateral ventricle and the dentate subgranular zone of the hippocampus.

Therefore, the pharmaceutical composition of the present invention may be an embodiment used by being administered to the lateral ventricle and/or the hippocampus where adult neural stem cells are present.

More specifically, the pharmaceutical composition of the present invention may be an embodiment used by being administered to the subventricular zone around the lateral ventricle and/or the dentate subgranular zone of the hippocampus.

The pharmaceutical composition of the present invention is preferably administered to a damaged site or hypofunctional site of the nervous system for use. As a result, regeneration of the nervous system at the damaged site or hypofunctional site can be efficiently performed, and a disease or the like can be efficiently treated.

In addition, it is preferable that the pharmaceutical composition of the present invention is administered to a site where damage or functional decline of neurons or glial cells are observed for use. As a result, regeneration of neurons or glial cells in the site can be efficiently performed, and a disease or the like can be efficiently treated.

Further, it is preferable that the pharmaceutical composition of the present invention is administered to a site where damage or functional decline of cell bodies, myelin sheaths, axons, or neuromuscular junctions is observed for use. As a result, regeneration of damaged or hypofunctional cell bodies, myelin sheaths, axons, or neuromuscular junctions can be efficiently performed, and a disease or the like can be efficiently treated.

It is preferable that the pharmaceutical composition of the present invention is administered to a site where damage or functional decline of mature neurons such as glutaminergic neurons, GABAergic neurons, dopaminergic neurons, serotonergic neurons, and cholinergic neurons is observed for use. As a result, regeneration of damaged or hypofunctional mature neurons can be efficiently performed, and a disease or the like can be efficiently treated.

Furthermore, it is preferable that the pharmaceutical composition of the present invention is administered to a site where damage or functional decline of retinal ganglion cells or Purkinje cells are observed for use. As a result, regeneration of damaged or hypofunctional retinal ganglion cells and Purkinje cells can be efficiently performed, and a disease or the like can be efficiently treated.

It is preferable that the pharmaceutical composition of the present invention is administered to a site where damage or functional decline of glial cells such as astrocytes, oligodendrocytes, ependymal cells, Schwann cells (sheath cells), and satellite cells is observed for use. As a result, regeneration of damaged or hypofunctional glial cells can be efficiently performed, and a disease or the like can be efficiently treated.

In addition, the pharmaceutical composition of the present invention can be used for treatment of a disease or the like caused by damage/functional decline of cardiomyocytes. The pharmaceutical composition of the present invention acts on cardiac stem cells/cardiac progenitor cells present in the heart to induce self-renewal and differentiation induction thereof, whereby damaged or hypofunctional cardiomyocytes can be regenerated.

The pharmaceutical composition of the present invention can be used for treatment of a disease or the like of the heart such as angina pectoris, myocardial infarction, valvular disease, cardiomyopathy, atrial septal defect, cardiac tumor, heart failure, and arrhythmia.

When the pharmaceutical composition of the present invention is used for treatment of the disease or the like of the heart, the pharmaceutical composition can be in an administration form such as local administration or oral administration to the heart, injection into a blood vessel, or intravenous injection.

Further, the pharmaceutical composition of the present invention can be used for treatment of a disease or the like caused by damage/functional decline of pancreatic cells. Examples of the pancreatic cells include β cells that secrete insulin, and the like. The pharmaceutical composition of the present invention acts on pancreatic stem cells/pancreatic progenitor cells present in the pancreatic duct to induce self-renewal and differentiation induction thereof, whereby damaged or hypofunctional pancreatic cells can be regenerated.

The pharmaceutical composition of the present invention can be used for treatment of a disease or the like of the pancreas such as type 1 diabetes, type 2 diabetes, acute pancreatitis, chronic pancreatitis, pancreatic cancer, and mucus-producing tumors.

When the pharmaceutical composition of the present invention is used for treatment of the disease or the like of the heart, the pharmaceutical composition can be in an administration form such as local administration or oral administration to the pancreas, injection into a blood vessel, or intravenous injection.

The pharmaceutical composition of the present invention can be used for treatment of hematological cancer. Examples of the hematological cancer include leukemias such as acute myelogenous leukemia, acute lymphocytic leukemia, chronic myelogenous leukemia and chronic lymphocytic leukemia; Hodgkin lymphoma such as classical Hodgkin lymphoma and nodular lymphocyte predominant Hodgkin lymphoma, malignant lymphomas such as B-cell lymphoblastic leukemia/lymphoma, T-cell lymphoblastic leukemia/lymphoma, chronic lymphocytic leukemia/small lymphocytic lymphoma, follicular lymphoma, MALT lymphoma, lymphoplasmacytic lymphoma, mantle cell lymphoma, diffuse large B-cell lymphoma, Burkitt lymphoma, peripheral T-cell lymphoma, adult T-cell leukemia/lymphoma, extranodal NK/T-cell lymphoma, lymphoma of the skin (mycosis fungoides, Sezary syndrome); and multiple myeloma such as symptomatic multiple myeloma, benign monoclonal hypergammaglobulinemia, asymptomatic myeloma, non-secretory myeloma, and isolated plasmacytoma of bone.

The pharmaceutical composition of the present invention can be used for inducing differentiation of cancer cells present in blood into vascular endothelial cells.

In one embodiment of the present invention, the pharmaceutical composition of the present invention can be used for the treatment of hematological cancer by inducing differentiation of cancer cells present in blood into vascular endothelial cells.

The dose can be set to preferably 0.0001 mg or more, more preferably 0.001 mg or more, more preferably 0.01 mg or more, and more preferably 0.1 mg or more per 1 kg of body weight at one time, as the weight of the active ingredient. In addition, it can be set to preferably 10,000 mg or less, more preferably 1000 mg or less, and more preferably 100 mg or less per 1 kg of body weight at one time.

Further, the dose can be set to preferably 0.01 mg or more, more preferably 0.1 mg or more, more preferably 1 mg or more, and more preferably 10 mg or more per patient as the weight of the active ingredient. In addition, it can be set to preferably 100,000 mg or less, more preferably 10,000 mg or less, more preferably 1000 mg or less, and more preferably 100 mg or less per patient.

Furthermore, the administration schedule can be adjusted, for example, between 3 times/day and 1 time/month, for example, between 1 time/day and 1 time/week, while observing the disease state and the trend of the blood test value.

Hereinafter, the usage of the pharmaceutical composition of the present invention in the treatment or prevention of the disease or the like of the cranial nervous system, the treatment or prevention of glaucoma, and the treatment of spinal cord injury will be described more specifically.

In the case of treating or preventing the disease or the like of the cranial nervous system, it is preferable to administer the pharmaceutical composition of the present invention by intracerebral injection, more specifically, intracerebroventricular injection, for use.

In this case, it is preferable to use an intracerebral administration system including an injection device including a syringe filled with an injection solution containing the pharmaceutical composition of the present invention and an injection pump, and a ventricular access device connected to the injection device by an extension line. The ventricular access device is inserted into the brain by perforating the skull, and the syringe is pierced to the target point for administration in the brain. Then, the injection pump is started, and the injection solution is injected at a rate of preferably 0.1 mL/hour to 10 mL/hour, and more preferably 1 mL/hour to 5 mL/hour.

The intracerebral injection can be carried out at a pace of preferably 1 time/day to 1 time/month, and more preferably 1 time/3 days to 1 time/3 weeks.

In addition, it is known that there is a route in which the administered substance directly migrates to the cerebrospinal fluid or the brain through the nasal mucosal layer without passing through the blood in the nasal cavity.

Therefore, in order to allow the active ingredient of the pharmaceutical of the present invention to reach the brain, preferred examples thereof include a form of intranasal administration.

In this case, the pharmaceutical of the present invention can be administered by intranasal spraying in the form of a powder nasal spray or a liquid nasal spray.

Moreover, the pharmaceutical of the present invention can be administered by being intranasally dropped as a form of nasal drops.

Further, the pharmaceutical of the present invention can be administered intranasally through a tube or a catheter as a dosage form of a gel preparation or an ointment.

Furthermore, the pharmaceutical of the present invention can be administered intranasally by submucosal injection into a nasal mucous membrane as a dosage form of an injection.

The administration pace can be preferably from 1 time/day to 1 time/week, and more preferably from 2 times/day to 1 time/3 days.

In the case of treating or preventing glaucoma, it is preferable to drop or apply the pharmaceutical composition of the present invention to an eyeball as a dosage form of an eye drop or an ophthalmic ointment.

The administration pace can be preferably from 1 time/day to 1 time/week, and more preferably from 2 times/day to 1 time/3 days.

In the case of treating spinal cord injury, it is preferable to inject the pharmaceutical composition of the present invention directly into the spinal cord injury site as a dosage form of an injection.

The injection can be intermittently carried out manually, but it is preferable to continuously administer the injection to the injury part using a mini pump, a catheter connected thereto, or the like.

The administration is preferably started as soon as possible after the injury, and the administration is preferably continued for at least one week, and still more preferably at least two weeks from the injury.

In the case of treating the disease or the like of the heart, the pharmaceutical composition of the present invention may be administered directly to the heart as a dosage form of an injection. In addition, the pharmaceutical composition of the present invention may be injected intravenously as a dosage form of an injection. Further, the pharmaceutical composition of the present invention may be orally administered as a dosage form of an oral administration agent.

In the case of treating the disease or the like of the pancreas, the pharmaceutical composition of the present invention may be administered directly to the pancreas as a dosage form of an injection. In addition, the pharmaceutical composition of the present invention may be injected intravenously as a dosage form of an injection. Further, the pharmaceutical composition of the present invention may be orally administered as a dosage form of an oral administration agent.

When the pharmaceutical composition of the present invention is used as a therapeutic agent for hematological cancer, the pharmaceutical composition may be injected intravenously as a dosage form of an injection. Further, the pharmaceutical composition of the present invention may be orally administered as a dosage form of an oral administration agent.

The present invention also relates to a pharmaceutical composition containing, as an active ingredient, a compound included in the general formula (I), the general formula (II), and the general formula (III) or a salt thereof or a hydrate of the compound or salt. Since the specific aspect is as described above, the description thereof is omitted here.

In an embodiment of the present invention, the active ingredient of the present invention is a compound selected from Compounds 1 to 7 described above or a salt thereof or a hydrate of the compound or salt. Since the specific aspect is as described above, the description thereof is omitted here.

### Examples

### [Test Example 1] Proliferation test of neural stem cells

### <Used animal>

Embryo of transgenic line zebrafish in which green fluorescent protein (GFP) is specifically expressed in mesencephalic neural stem cells

### <Test compound>

Compound 1: (3R)-Cyclopentyl-3-[4-(7H-pyrrolo[2,3-d]pyrimidin-4-yl)-1H-pyrazol-1-yl]propanenitrile phosphate
Compound 2: 4-[3-(6-Methylpyridin-2-yl)-1H-pyrazol-4-yl]quinoline
Compound 3: 2-Amino-5-(ethylamino)-5-oxopentanoic acid
Compound 4: N¹-Hydroxy-N⁸-phenyloctanediamino
Compound 5: 4-[(5-Bromopyridin-2-yl)amino]-4-oxobutyric acid
Compound 6: 5-Chloro-2-N-{4-[4-(dimethylamino)piperidin-1-yl]-2-methoxyphenyl}-4-N-[2-(dimethylphosphoryl)phenyl]pyrimidine-2,4-diamine
Compound 7: 2-[[5-Chloro-2-[2-methoxy-4-(4-methylpiperazin-1-yl)anilino]pyrimidin-4-yl]amino]-N-methylbenzenesulfonamide
(Compounds 1 to 7 are used separately in the experiments described below.)

### <Experimental method>

A solution (100 µM) in which the test compound was dissolved in E3 Ringer (zebrafish physiological saline) was prepared. This solution was microscopically injected into the ventricles of the embryos for 24 hours post-fertilization and cultured at 28.5°C until 72 hours post-fertilization.

In order to inhibit synthesis of melanin pigment that interferes with observation with a fluorescence microscope, 0.2 mM phenylthiourea (PTU) had been added to the culture solution.

In addition, at the time of microinjection, the embryos were anesthetized with 0.02% MS-222 (tricaine)/E3 Ringer, and then held with the dorsal side of the head up on a coffin (plastic plate with V-shaped grooves for holding), and a glass capillary was inserted into the vicinity of the pineal gland. Then, a nitrogen gas pressure (15 picosiemens) was applied for 15 to 45 milliseconds, and the mixture was injected into the ventricle.

At this time, 0.025% (final concentration) phenol red was mixed as a tracer of the injection solution.

The amount to be injected was adjusted by applying gas pressure in a pulsed manner until the anterior ventricle, the diencephalon and the middle ventricle became uniform light red.

As a control experiment (control), only E3 Ringer was administered into the ventricle in the same volume.

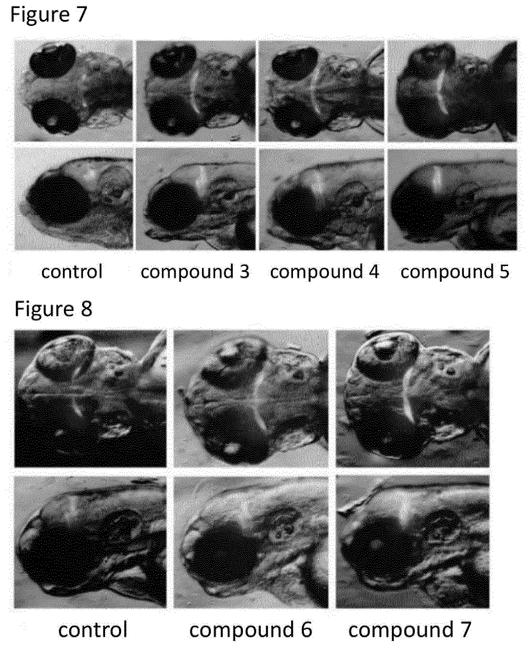

The solid cultured for up to 72 hours after fertilization was held in a 3% methylcellulose/E3 Ringer/0.02% tricaine solution on a slide glass, and then GFP was emitted with 488 nm excitation light and imaged by a CCD camera (Figs. 6 to 8).

The number of samples is 80 in the experiment using each test compound (N = 80).

Moreover, the brains of the treated zebrafish individuals were dissected and washed with sterile distilled water, and then crushed by pipetting or 1 ml syringe suction discharge with 18 G to 24 G needles in 500 µl of Sepasol-RNA I Super G (Nacalai Tesque). A deproteinization operation was conducted once by equivalent phenol/chloroform treatment and once by chloroform treatment, and the supernatant was subjected to RNA purification using an RNA purification column (RNeasy Plus Mini Kit, Qiagen). The elute was reverse-transcribed using a mixed primer of random hexamer and oligo-dT (ReverTra Ace qPCR Kit, TOYOBO) at 37°C for 15 minutes and 98°C for 5 minutes, and quantitative PCR of expression levels of sox1α, sox2, sox3, and her6, which are markers of neural stem cells, was performed. Quantitative PCR was performed with SYBER Green Realtime PCR Master Mix (TOYOBO). For the PCR reaction, ABI PRISM 7700 or LightCycler from Roche Diagnostics K.K. was used. Quantification was performed in 3 wells per identical sample (n = 3). The obtained data was comparatively quantified by a ΔΔCt method using a normalizer (β-actin, GAPDH, 18S, or the like). In order to confirm that the quantitative reaction was reliable, it was confirmed that the amplification product was in a band by agarose electrophoresis.

### <Result>

As shown in Figs. 6 to 8, in the zebrafish embryos injected with the test compound, the range in which fluorescence of GFP indicating mesencephalic neural stem cells was observed was significantly larger than that in the control.

This result indicates that Compounds 1 to 7 as test compounds have an action of remarkably promoting self-renewal of neural stem cells.

In addition, no tumor development was confirmed in all samples. This result indicates that self-renewal of neurons can be promoted without causing canceration, which is one of risks of regenerative medicine.

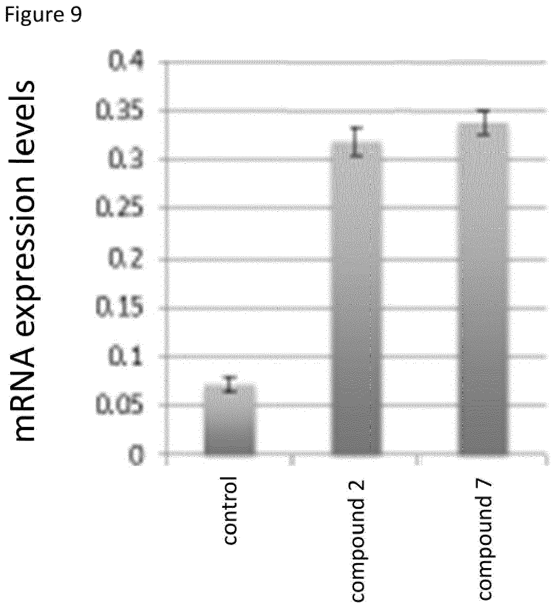

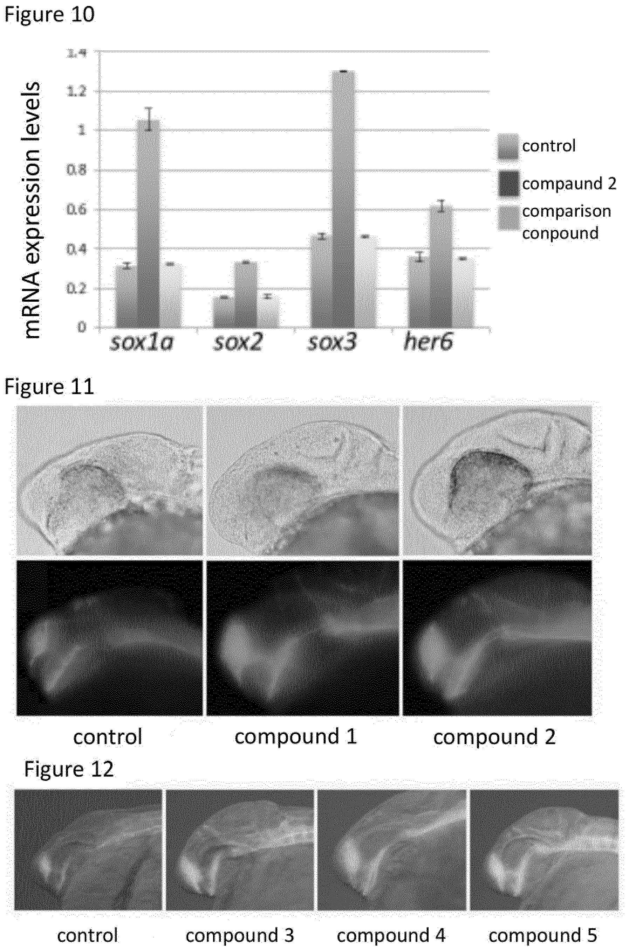

Further, as a result of quantitative PCR, in the brain of the individual zebrafish in which proliferation of neural undifferentiated cells was observed, a significant increase in expression levels of sox1α, sox2, sox3, and her6, which are markers of neural stem cells, was observed. Figs. 9 and 10 show the results of quantitative PCR in the individuals to which Compounds 2 and 7 were administered.

### [Test Example 2] Proliferation test of nervous system differentiated cells

### <Experimental method>

The individuals to whom the test compound was intracerebroventricularly administered in Test Example 1 were cultured into embryos for 40 hours and fixed with a 4% paraformaldehyde/phosphate buffer (PBS) at normal temperature for 90 minutes. Thereafter, the embryos were immunofluorescently labeled with an anti-acetylated tubulin antibody (Monoclonal, × 1/3000 dilution), which is a marker of differentiated neural cells, and an Alexa 488 anti-mouse IgG secondary antibody, and observed with excitation light at 488 nm (Figs. 11 to 13).

The number of samples is 10 in the experiment using each test compound (N = 10).

### <Result>

Parts where green fluorescence is observed in Figs. 11 to 13 are differentiated neurons, more specifically, cell body clusters of nerves (telencephalon and diencephalon) and axon bundles connecting them. As shown in Figs. 11 to 13, in the zebrafish embryos injected with the test compound, the range and intensity of the tendency to show cell body clusters of differentiated nerves were significantly larger than those in the control.

In addition, no tumor development was observed in all samples.

When the results of Test Examples 1 and 2 are considered together, it can be said that Compounds 1 to 7 as test compounds have an action of promoting self-renewal of neural undifferentiated cells, thereby inducing differentiation induction with respect to the proliferated neural undifferentiated cells, and proliferating nervous system differentiated cells.

In common knowledge in regenerative medicine, it has been considered that self-renewal and differentiation induction of undifferentiated cells need to be induced by different factors.

However, the results of Test Examples 1 and 2 prove the surprising fact that two processes of self-renewal and differentiation induction of undifferentiated cells can be achieved with a single agent (and do not cause canceration).

### [Test Example 3] Screening using zebrafish embryos

Compound libraries having completely different structures were prepared, and screening was performed according to Test Example 1 and Test Example 2. As a result, an increase in neural undifferentiated cells and neural differentiated cells was observed in 37 compounds. Table 1 summarizes primary actions of compounds in which an increase in neural undifferentiated cells and nervous system differentiated cells was confirmed.

### [Table 1]

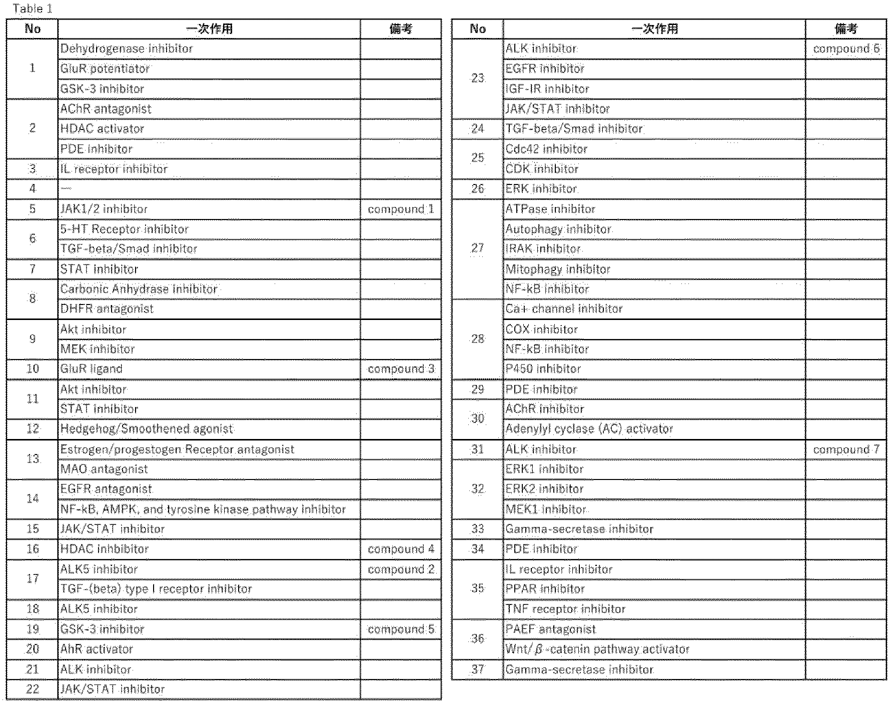

As shown in Table 1, the compounds determined to be active ingredients by screening are concentrated on those acting on signaling pathways (Notch signaling pathway, PI3K/AKT/mTOR signaling pathway, JAK/STAT signaling pathway, MAPK signaling pathway, TGFβ/SMAD signaling pathway, Wnt signaling pathway) involved in self-renewal and maintenance of totipotency or differentiation/development induction of stem cells. These signaling pathways are related to the control of self-renewal and differentiation induction of stem cells.

It is interesting that, in the reports in the in vitro tests so far, a compound said to have an action of maintaining or promoting self-renewal/totipotency of stem cells and a compound said to have an action of promoting differentiation/development bring about an increase in neural undifferentiated cells and neural differentiated cells by a single agent, respectively.

This result indicates that tests in vitro have revealed only one aspect in property control of stem cells. That is, the in vitro test system shows that the action of either "maintenance of self-renewal/pluripotency of stem cells" or "differentiation induction" can be observed only on one side.

On the other hand, the in vivo screening system using the living body of the present invention can present extremely effective results reflecting complicated information transmission between cells and tissues.

### [Test Example 4] Effect of inducing regeneration of compound on individual with spinal cord injury of adult zebrafish

### <Used animal> One-month-old zebrafish

### <Test compound>

Compounds 1 to 7 (Compounds 1 to 7 are used separately in the experiments described below.)

### <Experimental method>

One-month-old zebrafish individuals were anesthetized with 0.02% tricaine, and the spinal cord was then damaged by insertion of a glass capillary. After the injury, the individuals were raised 1 day, and 100 µM of a test compound dissolved in 0.1% to 0.3% agarose/E3 Ringer was injected into the injury site. At this time, 0.025% (final concentration) phenol red was mixed as a tracer. The individuals were observed with a stereomicroscope under visible light, and it was confirmed that phenol red accumulated around the spinal cord injury site, and the individuals were raised for 3 days.

As a control experiment (control), an experiment of injecting 0.1% to 0.3% agarose/E3 Ringer containing no test compound was also performed.

One group was weakly anesthetized with 0.02% tricaine as it was in a living body, and after being held in low-temperature soluble agarose 1%/E3 Ringer/0.02% tricaine, MRI imaging was performed in a reflux state in a 2.0 mL chamber. After the imaging, the individuals were subjected to anesthesia removal treatment (forced reflux of E3 Ringer through mouth and restart of spontaneous breathing were confirmed), and breeding was continued at 28.5°C. After 1 month, the individuals were fixed with a 4% paraformaldehyde/phosphate buffer (PBS) at normal temperature for 2 days, and then MRI imaging was performed again.

Another group was fixed with 4% paraformaldehyde/phosphate buffer (PBS) at normal temperature for 2 days immediately after spinal cord injury, and then MRI imaging was performed.

The individual subjected to the spinal cord injury treatment was fed by forcibly orally administering an artificial feed three times a day during the breeding period.

### <Result>

After spinal cord injury, an image was immediately taken by MRI to confirm the injury introduced into the spinal cord. As a result, it could be confirmed that spinal cord injury was caused to the zebrafish individual by the above experimental method.

The MRI images taken 3 days and 1 month after the injection of the compound into the spinal cord injury site were compared. As a result, it could be confirmed that the injury of the spinal cord was not cured and remained as it was in the control zebrafish individuals. On the other hand, in the zebrafish individuals injected with the test compound, spinal cord injury was not observed. That is, regeneration of the spinal cord at the injury site was observed.

In addition, in control zebrafish, swimming ability remained lost after spinal cord injury. On the other hand, in the zebrafish injected with the test compound, obvious recovery of swimming ability lost due to spinal cord injury was observed.

This result indicates that Compounds 1 to 7 as test compounds have a therapeutic effect on spinal cord injury.

When the results of Test Examples 1 to 3 are considered together, it can be said that Compounds 1 to 7 have an action of regenerating the damaged nervous system by promoting self-renewal of neural undifferentiated cells and inducing differentiation induction of the proliferated neural undifferentiated cells.

### [Test Example 5] Regeneration induction effect of mouse retinal neurons by eye drop

### <Used animal>

High intraocular glaucoma model mouse (transgenic lines specifically expressing green fluorescent protein (GFP) in retinal ganglion cells)

### <Test compound>

Compounds 1 to 7 (Compounds 1 to 7 are used separately in the experiments described below.)

### <Experimental method>

To an adult of a high intraocular pressure glaucoma model mouse, a solution prepared by dissolving 100 µM of the test compound in 1% methyl cellulose/physiological saline was administered by instillation to both eyes at 500 µl per a time three times a day. After 10 days to 1 month from instillation, the mice were sacrificed, a retinal tissue section was prepared, retinal ganglion cells were observed with a fluorescence microscope under 488 nm excitation light, and imaged by a CCD camera.

As a control experiment (control), an experiment of instilling 1% methyl cellulose/physiological saline containing no test compound was also performed.

### <Result>

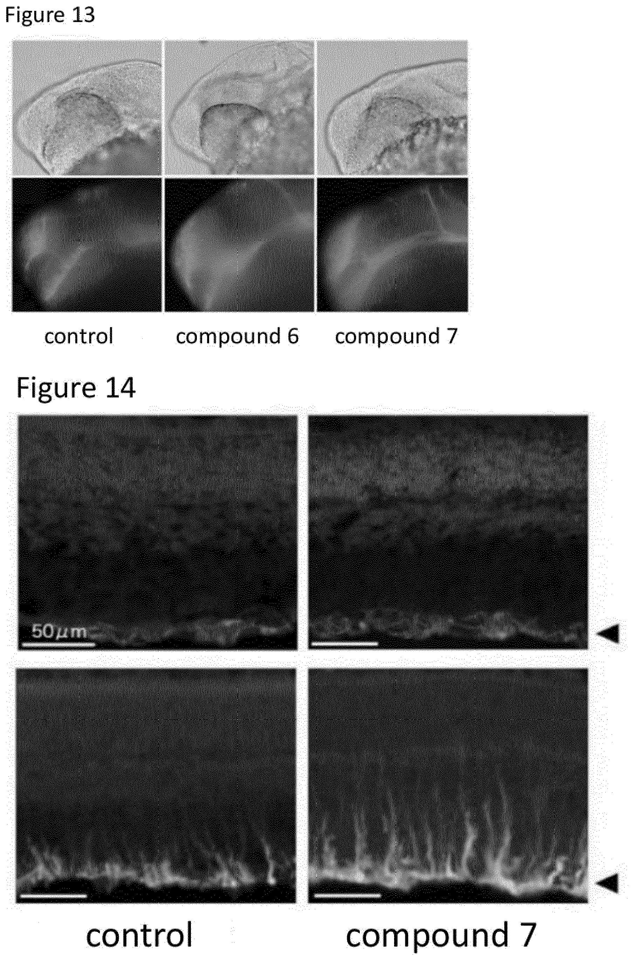

In the high intraocular pressure glaucoma model mouse to which Compounds 1 to 7 as test compounds was instilled, amplification of retinal ganglion cells was remarkably observed as compared with the control (the result of Compound 7 is shown in Fig. 14). In addition, in the high intraocular pressure glaucoma model mouse to which the test compound was instilled, it could be confirmed that visual acuity was clearly recovered from the behavior.

This result indicates that Compounds 1 to 7 as test compounds have a therapeutic effect or a preventive effect on glaucoma.

When the results of Test Examples 1 to 5 are considered together, it can be said that Compounds 1 to 7 have an action of regenerating the damaged nervous system by promoting self-renewal of neural undifferentiated cells and inducing differentiation induction of the proliferated neural undifferentiated cells.

### [Test Example 6] Increase in Purkinje cells of adult mouse by intracerebroventricular administration

### <Used animal>

E11.5 Mouse fetus

### <Test compound>

Compounds 1 to 7 (Compounds 1 to 7 are used separately in the experiments described below.)

### <Experimental method>

A solution prepared by dissolving 100 µM of the test compound in physiological saline was injected into E11.5 mouse fetal ventricle. Brains of adult mouse individuals were fixed, and slice sections of cerebellar Purkinje cell layers were immunostained with an anti-d-Calbindin antibody, which is a marker of cell bodies and neurites of Purkinje cells. After staining, the number of Purkinje cells was counted, and the average value thereof was calculated.

As a control experiment (control), a similar experiment was performed using physiological saline containing no test compound.

The number of samples is 10 for each of Compounds 1 to 7 and control (N = 10).

### <Result>

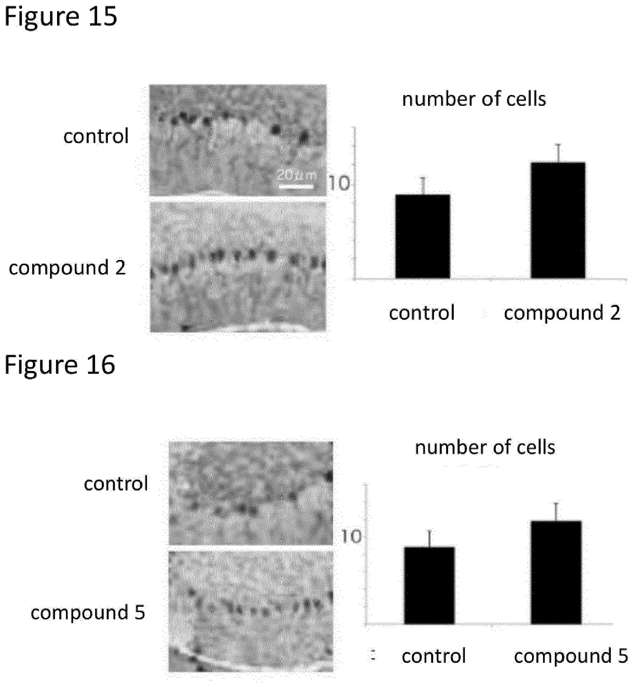

The number of Purkinje cells in the cerebellar Purkinje cell layers of adult mice injected with the test compound at fetal time was significantly higher than that of the control (Fig. 15, Fig. 16, and Fig. 17 show the results of Compounds 2, 5, and 7, respectively).

This result indicates that regeneration and enhancement of the cranial nervous system are possible according to Compounds 1 to 7 as test compounds. That is, it can be said that Compounds 1 to 7 exhibit a therapeutic effect on the disease or the like of the cranial nervous system.

### [Test Example 7] Increase in neural stem cells of adult zebrafish by intracerebroventricular administration

### <Used animal>

Reporter gene transgenic line of zebrafish specifically expressing green fluorescent protein in mesencephalic neural stem cells

### <Test compound>

Compounds 1 to 7 (Compounds 1 to 7 are used separately in the experiments described below.)

A solution (0.1% DMSO) in which the test compound (100 µM) was dissolved in E3 Ringer (zebrafish physiological saline) was prepared. Mesencephalic neural stem cells were microscopically injected into the ventricles of one-month-old adult fish individuals labeled with green fluorescent protein, and cultured at 28.5°C for 1 week.

As a control experiment (control), only E3 Ringer (0.1% DMSO plus 0.025% phenol red) was administered into the ventricle in the same volume.

Separately from the above method, the test compound was encapsulated in liposome, and lipofection was performed on the ventricular zone (Marine Biotechnology 8(3): 295-303 2006). Specifically, 10 µl of the test compound (0.1% DMSO) was mixed with 10 µl OPTI-MEM1 medium (Invitrogen) and 5 µl 0.8% phenol red (tube A). At the same time, a solution prepared by mixing 3 µl of Lipofectamine 2000 (Invitrogen) and 7 µl of OPTI-MEM1 medium (tube B) is separately prepared, and the tube B is allowed to stand at normal temperature for 5 minutes. Thereafter, the tube A and the tube B were mixed in the same amount and subjected to a package reaction at normal temperature for 20 minutes, and then the mixture was injected into the individual by the same method as described above.

In this lipofection method, the effect of the injected test compound is further localized, and cell permeability into cells also increases.

As a control experiment (control), a solution reacted only with E3 instead of the test compound was used.

At the time of microinjection in the above two methods, the individual was anesthetized with 0.02% MS-222 (tricaine)/E3 Ringer, and then held with the dorsal side of the head up on a coffin (a plastic plate in which a V-shaped groove for holding was engraved), and a glass capillary was inserted into the vicinity of the pineal gland. Then, a nitrogen gas pressure (15 picosiemens) was applied for 15 to 45 milliseconds, and the mixture was injected into the ventricle. At this time, 0.025% (final concentration) phenol red was mixed as a tracer of the injection solution. The amount to be injected was adjusted by applying gas pressure in a pulsed manner until the anterior ventricle, the diencephalon and the middle ventricle became uniform light red.

After culturing, the fluorescence of the green fluorescent protein was observed with a fluorescence microscope to evaluate the amount of neural stem cells in the zebrafish to which the test compound was locally administered. Specifically, the solid cultured for 7 days after injection was held in a 3% methylcellulose/E3 Ringer/0.02% tricaine solution on a slide glass, and then GFP was emitted with 488 nm excitation light and imaged by a CCD camera. In addition, the average fluorescence brightness of the green fluorescent protein in a rectangle with a width of 100 um and a height of 50 um with the rostral end up around the fluorescence expression part was measured with a brightness analysis software.

In many cases, since spontaneous respiration of the individual disappears and the individual is in a state of apparent death during these operations, E3 was caused to flow repeatedly from the mouth by manual operation with a micropipette with a 200 µL tip, and resuscitation work was continued until the spontaneous respiration returned.

In order to inhibit synthesis of melanin pigment that interferes with observation with a fluorescence microscope, 0.2 mM phenylthiourea (PTU) had been added to the culture solution.

### <Result>

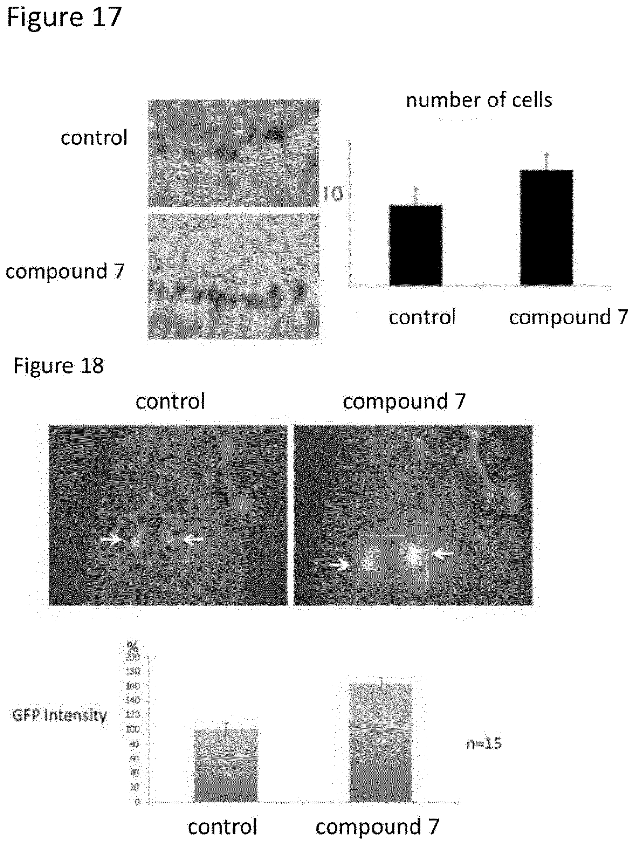

As a result of fluorescence observation, regardless of which injection method was adopted, fluorescence indicating the presence of mesencephalic neural stem cells in the brain of adult zebrafish into which the test compound was injected was strongly detected as compared with the control. Fig. 18 shows the results when Compound 5 was diluted and administered to E3 Ringer.

This result suggests that regeneration and enhancement of the cranial nervous system are possible in an adult according to Compounds 1 to 7 as test compounds. That is, it can be said that the active ingredient screened by the screening method of the present invention exhibits a therapeutic effect on the disease or the like of the cranial nervous system.

### [Test Example 8] Enhancement of myocardium

### <Used animal>

Zebrafish embryo

### <Test compound>

Compounds 1 to 7 (Compounds 1 to 7 are used separately in the experiments described below.)

### <Experimental method>

Zebrafish embryos at 10 hours after fertilization were held with the dorsal side of the head up on a coffin (plastic plate with V-shaped grooves for holding), and a glass capillary was inserted into a midline between head and ear vesicles (heart primordium). Nitrogen gas pressure (15 picosiemens) was applied for 15 to 45 milliseconds and injected into the gap just below the neural tube. The test compound concentration was 100 µM/E3, and 0.025% (final concentration) phenol red was mixed as a tracer of the injection solution. The injection was performed in pulses with a foot pedal and continued until phenol red was distributed in an elliptical shape with a major axis of 200 um from the insertion point. After injection, embryos were cultured at 28.5°C for 14 hours in E3. After chorions were removed with tweezers, the embryos were fixed with 4% paraformaldehyde/PBS at normal temperature for 1 hour, and in situ hybridization was performed overnight at 65°C in the presence of a DIG probe of nkx2.5 as a cardiac muscle marker according to a conventional method. After washing, cardiomyocytes were stained by an alkali phosphatase color reaction using an anti-DIG antibody. The stained embryos were washed with PBS, embedded in 75% glycerin/PBS and localized, and then microscopically photographed with incident light. The number of tested individuals was unified with n = 80 for each compound.

In addition, the zebrafish embryos injected with the test compound by the above-described method were cultured at 28.5°C until 48 hours after fertilization, embedded in 3% methyl cellulose/E3, and a moving image was photographed. The number of blood cells pumped in one heartbeat in time-lapse was counted n = 15 and compared to Ringer injected individuals.

### <Result>

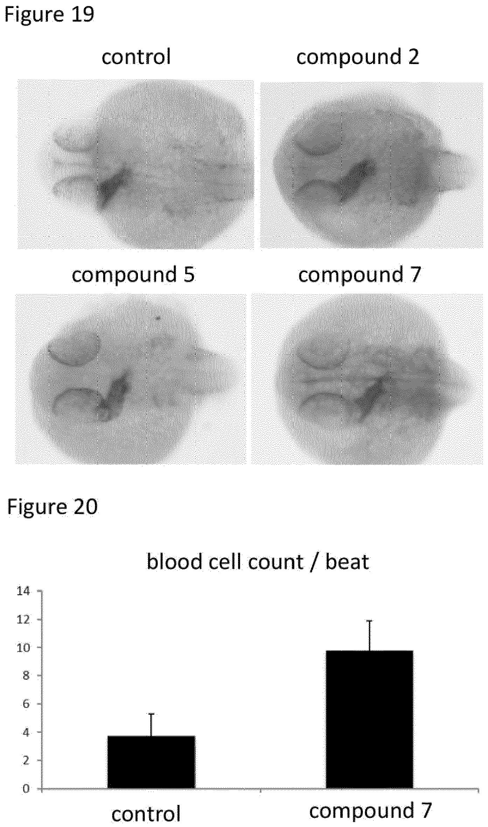

As a result of the in situ hybridization, in the zebrafish individual into which the test compound was injected, an increase in the amount of cardiomyocytes was observed as compared with the control. Fig. 19 shows micrographs of individuals injected with Compounds 2, 5, and 7.

In addition, in the zebrafish individual injected with the test compound, a remarkable increase in the number of blood cells pumped in one heartbeat was observed as compared with the control solid. Fig. 20 shows the results of counting stroke volume of blood cells in an individual injected with Compound 7.

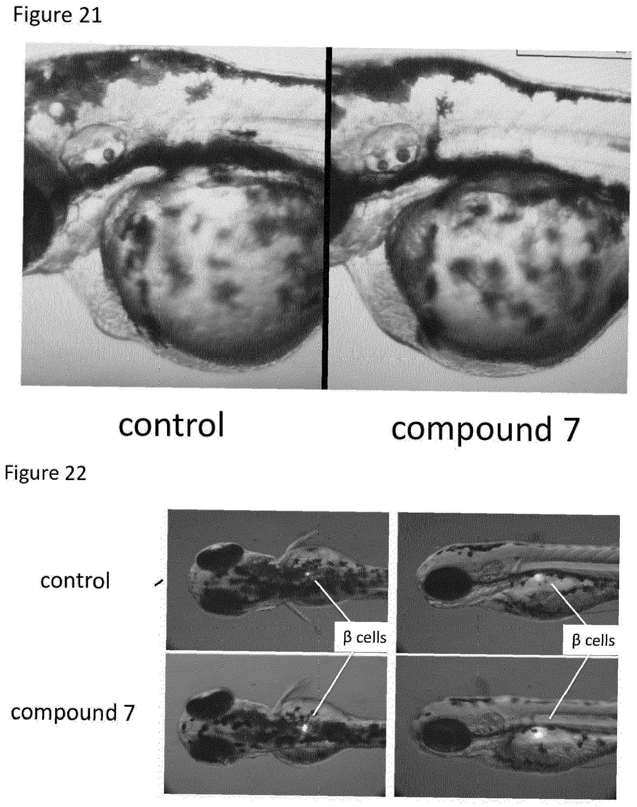

In addition, it could be confirmed from the moving image capturing the heartbeat of the individual injected with the test compound that the stroke volume of blood cells was remarkably improved. Fig. 21 shows a captured image of a moving image capturing the heartbeat of the individual injected with Compound 7.

The result of Test Example 8 indicates that the active ingredient screened by the screening method of the present invention has an effect of enhancing myocardium. That is, it has been demonstrated that the active ingredient has an effect of being able to treat a disease or the like of the heart caused by damage and functional deterioration of cardiomyocytes.

### [Test Example 9]

### <Used animal>

Transgenic zebrafish expressing pancreatic β-cell GFP

### <Test compound>

Compounds 1 to 7

### <Experimental Method 1> Fluorescence observation

Transgenic zebrafish embryos expressing pancreatic β-cell GFP at 10 hours after fertilization were held with the dorsal side of the head up on a coffin, and a glass capillary was inserted from the dorsal side into a gap just below the midline neural tube at about 2 times the distance between the rostral end and the ear vesicles to the caudal side (pancreatic primordium). A nitrogen gas pressure (15 picosiemens) was applied for 15 to 45 milliseconds, and the test compound was injected. The concentration of the test compound was 100 nM/E3, and 0.025% (final concentration) phenol red was mixed as a tracer of the infusion solution. The injection was performed in pulses with a foot pedal and continued until phenol red was distributed in an elliptical shape with a major axis of 200 um from the insertion point. The treated embryos were cultured at 28.5°C for up to 48 hours, anesthetized with 0.02% MS-222 (tricaine)/E3 Ringer, and pancreatic β-cell-specific GFP was observed with 488 nm excitation light (Fig. 22).

### <Experimental Method 2> In situ hybridization

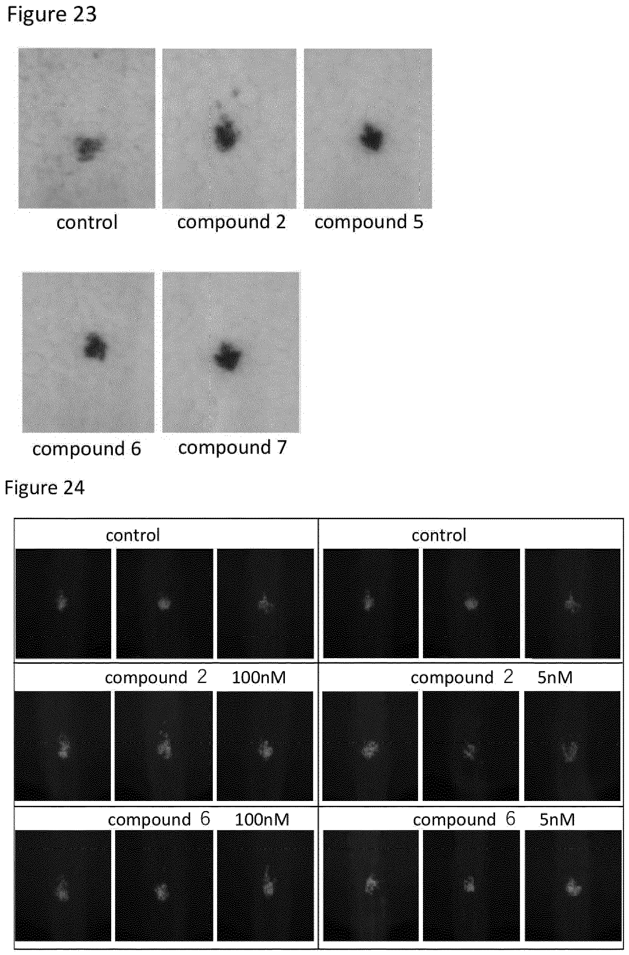

The test compound (100 nM) was injected on a coffin in transgenic zebrafish embryos expressing pancreatic β-cell GFP at 10 hours after fertilization. After injection, embryos were cultured in E3 at 28.5°C for 14 hours. After chorions were removed with tweezers, the embryos were fixed with 4% paraformaldehyde/PBS at room temperature for 1 hour, and in situ hybridization was performed overnight at 65°C in the presence of a DIG probe of insulin (ins) according to a conventional method. After washing, β cells were stained by an alkali phosphatase color reaction using an anti-DIG antibody. After staining, the embryos were washed with PBS, embedded in 75% glycerin/PBS and localized, and then microscopically photographed with incident light. The number of test individuals was unified with n = 100 for each test compound (Fig. 23).

### <Experimental Method 3> Immunostaining with anti-insulin antibody

Transgenic zebrafish embryos expressing pancreatic β-cell GFP at 10 hours after fertilization were injected with the test compound (100 nM or 5 nM) on the coffin. After injection, embryos were cultured in E3 at 28.5°C for 20 hours in the presence of 0.2 mM phenylthiourea (PTU). After chorions were removed with tweezers, the embryos were fixed with 4% paraformaldehyde/PBS at normal temperature for 1 hour, immunohistochemically reacted with an anti-insulin antibody according to a conventional method, and then reacted with an Alexa fluor 488 anti-mouse IgG secondary antibody. The reacted embryos were washed with PBS, embedded in 75% glycerin/PBS and localized, and then microscopically photographed with 488 nm excitation light (Fig. 24). The number of tested individuals was unified with n = 10 for each compound. The insulin amount was quantified by measuring the average fluorescence intensity in a circle with a radius of 100 um centered on the fluorescence point (Fig. 25).

### <Result>

It was confirmed that the number of β cells in the individual injected with Test Compounds 1 to 7 was increased as compared with the control individual. Fig. 22 shows the results of Compound 7.

In addition, it could be confirmed that the production amount of insulin by β cells in the individual injected with Test Compounds 1 to 7 was also significantly increased as compared with the control individual. Fig. 23 shows stained photographs of insulin by in situ hybridization when Compounds 2, 5, 6, and 7 were injected. Also, Figs. 24 and 25 show immunostained photographs with an anti-insulin antibody and a bar graph showing the average fluorescence intensity when Compounds 2 and 6 were injected.

This result indicates that Compounds 1 to 7 as test compounds have an effect of increasing differentiated pancreatic cells such as pancreatic stem cells and β cells. That is, it can be said that the active ingredient screened by the screening method of the present invention exhibits a therapeutic effect on the disease or the like of the pancreas.

### [Test Example 10] Verification of anticancer effect

### <Used animal>

T-cell type acute lymphocytic leukemia model specifically expressing GFP in vascular endothelial cells

### <Test compound>

### Compound 7

### <Experimental method>

A liquid agent prepared by adding a compound to E3 Ringer to adjust to 100 µM was microscopically injected into the body ventral main vessel of an embryo of a T celltype acute lymphatic leukemia model individual 24 hours after fertilization. E3 Ringer was similarly microscopically injected as a control. At the time of microinjection, embryos were held on the body side up in a 3% methylcellulose/E3 Ringer/0.02% tricaine solution on a glass slide, and then a glass capillary was inserted into an abdominal main vessel running in the head-to-tail direction at the boundary between the body wall and egg yolk. Then, a nitrogen gas pressure (15 picosiemens) was applied for 30 to 45 milliseconds, and the mixture was injected into the blood vessel. At this time, 0.025% (final concentration) phenol red was mixed as a tracer of the injection solution. The amount to be injected was adjusted by applying gas pressure in a pulsed manner until a large blood vessel of the whole body became uniform and thin red due to blood flow. Since the injected drug was stored in the kidney within one day and phenol red was localized, Compound 7 or control E3 Ringer was additionally injected under the same conditions also at 36 hours and 48 hours after fertilization. At 56 hours after fertilization, the individual was anesthetized again, and the vascular endothelial cells were fluorescently observed with 488 nm excitation light.

In addition, survival rates of drug-injected individuals and control individuals were counted. Verification test of the survival rate was performed three times. The number of test individuals for each of first time/second time/third time was 173/231/153 individuals for the control and 221/187/204 individuals for the drug treatment.

### <Result>

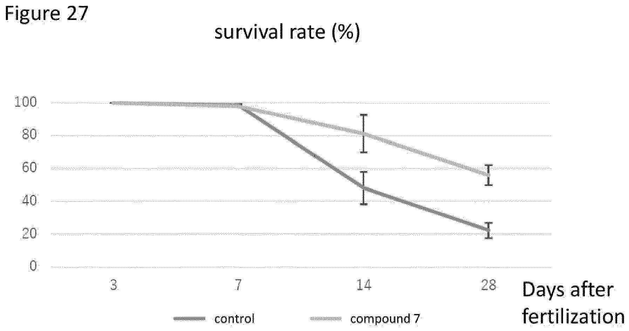

In the individuals injected with Compound 7, thickening and tumorigenesis of the endothelium were observed throughout the blood vessels of the body as compared with the control individuals (Fig. 26). Also, the survival rate on Day 28 was 22.4% ± 4.56 in the control, and 55.9% ± 6.07 in the individuals injected with Compound 7. That is, a remarkable increase in survival rate was observed in the individuals injected with Compound 7 as compared with the control individuals (Fig. 27).

These results indicate that Compound 7 exhibits an anticancer effect by forcibly differentiating cancer cells in blood into normal vascular endothelial cells.

### [Discussion]

As shown in Test Examples 1 to 8, it was found for the first time that there are a plurality of compounds that achieve self-renewal and differentiation induction of undifferentiated cells in vivo by a single agent. In other words, it has become clear that when a candidate substance is administered to an animal, undifferentiated cells and/or differentiated cells are observed, and the proliferation effect of these cells is used as an index, the active ingredient of regenerative medicine effective for diseases and the like of various tissues can be screened.

Meanwhile, as shown in Test Examples 1 and 2, the active ingredients screened by the screening method of the present invention achieve two processes of self-renewal and differentiation induction of undifferentiated cells by a single agent, and surprisingly, do not cause canceration. In addition, as shown in Figs. 6 to 8, 11 to 19, and 21, it can be understood that morphological abnormality or the like is not observed in the individual into which the active ingredient has been injected, and normal development/differentiation has occurred. This suggests that undifferentiated cells whose self-renewal has been promoted by administration of the active ingredient do not continue self-renewal indefinitely, but switch to the direction of promoting differentiation at an appropriate timing by being affected by tissues and cells present in the surrounding environment.

Based on this, the results of Test Example 9 will be considered. Acute leukemia is a hematopoietic neoplastic disease that occurs when differentiation arrest and acquisition of proliferation capability occur at a certain stage in the differentiation process of normal hematopoietic cells. Acute leukemia constitutes a leukemic cell population containing leukemic stem cells capable of self-renewal ability and differentiation into leukemic blasts, as if it mimics the normal hematopoietic stem cell system. Leukemic stem cells that maintain immaturity exhibit therapeutic resistance and are a source of relapse. It is known that it is important for the onset, maintenance, and proliferation of leukemia that leukemia stem cells and vascular endothelial cells adhere to each other and are affected by microenvironment (niche) formed thereby (Non Patent Literature 28).

As described above, the active ingredient screened in the present invention has an action of normally promoting self-renewal and differentiation induction of undifferentiated cells in a well-balanced manner without causing canceration under a surrounding environment in a living body. It can be said that the output when the action of the active ingredient acts on leukemia stem cells is the result of Test Example 9. That is, it can be said that the forced differentiation of cancer cells into vascular endothelial cells in Test Example 9 is a result of Compound 7 acting on leukemia stem cells and promoting differentiation into normal vascular endothelial cells under the effect of the microenvironment formed by adhesion with vascular endothelial cells. In other words, it can be said that the active ingredient screened in the present invention has an effect of acting on information transmission (phosphorylation, methylation, acetylation, and the like) in a microenvironment, which is performed by a leukemia stem cell having an abnormal proliferation capability and a normal vascular endothelial cell through physical contact, and urging the leukemia stem cell to switch the cell fate for differentiation to the vascular endothelial cell side.

From the above, it can be said that the compound that exhibits the proliferation action of undifferentiated cells and differentiated cells when administered to a living body is a compound that exhibits the action of promoting forced differentiation of cancer cells into normal cells under the effect of the microenvironment. In other words, it has become clear from the present test example that when a candidate substance is administered to an animal, undifferentiated cells and/or differentiated cells are observed, and the proliferation effect of these cells is used as an index, the active ingredient of an anticancer agent having an action of forcibly inducing differentiation of cancer cells into normal cells can be screened.

### Industrial Applicability

The present invention can be applied to screening active ingredients of a regenerative medicine or an anticancer agent.

## Claims

1. A method for screening active ingredients of a medicine, comprising the following
step A,
step B and/or step C, and
step D:
[step A] a step of administering a candidate substance to an animal (excluding human);
[step B] a step of observing undifferentiated cells in the animal that has undergone step A;
[step C] a step of observing differentiated cells in the animal that has undergone step A; and
[step D] a step of selecting, as the active ingredient,
a candidate substance that improves the amount of undifferentiated cells, or
a candidate substance that improves the amount of differentiated cells or
a candidate substance that improves a function of a tissue composed of differentiated cells,
as compared with a case where the candidate substance is not administered.

2. The screening method according to claim 1, which is a method for screening active ingredients of a regenerative medicine and/or an anticancer agent.

3. The screening method according to claim 1 or 2, which is a method for screening active ingredients for treatment or prevention of a disease, disorder, or illness of nervous system, heart, or pancreas, or symptoms thereof.

4. The screening method according to any one of claims 1 to 3, which is a method for screening active ingredients for treatment of hematological cancer.

5. The screening method according to any one of claims 1 to 4, wherein the candidate substance is one or more selected from a low molecular compound, a protein, a peptide, and a nucleic acid.

6. The screening method according to any one of claims 1 to 5, comprising step B and step C.

7. The screening method according to any one of claims 1 to 6, wherein the animal is a vertebrate.

8. The screening method according to claim 7, wherein the animal is a mammal, a fish, a bird, a reptile, or an amphibian.

9. The screening method according to claim 8, wherein the animal is a zebrafish or a mouse.

10. The screening method according to any one of claims 1 to 9, wherein a candidate substance is administered to an animal embryo in step A.

11. The screening method according to any one of claims 1 to 10, wherein the animal is a zebrafish embryo.

12. The screening method according to any one of claims 1 to 11, wherein in step A, the candidate substance is locally administered to the animal.

13. The screening method according to any one of claims 1 to 12, wherein in step A, the candidate substance is locally administered to an embryo of the animal.

14. The screening method according to any one of claims 1 to 13, wherein in step A, the candidate substance is locally administered to a region occurring in a specific tissue in the embryo of the animal or the vicinity thereof.

15. The screening method according to claim 14, wherein in step B, undifferentiated cells of the specific tissue are observed.

16. The screening method according to claim 14 or 15, wherein in step C, differentiated cells of the specific tissue are observed.

17. The screening method according to any one of claims 1 to 20, wherein
step A comprises locally administering the candidate substance to a region occurring in a specific tissue in the zebrafish embryo or the vicinity thereof,
step B comprises observing undifferentiated cells of the specific tissue, and
step C comprises observing differentiated cells of the specific tissue.

18. The screening method according to any one of claims 14 to 17, wherein the specific tissue is nervous system, heart, or pancreas.

19. The screening method of claim 18, wherein the nervous system is central nervous system.

20. The screening method according to claim 19, wherein the central nervous system is brain, spinal cord, or optic nerve.

21. The screening method according to any one of claims 14 to 20, which is a method for screening active ingredients for treatment or prevention of a disease, disorder, or illness of the same tissue as the specific tissue, or symptoms thereof.

22. The screening method according to any one of claims 14 to 20, which is a method for screening active ingredients for treatment or prevention of a disease, disorder, or illness of a tissue different from the specific tissue, or symptoms thereof.

23. The screening method according to any one of claims 1 to 22, wherein the animal is a transgenic animal into which a reporter gene specifically expressed in the undifferentiated cell and/or the differentiated cell has been introduced.

24. The screening method according to any one of claims 1 to 23, wherein the animal is a transgenic animal into which a reporter gene specifically expressed in the undifferentiated cell has been introduced, and
expression of a reporter gene specifically expressed in the undifferentiated cell in step B is observed.

25. The screening method according to any one of claims 1 to 24, wherein the animal is a transgenic animal into which a reporter gene specifically expressed in the differentiated cell has been introduced, and
expression of a reporter gene specifically expressed in the differentiated cell in step C is observed.

26. The screening method according to any one of claims 23 to 25, wherein the reporter gene encodes a fluorescent protein or a fusion protein of a fluorescent protein and another protein.

27. The screening method according to claim 26, wherein expression of the reporter gene is observed by observing fluorescence of the fluorescent protein.

28. The screening method according to any one of claims 1 to 27, wherein an undifferentiated cell marker is observed in step B.

29. The screening method according to any one of claims 1 to 28, wherein a differentiated cell marker is observed in step C.

30. The screening method according to claim 28 or 29, wherein a means for observing the undifferentiated cell marker and/or the differentiated cell marker is immunostaining or in situ hybridization.

31. The screening method according to any one of claims 1 to 30, wherein the animal is a transgenic animal into which a reporter gene specifically expressed in the undifferentiated cell has been introduced,
expression of a reporter gene specifically expressed in the undifferentiated cell in step B is observed, and
a differentiated cell marker is observed in step C.

32. The screening method according to any one of claims 1 to 31, wherein the animal is a transgenic animal into which a reporter gene specifically expressed in the differentiated cell has been introduced,
an undifferentiated cell marker is observed in step B, and
expression of a reporter gene specifically expressed in the differentiated cell in step C is observed.

33. The screening method according to any one of claims 1 to 32, wherein the animal is a transgenic animal into which a reporter gene specifically expressed in the undifferentiated cell and a reporter gene specifically expressed in the differentiated cell are introduced,
expression of a reporter gene specifically expressed in the undifferentiated cell in step B is observed; and
expression of a reporter gene specifically expressed in the differentiated cell in step C is observed.

34. The screening method according to any one of claims 1 to 33, wherein the candidate substance comprises one or more selected from a 5-HT receptor inhibitor, an AChR inhibitor, an adenylate cyclase activator, an AhR activator, an Akt inhibitor, an ALK inhibitor, an ATPase inhibitor, an autophagy inhibitor, a calcium channel inhibitor, a carbonic anhydrase inhibitor, a Cdc42 inhibitor, a CDK inhibitor, a COX inhibitor, a dehydrogenase inhibitor, a DHFR inhibitor, an EGFR inhibitor, an ERK inhibitor, an estrogen/progestogen receptor inhibitor, a γ-secretase inhibitor, a glutamate receptor ligand (potentiator), a GSK-3 inhibitor, an HDAC activator, an HDAC inhibitor, a Hedgehog/Smoothened agonist, an IGF-IR inhibitor, an interleukin receptor inhibitor, an IRAK inhibitor, a JAK/STAT inhibitor, a MAO inhibitor, a MEK inhibitor, a mitophagy inhibitor, an NF-kB inhibitor, an NF-kB-AMPK-tyrosine kinase pathway inhibitor, a Notch-1 inhibitor, a P450 inhibitor, a PAEF inhibitor, a PDE inhibitor, a PPAR inhibitor, a TGF-β receptor inhibitor, a TGF-beta/Smad inhibitor, a TNF receptor inhibitor, and a Wnt/β-catenin pathway activator.

35. The screening method according to any one of claims 1 to 34, wherein the candidate substance comprises a substance presumed by in silico method to be one or more selected from a 5-HT receptor inhibitor, an AChR inhibitor, an adenylate cyclase activator, an AhR activator, an Akt inhibitor, an ALK inhibitor, an ATPase inhibitor, an autophagy inhibitor, a calcium channel inhibitor, a carbonic anhydrase inhibitor, a Cdc42 inhibitor, a CDK inhibitor, a COX inhibitor, a dehydrogenase inhibitor, a DHFR inhibitor, an EGFR inhibitor, an ERK inhibitor, an estrogen/progestogen receptor inhibitor, a γ-secretase inhibitor, a glutamate receptor ligand (potentiator), a GSK-3 inhibitor, an HDAC activator, an HDAC inhibitor, a Hedgehog/Smoothened agonist, an IGF-IR inhibitor, an interleukin receptor inhibitor, an IRAK inhibitor, a JAK/STAT inhibitor, a MAO inhibitor, a MEK inhibitor, a mitophagy inhibitor, an NF-kB inhibitor, an NF-kB-AMPK-tyrosine kinase pathway inhibitor, a Notch-1 inhibitor, a P450 inhibitor, a PAEF inhibitor, a PDE inhibitor, a PPAR inhibitor, a TGF-β receptor inhibitor, a TGF-beta/Smad inhibitor, a TNF receptor inhibitor, and a Wnt/β-catenin pathway activator.

36. The screening method according to any one of claims 1 to 35, wherein the candidate substance is a substance that positively controls expression of one or more selected from c-Myc, Sox2, Klf4, and Oct4.

37. The screening method according to any one of claims 1 to 37, wherein the candidate substance is a substance presumed by in silico method to positively control the expression of one or more selected from c-Myc, Sox2, Klf4, and Oct4.

38. The screening method according to any one of claims 1 to 37, wherein the candidate substance is a substance that acts on one or more signaling pathways selected from a Notch signaling pathway, a PI3K/AKT/mTOR signaling pathway, a JAK/STAT signaling pathway, a MAPK signaling pathway, a TGFβ/SMAD signaling pathway, and a Wnt signaling pathway.

39. The screening method according to any one of claims 1 to 38, wherein the candidate substance is a substance presumed by in silico method to act on one or more signaling pathways selected from a Notch signaling pathway, a PI3K/AKT/mTOR signaling pathway, a JAK/STAT signaling pathway, a MAPK signaling pathway, a TGFβ/SMAD signaling pathway, and a Wnt signaling pathway.

40. The screening method according to any one of claims 35, 38, and 39, wherein the in silico method is SBDD method and/or LBDD method.

41. The screening method according to any one of claims 1 to 40, wherein
step A comprises locally administering the candidate substance to a region occurring in a specific tissue in the embryo of the animal or the vicinity thereof, and
step C comprises observing function of the specific tissue.

42. The screening method according to any one of claims 1 to 41, wherein
step A comprises locally administering the candidate substance to a region occurring in a specific tissue in the embryo of the animal or the vicinity thereof, and
step C comprises observing motor function of the specific tissue or a secretion amount of a substance secreted by the specific tissue.

43. The screening method according to any one of claims 1 to 42, wherein
step A comprises locally administering the candidate substance to a region occurring in a heart in the embryo of the animal or the vicinity thereof, and
step C comprises observing motor function of the heart.

44. The screening method according to any one of claims 1 to 43, wherein
step A comprises locally administering the candidate substance to a region occurring in a pancreas in the embryo of the animal or the vicinity thereof, and
step C comprises observing a production amount or a secretion amount of insulin by β cells.

45. The screening method according to any one of claims 1 to 44, wherein the animal is a model animal of a disease, disorder, or illness of the nervous system.

46. The screening method according to any one of claims 1 to 45, which is a method for screening active ingredients for treatment or prevention of glaucoma.

47. The screening method according to claim 46, wherein
step A is a step of administering a candidate substance to the retina of the animal,
step B is a step of observing precursor cells of retinal ganglion cells or neural stem cells that differentiate into these cells, and
step C is a step of observing retinal ganglion cells.

48. The screening method according to claim 46 or 47, wherein the animal is a glaucoma model.

49. The screening method according to any one of claims 1 to 44, which is a method for screening active ingredients for treatment of spinal cord injury.

50. The screening method according to claim 49, wherein
step A is a step of administering a candidate substance to the spinal cord of the animal,
step B is a step of observing undifferentiated cells in the spinal cord, and
step C is a step of observing differentiated cells in the spinal cord.

51. The screening method according to claim 49 or 50, wherein the animal is a spinal cord injury model.

52. A method for screening active ingredients for treatment or prevention of a disease, disorder, or illness, or symptoms thereof, comprising the following step E, step F, and step G:
[step E] a step of administering a candidate substance to a model animal (excluding human) of a disease, disorder, or illness;
[step F] a step of determining a therapeutic effect of a disease, disorder, or illness in the animal that has undergone step E; and
[step G] a step of selecting a candidate substance having a therapeutic effect as the active ingredient.

53. The screening method according to claim 52, which is a method for screening active ingredients of a regenerative medicine and/or an anticancer agent.

54. The screening method according to claim 52 or 53, which is a method for screening active ingredients for treatment or prevention of a disease, disorder, or illness of nervous system, heart, or pancreas, or symptoms thereof.

55. The screening method according to any one of claims 52 to 54, wherein in step E, a candidate substance is administered to a retina of a glaucoma model animal, and
in step F, a therapeutic effect on glaucoma is determined.

56. The screening method according to claim 55, wherein in step F, the presence or absence of an increase in retinal ganglion cells is observed.

57. The screening method according to claim 55 or 56, wherein in step F, a therapeutic effect on glaucoma is determined by determining whether or not visual acuity is recovered by behavior observation of the animal.

58. The screening method according to any one of claims 52 to 54, wherein in step E, a candidate substance is administered to a spinal cord injury site in a spinal cord injury model animal, and
in step F, a therapeutic effect on spinal cord injury is determined.

59. The screening method according to claim 58, wherein in step F, a regeneration effect of the spinal cord at the spinal cord injury site is determined.

60. The screening method according to claim 58 or 59, wherein in step F, the therapeutic effect on spinal cord injury is determined by determining whether or not exercise capacity is recovered by behavior observation of the animal.

61. The screening method according to any one of claims 52 to 54, wherein in step E, a candidate substance is injected into a blood vessel of a cancer model animal, and
in step F, a therapeutic effect on cancer is determined.

62. The screening method according to claim 61, wherein in step E, a candidate substance is administered to a leukemia model animal, and
in step F, the presence or absence of thickening or tumorigenesis of vascular endothelium is observed.

63. The screening method according to claim 61 or 62, wherein in step E, a candidate substance is administered to a leukemia model animal, and
in step F, the survival rate of the animal is observed.

64. A method for screening active ingredients for treatment or prevention of a disease, disorder, or illness, or symptoms thereof, wherein primary screening by the screening method according to any one of claims 1 to 51 is performed, and a candidate substance selected as an active ingredient in the primary screening is subjected to secondary screening by the screening method according to any one of claims 52 to 63.

65. The screening method according to claim 64, wherein a candidate substance is administered to a normal animal in step A of the primary screening.

66. The screening method according to any one of claims 1 to 65, wherein the candidate substance is a compound encompassed by the following general formula (I), general formula (II), or general formula (III) or a salt thereof or a hydrate of the compound or salt: 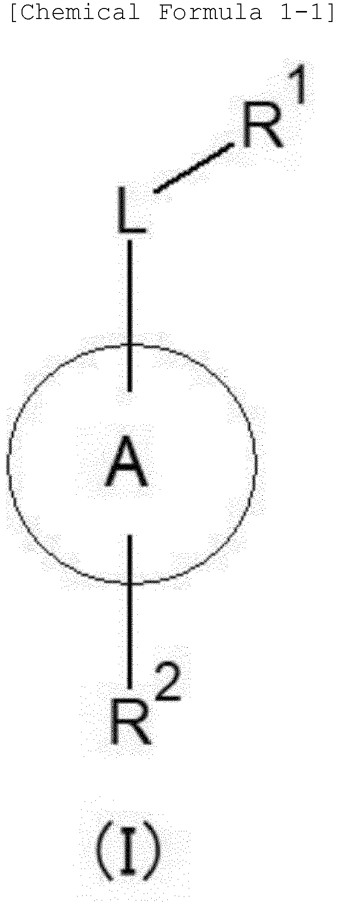 in the general formula (I),
ring A is
a 3- to 8-membered
monocyclic or fused bicyclic group,
which may contain 1 to 4 heteroatoms independently selected from an oxygen atom, a nitrogen atom, and a sulfur atom,
is saturated or unsaturated, and
may be further substituted with a C1-3 alkyl group or a halogen atom;
L is a C1-10 saturated or unsaturated hydrocarbon chain, which may be substituted with substituent R³, or L is absent,
the substituent R³ is a C1-10 saturated or unsaturated hydrocarbon group which may be substituted with a halogen atom, and may have a cyclic structure;
R¹ is
-C≡N, -COOH, -CHO, -COOCH₃, -NO₂, or a halogen atom,
or
a 3- to 8-membered
monocyclic or fused bicyclic group,
which is
may contain 1 to 4 heteroatoms independently selected from an oxygen atom, a nitrogen atom, and a sulfur atom,
is saturated or unsaturated, and
may be further substituted with a C1-3 alkyl group or a halogen atom;
R² is a 3- to 20-membered monocyclic or fused bicyclic group,
which may contain 1 to 6 heteroatoms independently selected from an oxygen atom, a nitrogen atom, and a sulfur atom,
is saturated or unsaturated, and
may be further substituted with a C1-3 alkyl group or a halogen atom, 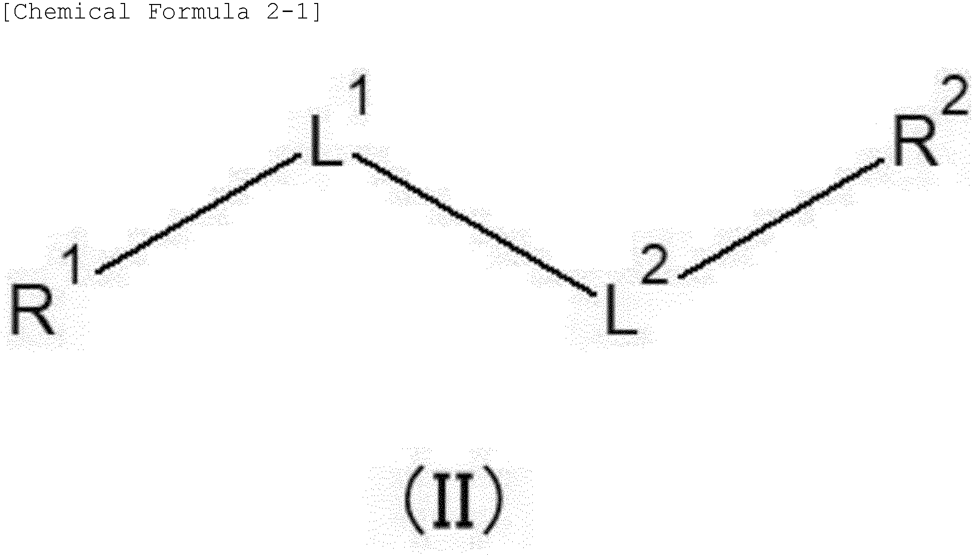 in the general formula (II),
R¹ is
a hydrogen atom,
a halogen atom, or
a C1-20 hydrocarbon group which is saturated or unsaturated, linear or branched, may have a cyclic structure, may be substituted with a halogen atom, and may contain 1 to 4 heteroatoms independently selected from an oxygen atom, a nitrogen atom, and a sulfur atom;
L¹ is any of the following structures: 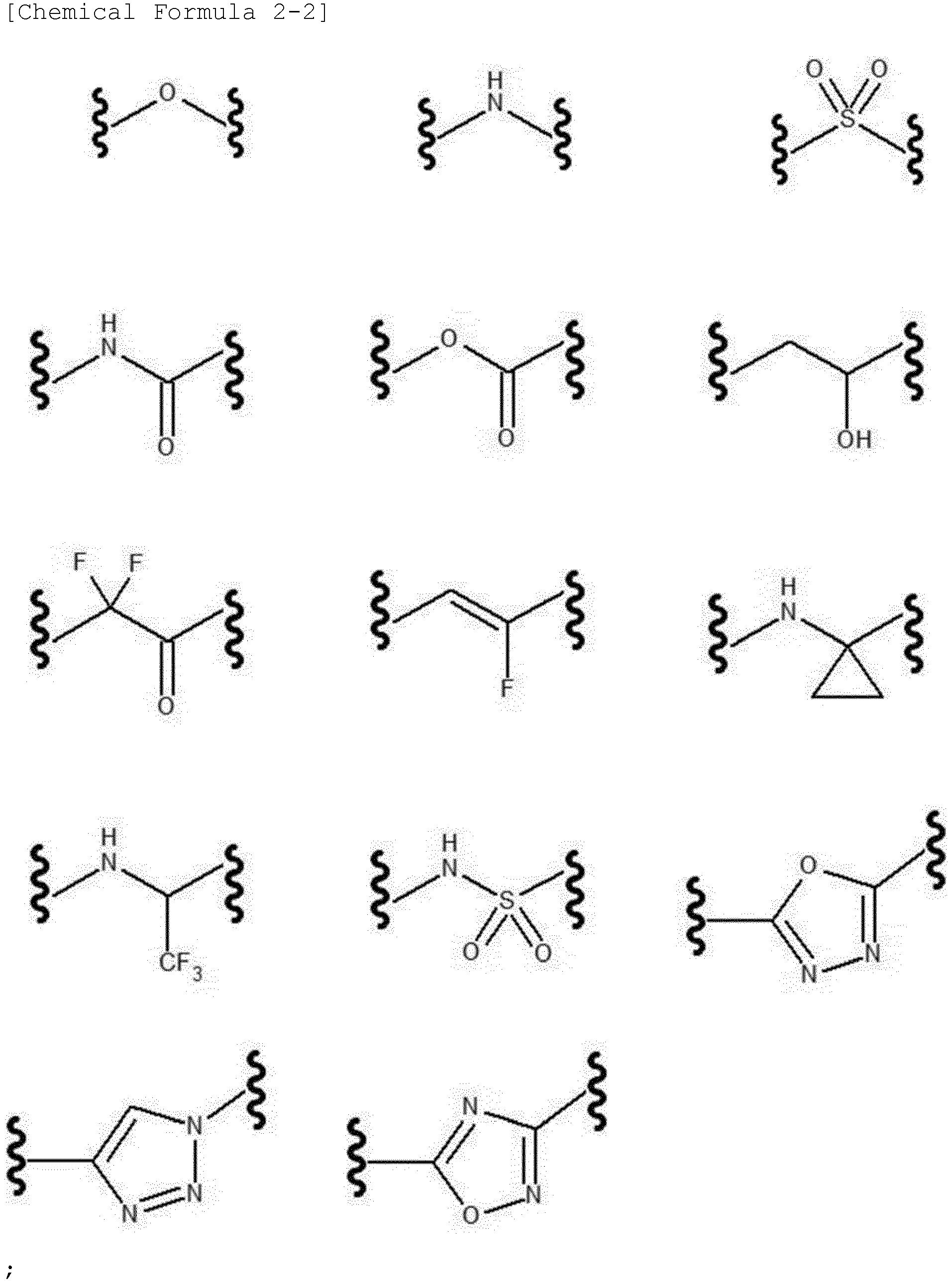
L² is
a C1-30 hydrocarbon chain,
which is saturated or unsaturated,
may be substituted with a group selected from a C1-3 hydrocarbon group, a hydroxyl group which may be substituted with a C1-3 hydrocarbon group, an amino group which may be substituted with a C1-3 hydrocarbon group, a sulfuric acid group which may be substituted with a C1-3 hydrocarbon group, a phosphoric acid group which may be substituted with a C1-3 hydrocarbon group, and a halogen atom, and
may contain 1 to 4 heteroatoms independently selected from an oxygen atom, a nitrogen atom, and a sulfur atom;
R² is a carboxyl group which may be substituted with a C1-3 hydrocarbon group, a hydroxyl group which may be substituted with a C1-3 hydrocarbon group, a hydroxamic acid group which may be substituted with a C1-3 hydrocarbon group, a sulfo group which may be substituted with a C1-3 hydrocarbon group, a boronic acid group which may be substituted with a C1-3 hydrocarbon group, a carbamoyl group which may be substituted with a C1-3 hydrocarbon group, a sulfamoyl group which may be substituted with a C1-3 hydrocarbon group, a sulfoximine group which may be substituted with a C1-3 hydrocarbon group, a cyano group, or a tetrazolyl group, 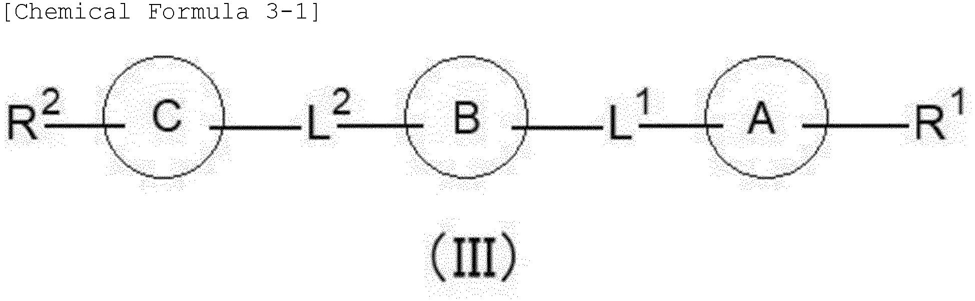 in the general formula (III),
ring A, ring B, and ring C are each independently
a 3- to 8-membered
monocyclic ring
which may contain 1 to 4 heteroatoms independently selected from an oxygen atom, a nitrogen atom, and a sulfur atom,
is saturated or unsaturated, and
further which may be substituted with a C1-3 hydrocarbon group, a halogen atom, a hydroxyl group which may be substituted with a C1-3 hydrocarbon group, an amino group which may be substituted with a C1-3 hydrocarbon group, a sulfuric acid group which may be substituted with a C1-3 hydrocarbon group, or a phosphoric acid group which may be substituted with a C1-3 hydrocarbon group;
R¹ is a group selected from the following: 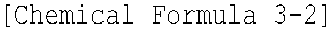 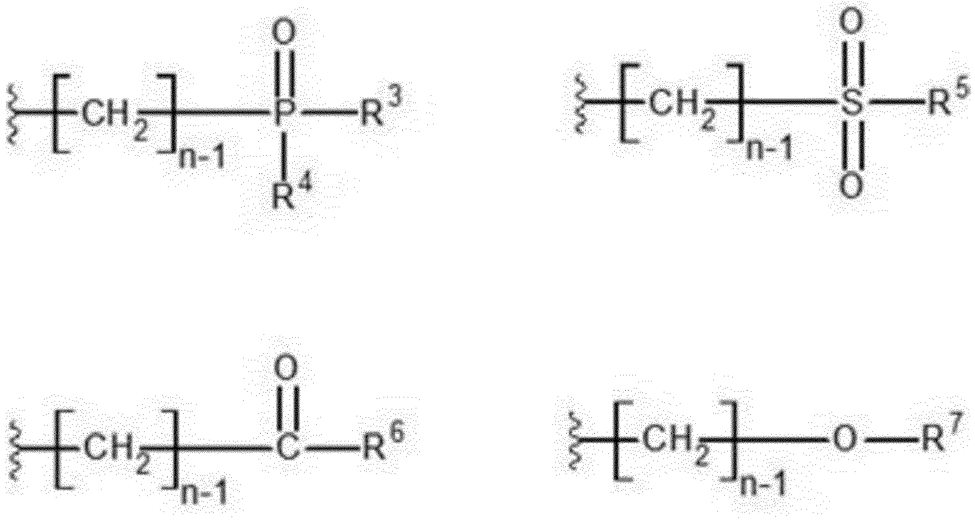
R³ to R⁶ are each independently
a hydrogen atom, a C1-3 hydrocarbon group, a hydroxyl group which may be substituted with a C1-3 hydrocarbon group, or an amino group which may be substituted with a C1-3 hydrocarbon group,
R⁷ is a hydrogen atom, a C1-3 hydrocarbon group, or an amino group which may be substituted with a C1-3 hydrocarbon group, and
n represents an integer of 1 to 4;
L¹ and L² are each independently
a C1-3 alkylene group or alkenylene group, -N(H)-, or -O-,
among these divalent groups, a group other than -O-may be further substituted with a C1-30 hydrocarbon group which is saturated or unsaturated, linear or branched, may have a cyclic structure, may be substituted with a halogen atom, and may contain 1 to 6 heteroatoms independently selected from an oxygen atom, a nitrogen atom, and a sulfur atom; and
R² is a C1-30 hydrocarbon group which is saturated or unsaturated, linear or branched, may have a cyclic structure, may be substituted with a halogen atom, may contain 1 to 6 heteroatoms independently selected from an oxygen atom, a nitrogen atom, and a sulfur atom.

67. The screening method according to any one of claims 1 to 66, wherein the candidate substance is a derivative of any one of Compounds 1 to 7 below or a salt thereof or a hydrate of the compound or salt.

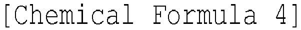

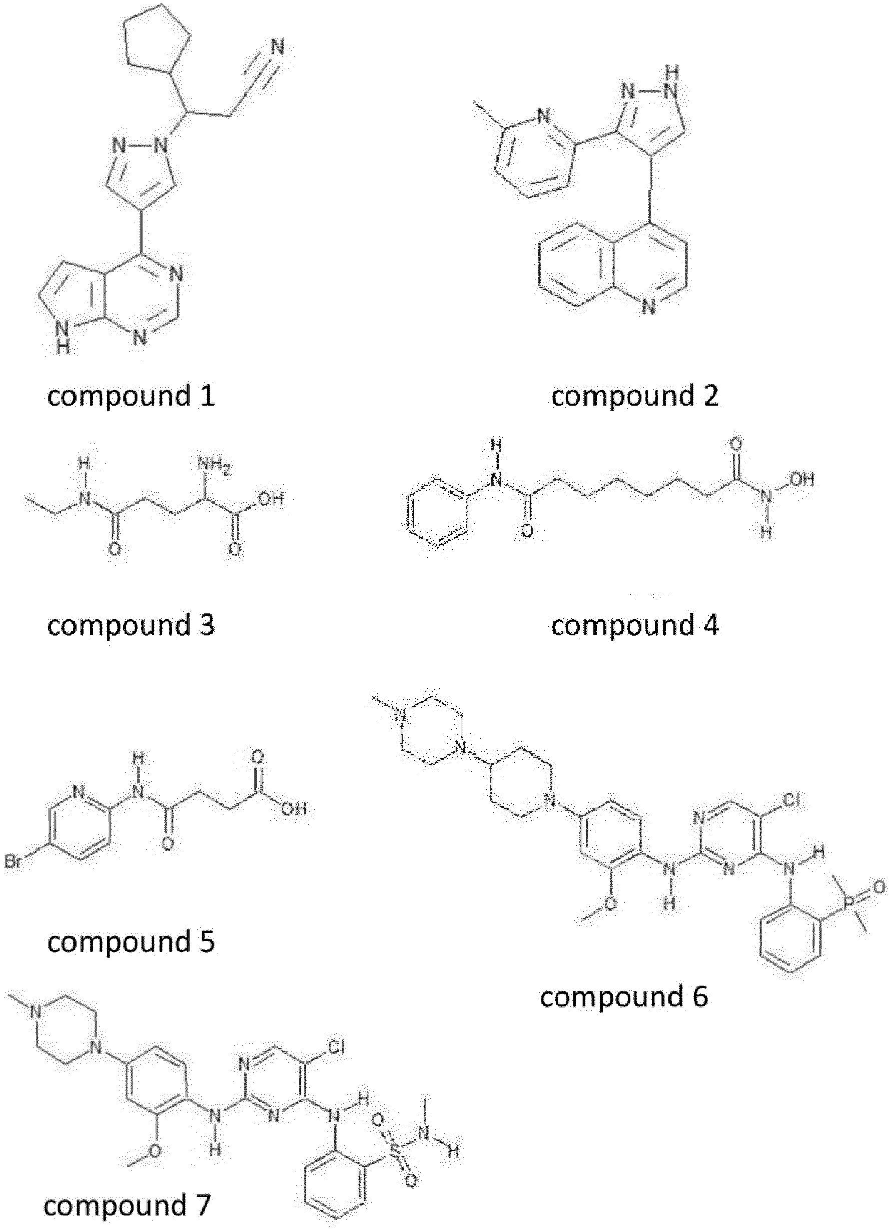

68. A method for producing a pharmaceutical composition, comprising a formulation step of mixing and formulating a substance selected as an active ingredient and a pharmaceutical additive by the screening method according to any one of claims 1 to 67.

69. The production method according to claim 67, wherein the screening method is the screening method according to claim 66 or 67.

70. The production method according to claim 68 or 69, wherein the pharmaceutical composition is a regenerative medicine and/or an anticancer agent.

71. The production method according to any one of claims 68 to 70, wherein the pharmaceutical composition is used for treatment or prevention of a disease, disorder, or illness of nervous system, heart, or pancreas, or symptoms thereof.

72. The production method according to any one of claims 68 to 71, wherein the pharmaceutical composition is used for treatment or prevention of glaucoma.

73. The production method according to any one of claims 68 to 71, wherein the pharmaceutical composition is used for treatment of spinal cord injury.

74. The production method according to any one of claims 68 to 71, wherein the pharmaceutical composition is used for treatment of hematological cancer.

75. The production method according to any one of claims 68 to 71, wherein the pharmaceutical composition is used for treatment of leukemia.

76. The production method according to any one of claims 68 to 75, wherein the pharmaceutical composition is a liquid agent, and
the formulation step comprises mixing the active ingredient and an aqueous medium.

77. The production method according to claim 76, wherein the pharmaceutical composition is an injection.

78. The production method according to claim 76, wherein the pharmaceutical composition is an eye drop.

79. The production method according to any one of claims 68 to 75, wherein the pharmaceutical composition is a lyophilized preparation, and
the formulation step comprises dissolving the active ingredient in a solvent and freeze-drying the dissolved active ingredient.

80. The production method according to any one of claims 68 to 75, wherein the pharmaceutical composition is an ointment, and
the formulation step comprises mixing the active ingredient and a substrate.

81. The production method according to claim 80, wherein the pharmaceutical composition is an ophthalmic ointment.

82. The production method according to any one of claims 68 to 75, wherein the pharmaceutical composition is a preparation for nasal administration.

83. The production method according to any one of claims 68 to 75, wherein the pharmaceutical composition is a preparation for oral administration.

84. A method for designing a pharmaceutical composition, comprising the step of selecting a pharmaceutical additive to be combined with a substance selected as an active ingredient by the screening method according to any one of claims 1 to 67.

85. The design method according to claim 84, wherein the pharmaceutical composition is a regenerative medicine and/or an anticancer agent.

86. The design method according to claim 84 or 85, comprising the step of selecting an indication of the pharmaceutical composition.

87. The design method according to claim 86, wherein the indication is a disease, disorder, or illness of nervous system, heart, or pancreas, or symptoms thereof, or cancer.

88. The design method according to any one of claims 84 to 87, comprising the step of selecting a dosage form of the pharmaceutical composition.

89. The design method according to claim 88, comprising selecting a liquid agent as the dosage form and selecting an aqueous medium to be mixed with the active ingredient.

90. The design method according to claim 89, wherein an injection is selected as the dosage form.

91. The design method according to claim 89, wherein an eye drop is selected as the dosage form.

92. The design method according to claim 88, wherein a lyophilized preparation is selected as the dosage form.

93. The design method according to claim 88, comprising selecting an ointment as the dosage form and selecting a substrate to be mixed with the active ingredient.

94. The design method according to claim 93, wherein an ophthalmic ointment is selected as the dosage form.

95. The design method according to claim 88, wherein a preparation for nasal administration is selected as the dosage form.

96. The design method according to claim 88, wherein a preparation for oral administration is selected as the dosage form.

97. A method comprising designing a pharmaceutical composition according to the method of any one of claims 84 to 96,
preparing a substance selected as an active ingredient by the screening method according to any one of claims 1 to 67,
producing a pharmaceutical composition by the production method according to any one of claims 68 to 83, and
statistically processing a data set obtained from persons to whom the pharmaceutical composition was administered in a clinical trial.
